# EUROPÄISCHE PATENTSCHRIFT

(11) **EP 1 254 254 B1**
(45) Veröffentlichungstag und Bekanntmachung des Hinweises auf die Patenterteilung: **09.11.2011**
(21) Anmeldenummer: 00964152.3
(22) Anmeldetag: 08.09.2000
(51) Int. Cl.: C12Q 1/68

(54) **NUKLEINSÄUREMOLEKÜLE ZUM NACHWEIS VON BAKTERIEN UND PHYLOGENETISCHEN EINHEITEN VON BAKTERIEN**
NUCLEIC ACID MOLECULES FOR DETECTING BACTERIA AND PHYLOGENETIC UNITS OF BACTERIA
MOLECULES D'ACIDE NUCLEIQUE DESTINEES A LA DETECTION DE BACTERIES ET D'UNITES PHYLOGENETIQUES DE BACTERIES

(30) Priorität: 24.09.1999 DE 19945916
(43) Veröffentlichungstag der Anmeldung: 06.11.2002
(73) Patentinhaber: Biotecon Diagnostics GmbH, 10589 Berlin (DE)
(72) Erfinder: GRABOWSKI, Reiner, 37075 Göttingen (DE); BERGHOF, Kornelia, 12355 Berlin (DE)
(74) Vertreter: Grünecker, Kinkeldey, Stockmair & Schwanhäusser
(86) Internationale Anmeldenummer: PCT/EP2000/008813
(87) Internationale Veröffentlichungsnummer: WO 2001/023606

(56) Entgegenhaltungen:
- EP-A- 0 739 988
- EP-A1- 0 337 896
- WO-A-90/15157
- WO-A-93/11264
- WO-A-95/13396
- DE-A- 19 616 750
- DE-A- 19 731 292
- DE-A- 19 739 611
- US-A- 5 521 300
- US-A- 5 595 874
- US-A- 5 595 874
- US-A- 5 776 680
- BLATTNER FREDERICK R ET AL: "The complete genome sequence of Escherichia coli K-12." SCIENCE (WASHINGTON D C), Bd. 277, Nr. 5331, 1997, Seiten 1453-1462, XP002069950 ISSN: 0036-8075
- ANTON ANA I ET AL: "Intraspecific diversity of the 23S rRNA gene and the spacer region downstream in Escherichia coli." JOURNAL OF BACTERIOLOGY, Bd. 181, Nr. 9, Mai 1999 (1999-05), Seiten 2703-2709, XP002179168 ISSN: 0021-9193
- BRENNER D J ET AL: "CONSERVATION OF TRANSFER RNA AND 5S RNA CISTRONS IN ENTEROBACTERIACEAE" JOURNAL OF BACTERIOLOGY, Bd. 129, Nr. 3, 1977, Seiten 1435-1439, XP002082055 ISSN: 0021-9193
- DATABASE EBI EBI 19 Dezember 1996 'http://www.ebi.ac.uk/cgi-bin/epo/epofetch? AAT48620' Retrieved from EBI Database accession no. AAT48620
- DATABASE EBI 'http://www.ebi.ac.uk/cgi-bin/epo/epofetch? AAV61640' Retrieved from EBI Database accession no. AAV61640
- DATABASE EBI [Online] 05 März 1997 'http://srs.ebi.ac.uk/srsbin/cgi-bin/wgetz? -e+([emblidacc-id:A41761]>embl)¦[embl-acc:A 41761]+-vn+2+-noSession' Retrieved from EBI Database accession no. A41761
- BRANLANT C ET AL: "Structural study of ribosomal 23 S RNA from Escherichia coli", FEBS LETTERS, ELSEVIER, AMSTERDAM, NL, vol. 107, no. 1, 1 November 1979 (1979-11-01), pages 177-181, XP025569492, ISSN: 0014-5793, DOI: DOI:10.1016/0014-5793(79)80490-1 [retrieved on 1979-11-01]
- DATABASE EBI EBI; 19. Dezember 1996 (1996-12-19), "http://www.ebi.ac.uk/cgi-bin/epo/epofetch ?AAT48620" gefunden im EBI Database accession no. AAT48620
- DATABASE EBI "http://www.ebi.ac.uk/cgi-bin/epo/epofetch ?AAV61640" gefunden im EBI Database accession no. AAV61640
- DATABASE EBI 5. März 1997 (1997-03-05), "http://srs.ebi.ac.uk/srsbin/cgi-bin/wgetz ?-e+([emblidacc-id:A41761]>embl)¦[embl-acc :A41761]+-vn+2+-noSession" gefunden im EBI Database accession no. A41761

## Beschreibung

Die vorliegende Erfindung betrifft Nukleinsäuremoleküle, die die Identifikation von Bakterien oder Bakteriengruppen ermöglichen.

Bakterien sind ein allgegenwärtiger Bestandteil der menschlichen Umwelt. Sie verursachen jedoch als Lebensmittelverderber oder Krankheitserreger so häufig Probleme, daß einer effektiven, schnellen und zuverlässigen Diagnostik sehr große Bedeutung zukommt.

Zu den wichtigsten Mikroorganismen, die Lebensmittel verderben gehören Clostridium Botulinum, der Erreger des Botulismus, Campylobacter jejuni, Clostridium perfringens, Cryptosporidium parvum, enteropathologische Escherichia coli Stämme, Shigella, Listeria monocytogenes, Salmonellen Spezies, Staphylococcus aureus, Vibrio vulnificus und Yersinia enterolytica. In den USA hat das General Accounting Office (GAO) 1996 berichtet, daß 6,5-81 Millionen Fälle von Lebensmittelvergiftung jährlich vorkommen. Die amerikanische Food and Drug Administration (FDO) schätzt, daß 2-3 % aller Lebensmittelvergiftungen zu sekundären chronischen Erkrankungen führt. Es wird außerdem geschätzt, daß jedes Jahr 2-4 Millionen Erkrankungen in den USA durch mehr als 2000 Salmonella Stämme verursacht werden. Diese Schreckensstatistik ließe sich für andere Lebensmittelverderber beliebig verlängern. Lebensmittelvergiftungen verursachen jedoch nicht nur menschliches Leid, im Extremfall den Tod, sondern auch einen beträchtlichen ökonomischen Schaden. Dieser wird in den USA für 1991 z.B. auf 5,6-9,4 Milliarden $ geschätzt.

Es ist allgemein bekannt, daß von Mikroorganismen als Infektionserreger große Gefahren ausgehen, die in ihrem Potential kaum abzuschätzen sind. Statistische Größenordnungen werden z.B. vom Weltgesundheitsbericht der WHO reflektiert. So waren 1998 Infektionserreger, inklusive Parasiten, für 9,8 Millionen Todesfälle verantwortlich (ohne prä- oder postnatale Infektionen), was einem Anteil von 18,2 % an allen krankheitsbedingten Todesfällen entspricht. Die gefährlichen Erreger lassen sich nicht so gut zusammenfassen wie die Lebensmittelverderber, da sie sich aus vielen phylogenetischen Zweigen der Eubakterien rekrutieren. Ein besonders großes "infektziöses Potential" besteht vor allem innerhalb der Familie der Enterobakterien.

Im Kampf gegen humanpathogene Bakterien besteht ein wesentlicher Schritt in der Identifizierung des für eine Krankheit oder ein pathologisches Symptom ursächlichen Keims. Häufig können erst nach der Identifizierung geeignete medizinische Maßnahmen ergriffen werden. Darüber hinaus können gut funktionierende Nachweismethoden für Bakterien als präventive Werkzeuge der Qualitätssicherung von Lebensmitteln eingesetzt werden.

Der klassische Nachweis von Bakterien besteht in der mikrobiologischen Identifizierung, die in der Regel eine Vereinzelung auf Agar-Agar-haltigen selektiven Medien umfaßt. Dieses Verfahren hat jedoch zwei wesentliche Nachteile: erstens ist der Nachweis häufig nicht zuverlässig oder spezifisch. Zweitens benötigen viele Bakterien eine Wachstumszeit von mindestens 18 Stunden zur Vereinzelung als Kolonie. In vielen Fällen ist zudem eine Sekundärvereinzelung oder ein Sekundärnachweis erforderlich. Alles in allem sind dann Diagnosezeiten von bis zu einer Woche keine Seltenheit. Darüber hinaus gibt es auch pathogene Keime, die sich nicht kultivieren lassen (J.J. Byrd et al. 1991, Appl. Environ. Microbiol. 57, 875-878). In Zeiten schneller Transportwege und globalen Warenverkehrs sind jedoch schnelle diagnostische Verfahren, die im optimalen Fall nicht länger als 24 h dauern, essentiell, um eine Verbreitung Krankheitserregern oder global gestreute Lebensmittelvergiftungen aus nur einer lokalen Quelle zu verhindern.

Um modernen Anforderungen gerecht zu werden wurden in den letzten Jahren eine Reihe von Verfahren entwickelt, die eine schnelle und zuverlässige routinemäßige Identifizierung von Keimen leisten sollen. So nutzen z.B. immunologische Methoden die spezifische Bindung monoklonaler oder polyklonaler Antikörper an bakterielle Oberflächenantigene. Solche Verfahren werden insbesondere zur Serotypisierung, z.B. von Salmonellen verwendet. Im allgemeinen ist der Nachweis mit einem ELISA zwar relativ schnell, verlangt jedoch die Prozessierung und Isolierung des jeweiligen Antigens, was mit vielen Problemen behaftet sein kann. Als besonders leistungsfähig erwiesen sich bakterielle Nachweismethoden, die sich DNA-Sonden bedienen, denn sie sind sehr sensitiv, relativ spezifisch und lassen sich in einem experimentellen zeitlichen Gesamtrahmen von 2-3 Tagen zum Nachweis von Mikroorganismen nutzen.

### Hintergrund der Erfindung

Die Erfindung besteht in der Bereitstellung spezifischer DNA-Sequenzen und in der Auswahl von DNA-Regionen, die zum Nachweis von Bakterien besonders geeignet sind. Dieser Anwendung liegt also die Identifikation von Organismen anhand ihrer Erbinformationen zugrunde. Abweichungen in der Nukleotidsequenz bestimmter DNA-Bereiche in mindestens einem einzigen Baustein können bereits zur Differenzierung von Spezies genutzt werden.

Historisch betrachtet wurden ribosomale RNA Gene bereits für die phylogenetische Einordnung von Organismen genutzt. Sequenzvergleiche von 5S und 16S ribosomalen Genen verschiedener Bakterien fuhrten zu beträchtlichen Korrekturen bei verwandtschaftlichen Zuordnungen und zur Entdeckung des Reiches der Archaebacteria. Aufgrund ihrer Größe und dem dementsprechend hohen Sequenzieraufwand wird die 23S RNA erst in den letzten Jahren zum Zwecke systematischer Einteilungen genutzt.

In praktischen Anwendungen war die direkte Sequenzierung von Genen zu identifizierender Mikroorganismen zu aufwendig und zeitraubend. In den 80er Jahren wurden deshalb insbesondere Nukleotidsonden zum Nachweis von Bakterien verwendet. Diese können zwar eine sehr gute Spezifität aufweisen, die Nachweisgrenze ist jedoch häufig zu niedrig. Eine entscheidende Verbesserung erfuhr die Sondentechnologie in der Kombination mit Amplifikationstechniken, die also die nachzuweisende Nukleotidsequenz vermehren und dadurch die Sensitivität des Nachweises beträchtlich erhöhen. So ist es möglich im Extremfall ein einziges isoliertes Genom nachzuweisen. In der Praxis treten bei der Isolierung von DNA Verluste auf, die die Nachweisgrenze der Zellzahl auf ca. 10² bis 10⁴ erhöhen.

Basierend auf Arbeiten der Grundlagenforschung wurden DNA-Sonden aus den 5S-, 16S- und 23S-Genen zur praktischen Anwendung genutzt. Erwähnt werden können z.B. die Patente Nietupski et al. (US 5,147,778) zur Detektion von Salmonella, Mann und Wood (US 6,554,144) zur Detektion von Yersinia Spezies, Leong (EP 0479117 A1) zur Detektion verschiedener gramnegativer und grampositiver Bakterien, Carico et al. (EP 133671 B1) zur Detektion verschiedener enterobakterieller Spezies, Shah et al. (EP 0339783 B1) zur Detektion von Yersinia enterolytica, Carrico (EP 0163220 B1) zur Detektion von Escherichia coli, Hogan et al. (WO 88/03957) zur Detektion von Spezies der Enterobakterien, Mycobacterium, Mycoplasma und Legionella, Leiser et al. (WO 97/41253) zur Detektion verschiedener Mikroorganismen, Grosz und Jensen (WO 95/33854) zur Detektion von Salmonelle enterica, Stackebrandt und Curiaie (EP 0314294 A2) zur Detektion von Listeria monocytogenes, Wolff et al. (EP 0408077 A2), Hogan und Hammond (US 5681698) zum Nachweis von Mycobacterium kansasii, Hogan et al. (US 5 679 520) zum Nachweis verschiedener Bakterien, Kohne (US 5 567 587) insbesondere zur Detektion von bakterieller rRNA, Kohne (US 5 714 324) zur Detektion verschiedener Bakterien, Pelletier (WO 94/28174) zum Nachweis von Legionella und Kohne (US 5 601 984) zum Nachweis verschiedener Bakterien. Das Gros der Patentschriften bezieht sich dabei auf die Sequenz des 16 S rDNA-Gens, viele auch auf die 23 S rDNA.

Es zeigte sich jedoch, daß letztere Gene für viele Differenzierungsleistungen in der praktischen Anwendung nicht geeignet sind, da sie zu stark konserviert sind. Insbesondere nahe verwandte Mikroorganismen lassen sich nicht unterscheiden. Auf der anderen Seite wurde das 5 S rDNA-Gen in der Grundlagenforschung auf Grund der geringen Größe ursprünglich zwar für phylogenetische Studien genutzt, in der praktischen Anwendung ist es jedoch in der Regel zu variabel und das Differenzierungspotential zu gering.

Da 5 S, 16 S und 23 S rDNA-Gene als diagnostische Hilfsmittel mit vielen Nachteilen behaftet sind, wurde nach DNA-Regionen gesucht, die zur Identifizierung aller Eubakterien herangezogen werden können. Eine solche DNA-Region sollte sehr variable und zugleich stark konservierte Sequenzen aufweisen. Die variablen Bereiche wären dann zur Differenzierung nahe verwandter Spezies, wie Stämme und Arten, geeignet. Die konservierten Sequenzabfolgen wären zum Nachweis entfernt verwandter Bakterien oder von höheren taxonomischen Einheiten zu nutzen.

Im Kontext ausgedehnter Studien über ribosomale Operons wurde auch der 16 S-23 S-transkribierte Spacer in jüngster Vergangenheit in der Literatur diskutiert; die Anwendbarkeit in der systematischen Bakteriologie jedoch in Frage gestellt. So sehen Nagpal et al. (J. Microbiol. Meth. 33, 1998, S. 212) die Nutzbarkeit dieses Spacer äußerst kritisch: Ein besonderes Problem dieses transkribierten rDNA-Spacers liegt vor allem auch darin, daß er häufig tRNA-Insertionen enthält. Solche Insertionen stellen dramatische Sequenzabweichungen dar, und haben nicht notwendigerweise eine Relation zu phylogenetischen Abständen. Sie wurden jedoch in der Vergangenheit genutzt, um den durch sie verusachten Längenpolymorphismus als phylogenetisches Charakteristikum heranzuziehen (Jensen et al. 1993, Appl. Envir. Microb. 59, 945-952, Jensen, WO 93/11264, Kur et al. 1995, Acta Microb. Pol. 44, 111-117).

Eine alternative Zielsequens zur Identifizierung von Bakterien besteht in dem transkribierten Spacer zwischen 23 S und 5 S rDNA. So publizierten Zhu et al. (J. Appl. Bacteriol. 80, 1996, 244-251) den Nachweis von Salmonella typhi mit Hilfe dieser diagnostischen DNA-Region. Die generelle Nutzbarkeit dieses Spacers zum Nachweis auch anderer Bakterien läßt sich aus dieser Arbeit aber nicht ableiten. Es gibt sehr viele Beispiele, die zeigen, daß eine DNA-Region nur zur Identifikation einer oder weniger bakterieller Spezies geeignet ist. Einzelne Patente implizieren eine mögliche, doch sehr begrenzte Anwendbarkeit des 23 S-5 S transkribierten DNA-Bereichs für die bakterielle Diagnostik. Sie alle haben gemeinsam, daß ihe Anwendbarkeit sich nur auf eine bakterielle Spezies bezieht, und zwar auf den Nachweis von Legionellen (Heidrich et al., EP 0739988 A1), von Pseudomonas aeruginosa (Berghof et al., DE 19739611 A1) und auf den Nachweis von Staphylococcus aureus (Berghof et al., WO 99/05159).

Der vorliegenden Erfindung lag das technische Problem zugrunde, Materialien und Verfahren anzugeben, die es ermöglichen, ein beliebiges Bakterium aus der Gruppe der Enterobakterien in einem Untersuchungsmaterial nachzuweisen.

Dieses Problem wird erfindungsgemäß gelöst durch ein Nukleinsäuremolekül als Sonde und/oder Primer zum Nachweis von der taxonomischen Einheit der Enterobacteriaceae, ausgewählt aus
a) einem Nukleinsäuremolekül bestehend aus einer Sequenz mit einer der SEQ ID-Nrn. 2-26, 34, 42, 54, 66, 78, 85 bzw. 135;
b) einem Nukleinsäuremolekül, das spezifisch mit einer Nukleinsäure nach a) hybridisiert;
c) einem Nukleinsäuremolekül, das mindestens 90% Identität zu einer Nukleinsäure nach a) aufweist,
d) einem Nukleinsäuremolekül, das komplementär zu einer Nukleinsäure nach a) bis
e) ist;
wobei das Nukleinsäuremolekül mindestens 14 Nukleotide lang ist.

Kombinationen der genannten Nukleinsäuren nach a) bis d).

Die weiteren Ansprüche betreffen bevorzugte Ausführungsformen.

Bei einer besonders bevorzugten Ausführungsform wird das Vorliegen von Enterobakterien in einer Analysenprobe nachgewiesen, indem die Analysenprobe mit einer Sonde in Kontakt gebracht wird, die das Vorliegen einer Nukleinsäure aus dem 23S/5S rDNA Genomabschnitt der Enterobakterien nachweist.

Die Sequenz mit der Nr. 1 stammt aus *E*. *Coli.* Homologe DNA Sequenzen sind solche, die anderen Bakterien als die gezeigte *E*. *Coli* Sequenz entstammen, wobei jedoch der Genomabschnitt der anderen Bakterien dem der SEQ ID Nr. 1 zugrundeliegenden Sequenz entspricht. Für nähere Einzelheiten wird auf die noch folgende Denfinition der homologen DNA Sequenzen verwiesen.

Das erfindungsgemäße Nukleinsäuremolekül umfaßt mindestens 14 Nukleotide. Nukleinsäuremoleküle dieser Länge werden vorzugsweise als Primer eingesetzt, während als Sonde verwendete Nukleinsäuren vorzugsweise mindestens 50 Nukleotide umfassen.

Bei einer weiteren bevorzugten Ausführungsform können auch Nukleotide der Sonde bzw. des Primers ersetzt sein durch modifizierte Nukleotide, enthaltend z. B. angefügte Gruppen, die letztlich einer Nachweisreaktion dienen. Besonders bevorzugte Derivatisierungen sind in Anspruch 3 angegeben.

Bei einer weiteren bevorzugten Ausführungsform werden Kombinationen der genannten Nukleinsäuremoleküle eingesetzt. Die Zusammenstellung der Kombination aus Nukleinsäuremolekülen gestattet es, die Selektivität der Nachweisreaktion festzulegen. Dabei können durch Auswahl der Primerkombinationen und/oder Sondenkombinationen die Bedingungen der Nachweisreaktion so festgelegt werden, daß diese entweder generell das Vorhandensein von Bakterien in einer Probe nachweisen oder aber spezifisch das Vorhandensein einer bestimmten Bakterienspezies anzeigt.

Ein erfindungsgemäßer Kit enthält mindestens eine erfindungsgemäße Nukleinsäure zusammen mit den weiteren üblichen Reagenzien, die bei Nukleinsäurenachweisen eingesetzt werden. Hierzu zählen u. a. geeignete Puffersubstanzen und Nachweismittel wie Enzyme, mit denen beispielsweise gebildete biotinylierte Nukleinsäurehybride nachgewiesen werden können.

Bei einer weiteren bevorzugten Ausführungsform, hierin auch als Konsensus PCR bezeichnet, wird das Verfahren gemäß Anspruch 7 durchgeführt. Dabei wird zunächst durch den Einsatz konservierter Primer (diese hybridisieren an Nukleinsäuren verschiedener bakterieller taxonomischer Einheiten) ein Nukleinsäurefragment amplifiziert und durch den Einsatz weiterer spezifischerer Nukleinsäuren (diese hybridisieren nur noch mit wenigen taxonomischen Einheiten oder nur einer bestimmten Spezies) werden dann spezifischere Nukleinsäureabschnitte nachgewiesen. Letztere erlauben dann den Rückschluß auf das Vorhandensein einer bestimmten Gattung, Art oder Spezies in der Analysenprobe.

In den eingesetzten Verfahren zum Nachweis von Nukleinsäure können die verschiedensten etablierten Nachweise verwendet werden. Hierzu zählen Southern Blot-Techniken, PCR-Techniken, LCR-Techniken usw.

In einer breit angelegten Studie wurde transkribierter Spacer zwischen 23S- und 5S-rDNA in seiner generellen Nutzbarkeit als diagnostisches Zielmolekül untersucht. Zu diesem Zweck wurde genomische DNA sehr vieler bakterieller Stämme isoliert, aufgereinigt, in einen Vektor kloniert, sequenziert und schließlich in einem umfangreichen Sequenzvergleich ausgewertet. Überraschenderweise war dieser Sequenzabschnitt durchaus zur Identifizierung fast aller bakteriellen Spezies geeignet. Ermutigt durch diesen Befund wurden die Analysen auf die benachbarten Bereiche des Spacers ausgedehnt. Alles in allem wurden DNA-Fragmente von allen bakteriellen Klassen oder kleineren phylogenetischen Einheiten untersucht. Sie haben eine Ausdehnung von 400-750 Basenpaaren und umfassen das Ende, d.h. speziesabhängig die letzten 330-430 Nukleotide des 23 S rDNA-Gens, den transkribierten Spacer und das komplette 5 S rDNA-Gen. Die Gesamtgröße der Fragmente beträgt 400-750 Basenpaare. Die Experimente zeigten, daß bei fast allen bakteriellen Spezies das 23S rDNA-Gen und das 5 S rDNA-Gen benachbart sind. Diese Erkenntnis ist eine wichtige Voraussetzung für die Nutzung und Anwendbarkeit der vorliegenden Erfindung.

Die vorliegende Erfindung beruht insbesondere darauf, daß eine DNA-Region zum Nachweis von Mikroorganismen ausgewählt wird, die wesentliche Anteile von mindestens zwei benachbarten Genen beinhalten kann. In der Praxis wird die Nützlichkeit des Bereiches insbesondere durch die phylogenetische Variabilität desselben bestimmt. Je nachdem ob enfernt verwandte Bakterien, bzw. taxonomische Einheiten oder ob Stämme einer Spezies bestimmt werden sollen, kann es diesbezüglich völlig konträre Anforderungen geben. Die Häufigkeit des Vorkommens sowohl variabler, als auch konservierter Regionen ist nun, wie am Beispiel des 23 S-5 S-Tandems gezeigt, bei zwei Genen größer als bei einem Gen. Das Nutzen von zwei benachbarten Genen, inklusive der variablen interkalierenden Sequenzen stellt also einen beträchtlichen Vorteil dar.

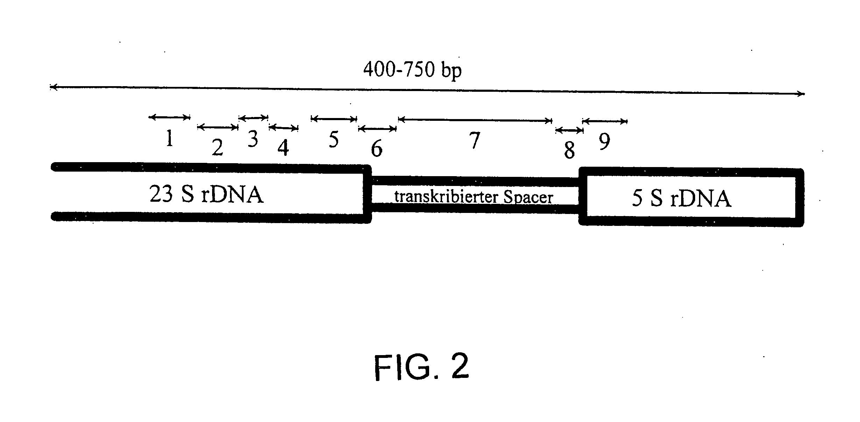

Es wurde ferner gefunden, daß das Ende des 23 S rDNA-Gens, der dazwischenliegende transkribierte Spacer und das 5S rDNA Gen, Nukleotidsequenzen enthalten, die ein vielfältiges Spektrum von sehr variabel bis sehr konserviert abdecken. Eine Feinanalyse dieser Region ergab sehr interessante weitere Aufschlüsse bezüglich des Differenzierungspotentials verschiedener phylogenetischer bakterieller Einheiten (Abb. 2, Tab. 6). Nahezu alle taxonomischen Einheiten sind unter Nutzung von Subbereichen nachzuweisen und/oder zu differenzieren. Dargestellt wurden in Abb. 2 mit den Sektionen 1-9 insbesondere mehr oder weniger variable Bereiche, während die stark konservierten diese interkalieren und diesen benachbart sind. Letztere eignen sich also besonders zum Nachweis hoher taxonomischer Einheiten, wie der gesamten Eubakterien oder von Klassen oder Abteilungen aus diesen.

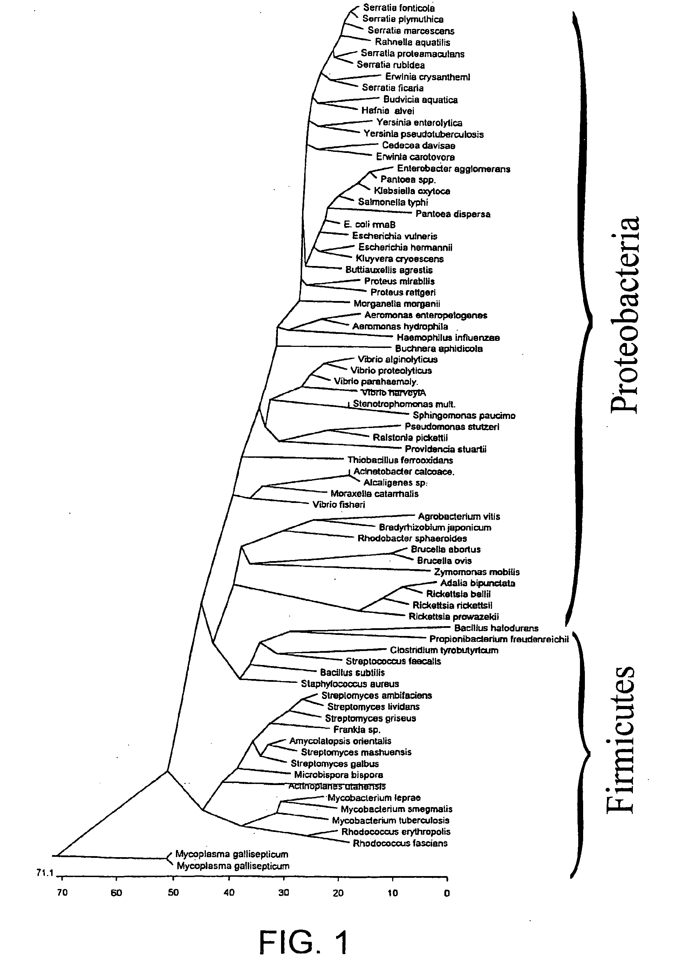

Einen weiteren Hinweis über die Nutzbarkeit der Region gibt auch der phylogenetische Stammbaum aus Abbildung 1. Es ist zu erkennen, daß die 23 S rDNA-5 S rDNA-Region bezüglich der groben Klassifizierung ein sehr gute Differenzierung ermöglicht, da Mitglieder der Proteobakterien in 1-2 Gruppen angeordnet werden, während die Firmicutes abgetrennt sind. Des weiteren indiziert die Länge der Zweige auch bei nahe verwandten Spezies, daß sie sich gut voneinander unterscheiden lassen. Dabei ist ein phylogenetisch korrekte Zuordnung der eng Verwandten in dem Stammbaum durchaus unerwünscht, denn dann würden sie in einer eng zusammenliegenden koherenten Gruppe liegen und ließen sich möglicherweise nicht mehr so leicht voneinander unterscheiden.

### Ausführliche Beschreibung der Abbildungen

Abb. 1: Phylogenetischer Stammbaum einiger in dieser Arbeit nachgewiesener Bakterien. Es ist zu erkennen, daß die Proteobakteria und die Firmicutes abtrennbare Zweige bilden.

Abb. 2: Schematische Darstellung des hierin beschriebenen ribosomalen Bereiches bestehend aus dem terminalen Bereich der 23 S rDNA, dem transkribierten Spacer und der 5 S rDNA. Dieser Bereich oder Teile davon werden zum Nachweis von Bakterien verwendet. Eine detaillierte Charakterisierung einzelner Domänen ist Tabelle 6 zu entnehmen.

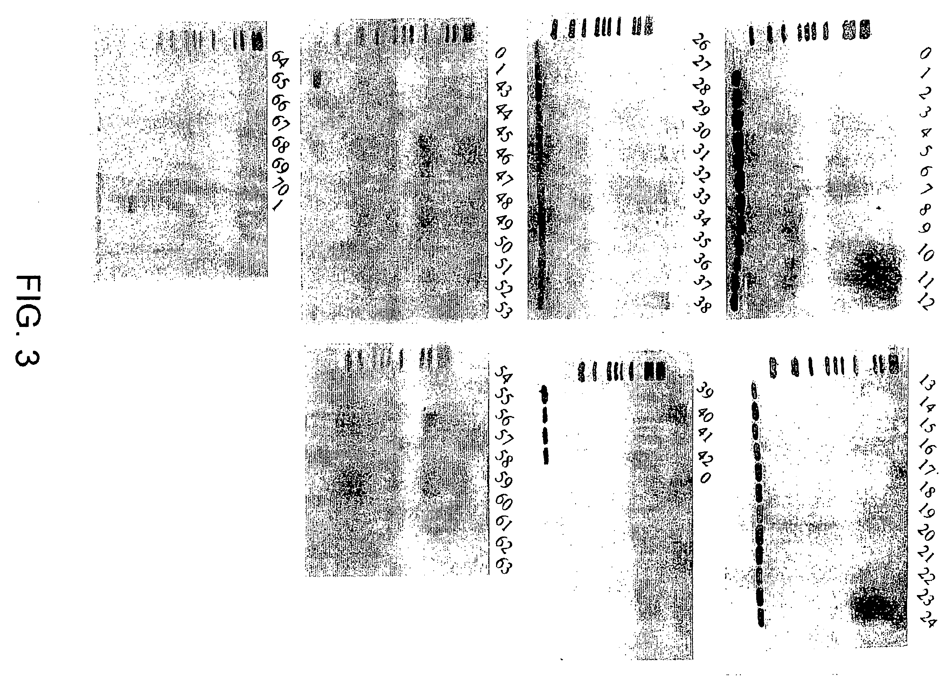

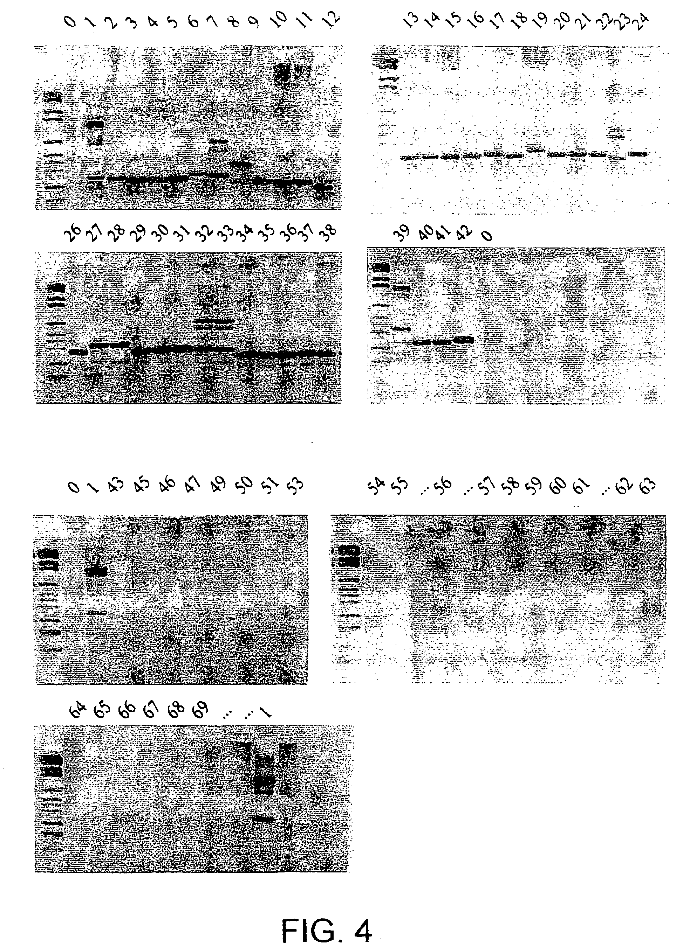

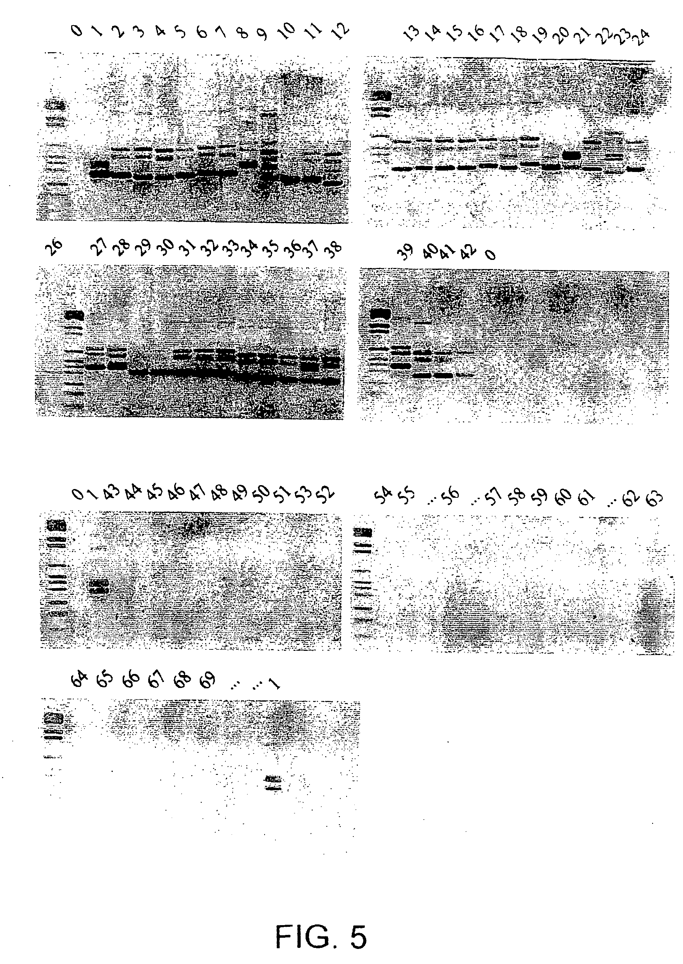

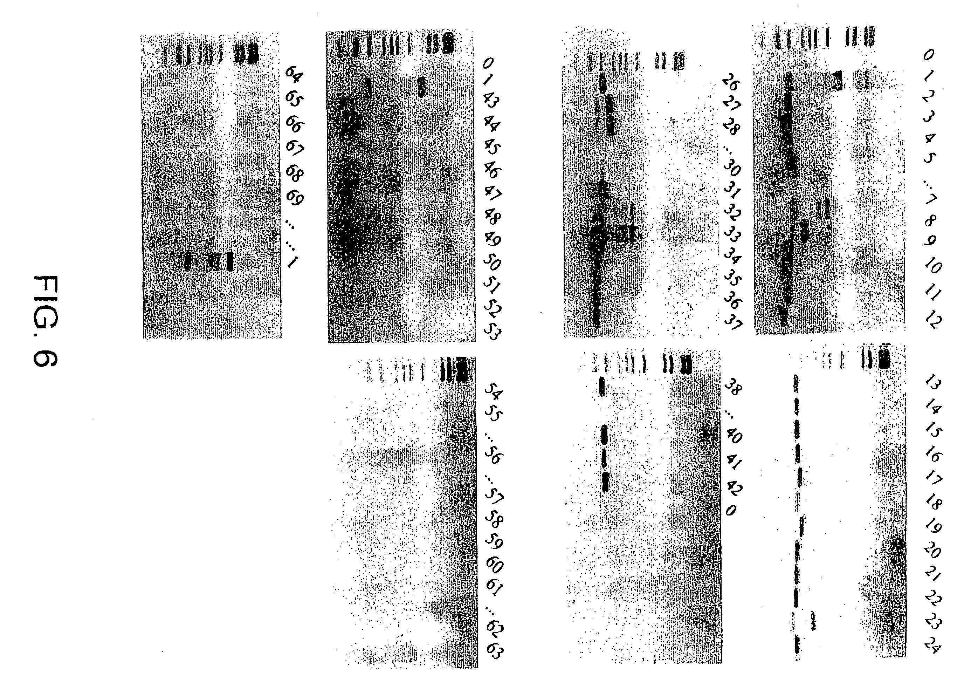

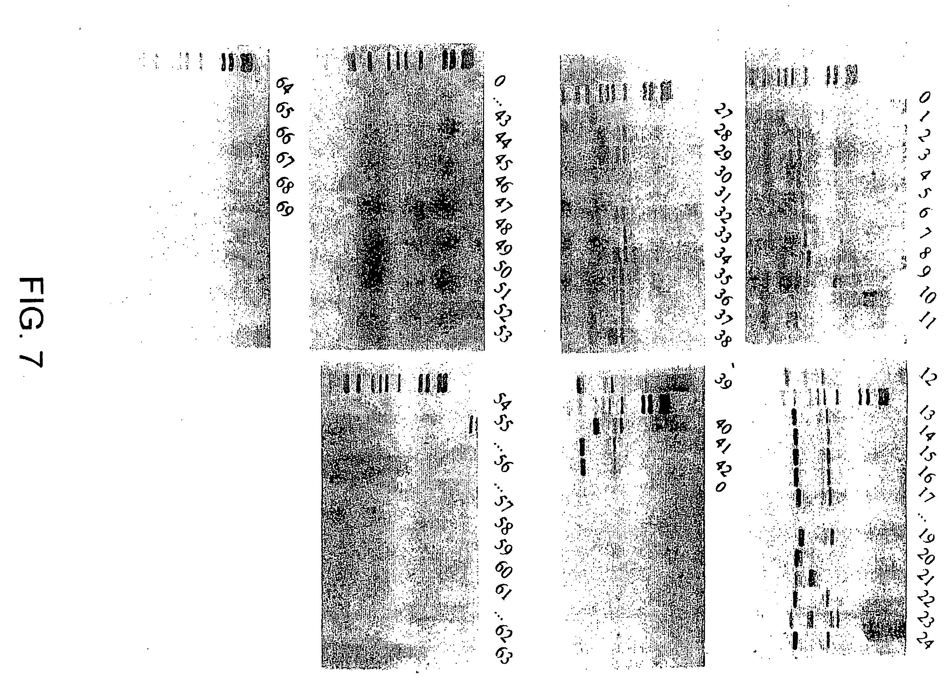

Abb. 3-7: Nachweis der Enterobakterien durch PCR. Gezeigt sind Ethidiumbromidgefärbte Gele. Das Vorhandensein von Banden ist charakteristisch für die Anwesenheit von Enterobakterien. In der oberen Hälfte der Abbildungen befinden sich die positiven Nachweise, in der unteren die Negativkontrollen. Die Verwendung der Primer ist in Tabelle 7 zusammengefaßt. Als DNA-Größenstandard wurde eine Mischung von Bgl 1 und Hinf 1 restriktionsverdauter pBR328-Plasmid-DNA (Boehringer Mannheim) verwendet. Die DNA-Leiter umfaßt die Restriktionsfragmentgrößen 154, 220, 234, 298, 394, 453, 517, 653, 1033, 1230, 1766 und 2176 Basenpaare.

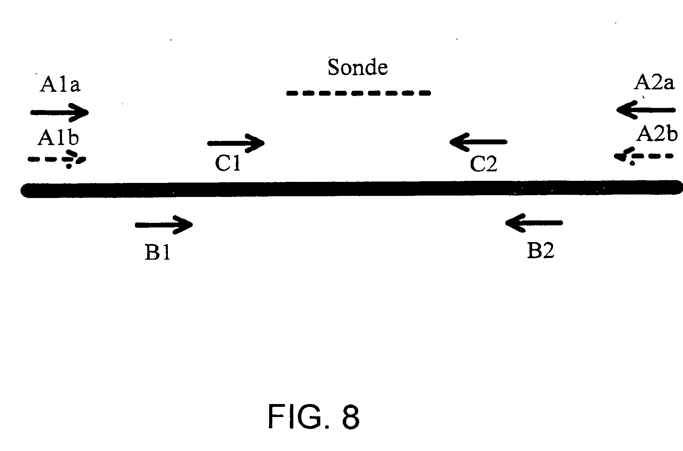

Abb. 8: Schema einer Konsensus-PCR. Konservierte Primer sind peripher angeordet, weniger konservierte liegen verschachtelt intern. Die Konsensus-PCR erlaubt zunächst die Amplifikation von DNA mit hoher taxonomischer Breite, im Extremfall aller bakteriellen Spezies. In nachfolgenden Schritten können weitere Amplifikationsrunden stattfinden, eventuell in separaten Reaktionsgefäßen, mit Primern, die für kleinere taxonomische Einheiten spezifisch sind. Im abschließenden Schritt können Sonden verwendet werden, die ebenfalls zur Spezifität des Nachweises beitragen und außerdem die Detektion, z.B. über Farbstoffe unterstützen können. In dieser Abbildung und hierin gilt folgende Nomenklatur: Primer A = die konserviertesten und peripher gelegenen Primer innerhalb des Nachweissystems, Primer [A, B, C....] = Reihenfolge der Primer innerhalb der Verschachtelung wie oben gezeigt, Primer [Großbuchstabe] 1 = vorwärts-Primer, Primer [Großbuchstabe]2 = rückwärts-Primer, Primer [Großbuchstabe] [Zahl] [Kleinbuchstabe] = die Kleinbuchstaben kennzeichnen ähnliche Primer oder solche, die an homologen oder vergleichbaren Positionen innerhalb einer Ziel-DNA hybridisieren. Die Sonde liegt bevorzugt im zentralen, hochvariablen Bereich wenn Spezies oder Stämme nachgewiesen werden sollen.

### Beispiel 1): Nachweis der Familie der Enterobacteriaceae

Aus Reinkulturen der in Tabelle 1 aufgeführten Bakterien wurde in an sich bekannten Standardverfahren genomische DNA isoliert. Je ca. 1 bis 100 ng dieser Präparationen wurden dann in einer PCR eingesetzt. Der Reaktionsansatz hatte die nachfolgende Zusammensetzung:

| | |
|---|---|
| genomische DNA | - 1 µl |
| H₂O | - 19,8 µl |
| Puffer (10x) *¹ | - 2,5 µl |
| dNTP (10 mM)*² | - 0,25 µl |
| vorwärts-Primer (10 µM)*³ | - 0,20 µl |
| rückwärts-Primer (10 µM)*³ | - 0,20 µl |
| MgCl₂ | - 0,75 µl |
| Taq-Polymerase (5 U/µl)*¹ | - 0,3 µl |

| | |
|---|---|
| *¹ Puffer und Enzym von Biomaster oder einem beliebigen anderen Lieferanten *² Nukleotide von Boehringer Mannheim oder einem beliebigen anderen Lieferanten *³ equimolare Mengen von Primern, falls Gemische in einer Gesamtendkonzentration vorwärts-Primer bzw. rückwärts-Primer von je 10 µM | |

Die PCR wurde in einem Perkin Elmer 9600 Thermocycler mit dem nachfolgend aufgeführten Thermoprofil durchgeführt:

| | | |
|---|---|---|
| initiale Denaturierung | 95 °C | 5 min |
| Amplifikation (35 Zyklen) | 92 °C | 1 min |
| | 62 °C | 1 min |
| | 72 °C | 30 s |
| finale Synthese | 72 °C | 5 min |

Zur Identifikation der Familie der Enterobakterien wurden die in Tabelle 1 aufgelisteten Arten getestet. Die verwendeten Primerkombinationen und primerspezifischen Parameter sind in Tabelle 7 aufgelistet. Soweit mehr als ein vorwärts- oder rückwärts-Primer in Tabelle 7 angegeben ist, soll das jeweilige Gemisch verwendet werden.

Das Resultat der PCR wurde mittels Agarose-Gelelektrophorese und Anfärben mit Ethidiumbromid analysiert. Die Anwesenheit von PCR-Produkten ist indikativ für das Vorhandensein von Enterobakterien.

Die synthetisierten PCR-Produkte liegen meist in einer Größenordnung zwischen 400 und 750 Basenpaaren. Dabei können durchaus mehrere Banden auftreten, weil ribosomale Allele bei vielen bakteriellen Spezies heterogen sind. Die erhaltenen Ergebnisse sind in Tabelle 1 zusammengefaßt und zeigen die umfassende Abgrenzung der Enterobakterien von Representanten anderer Taxa.

### Beispiel 2): Nachweis einer bakteriellen Spezies am Beispiel von Pantoea dispersa

Aus Reinkulturen von Bakterien kann in an sich bekannten Standardverfahren genomische DNA isoliert werden. Je ca. 1 bis 100 ng dieser Präparationen können in einer PCR eingesetzt werden. Der Reaktionsansatz kann folglich die nachfolgende Zusammensetzung haben:

| | |
|---|---|
| genomische DNA | - 1 µl |
| H₂O | - 19,8 µl |
| Puffer (10x) *¹ | - 2,5 µl |
| dNTP (10 mM)*² | - 0,25 µl |
| vorwärts-Primer A (10 MM)*³ | - 0,20 µl |
| rückwärts-Primer (10 mM)*³ | - 0,20 µl |
| MgCl₂ | - 0,75 µl |
| Taq-Polymerase (5 U/gl)*¹ | - 0,3 µl |

| | |
|---|---|
| *¹ Puffer und Enzym von Biomaster *² Nukleotide von Boehringer Mannheim oder einem beliebigen anderen Lieferanten *³ equimolare Mengen von Primern, falls Gemische in einer Gesamtendkonzentration vorwärts-Primer bzw. rückwärts-Primer von je 10 µM. | |

Zum Nachweis von Pantoea dispersa können die Primerkombinationen SEQ ID 2 + Primer x1, SEQ ID (3-6) + Primer x1 oder die komplementäre Sequenz zu Primer x1 + die komplementäre Sequenz zu SEQ ID 147 verwendet werden. Dabei entspricht Primer x1 dem Nucleotid CGTTGCCCCGCTCGCGCCGCTCAGTCAC. Primer x1 ist eine Partialsequenz aus SEQ ID 108.

Die PCR kann in einem Perkin Elmer 9600 Thermocycler mit dem nachfolgend aufgeführten Thermoprofil durchgeführt werden:

| | | |
|---|---|---|
| initiale Denaturierung | 95 °C | 5 min |
| Amplifikation (35 Zyklen) | 92 °C | 1 min |
| | 62 °C | 1 min |
| | 72 °C | 20 s |
| finale Synthese | 72 °C | 5 min |

Das Resultat der PCR kann mittels Agarose-Gelelektrophorese und Anfärben mit Ethidiumbromid sichtbar gemacht werden. Die synthetisierten PCR-Produkte liegen in einer Größenordnung von 370, 320 und 70 Basenpaaren. Die Abwesenheit von Amplifikaten ist indikativ für die Abwesenheit von genomischer DNA von Pantoea dispersa. Mit diesem experimentellen Ansatz lassen sich die in Tabelle 2 zusammengefaßten Ergebnisse erzielen.

### Beispiel 3): Verwenden einer Konsensus-PCR in der Chiptechnologie

### 3a) Prinzip der Konsensus-PCR

In einer Konsensus-PCR, wie schematisch in Abb. 8 gezeigt, werden mindestens zwei sogenannte Konsensus-Primer (A1, A2) verwendet, die in der Lage sind DNA von mindestens zwei taxonomischen Einheiten nachzuweisen. Bei diesen kann es sich um Stämme, Arten oder auch höhere taxonomische Einheiten wie Reiche oder Klassen handeln. In dem Detektionssystem folgt in mindestens einem zweiten Nachweisschritt die Differenzierung der amplifizierten taxonomischen Einheiten mittels einer weiteren PCR und/oder mit Sonden. Die PCR-Primer (B1, B2) des zweiten, bzw. folgenden Amplifikationsschritts werden jeweils so gewählt, daß sie innerhalb des Amplifikationsproduktes liegen und für eine bestimmte taxonomische Einheit ein Nachweispotential haben. Durch Verwendung weiterer Primer (C, D, E....) kann ein Pool vieler taxonomischer Einheiten gegebenenfalls simultan eingeschränkt werden. Außerdem kann in einem Multiplex-Gemisch (z.B. A1a, A1b, A1c, .....) das Nachweispotential auf mehrere taxonomische Einheiten erweitert werden. Letzterer Fall liegt vor, wenn einzelne Nukleotide in einem Primer variieren oder die Primer völlig unterschiedlich sind. Die Nomenklatur der Konsensusprimer ist auch der Legende von Abb. 8 zu entnehmen.

Die Identifizierung von Amplifikationsprodukten kann durch die Primer erfolgen. Ein positiver Nachweis liegt dann vor, wenn die Primer die Ziel-DNA erkennen und erfolgreich amplifizieren. Außerdem können Sonden einen spezifischen Nachweis leisten. Sie hybridisieren spezifisch an die amplifizierte DNA und erlauben durch direkte oder indirekte Koppelung an Farbstoffe den Nachweis einer bestimmten DNA-Sequenz. Alles in allem können Sonden in einer Vielzahl von dem Fachmann bekannten technischen Ausführungen eingesetzt werden. Genannt seien z.B. das Southern Blotting, die LightcyclerTechnologie mit fluoreszierenden Sonden oder die Chiptechnologie, in der beliebig viele Sonden in einem Microarray angeordnet werden.

Besonders vorteilhaft für das Gelingen der Konsensus-PCR ist, daß die Primer in der Reihe A, B, C... zunehmend spezifisch werden. Durch die Auswahl der DNA-Zielregion gemäß Abb. 2 ist dies gewährleistet.

Die Konsensus-PCR hat den Vorteil, daß sie den simultanen Nachweis von mehr als zwei taxonomischen Einheiten aus nur einer Nukleinsäureprobe, die entsprechend klein sein kann, erlaubt. Der Zahl der nachweisbaren Mikroorganismen läßt sich dabei auf verschiedenen Wegen erhöhen. So wächst das Nachweispotential eines Konsensussystems mit der Zahl der Primerspezies A, B, C, ... oder A1a, A1b, A1c, ..., wie sie in Abb. 8 definiert sind. Auch läßt sich ein PCR-Ansatz nach einer ersten Durchführung mit einem Primerpaar A1, A2 trennen und in separaten Ansätzen mit weiteren Primerpaaren B1a + B2a, bzw. B1b + B2b amplifizieren. Schließlich kann die Identität von PCR-Amplifikaten durch Hybridisierung mit Sonden festgestellt werden.

### 3b) Beispiel des Nachweises einer Gruppe von Gattungen der Enterobakterien

Aus Reinkulturen von Bakterien kann in an sich bekannten Standardverfahren genomische DNA isoliert werden. Je ca. 1 bis 100 ng dieser Präparationen können in einer PCR eingesetzt werden. Der Reaktionsansatz kann folglich die nachfolgende Zusammensetzung haben:

| | |
|---|---|
| genomische DNA | - 1 µl |
| H₂O | - 19,8 µl |
| Puffer (10x) *¹ | - 2,5 µl |
| dNTP (10 mM)*² | - 0,25 µl |
| vorwärts-Primer (10 µM)*³ | - 0,20 µl |
| rückwärts-Primer (10 µM)*³ | - 0,20 µl |
| MgCl₂ | - 0,75 µl |
| Taq-Polymerase (5 U/µl)*¹ | - 0,3 µl |

| | |
|---|---|
| *¹ Puffer und Enzym von Biomaster *² Nukleotide von Boehringer Mannheim oder einem beliebigen anderen Lieferanten *³ equimolare Mengen von Primern, falls Gemische in einer Gesamtendkonzentration vorwärts-Primer bzw. rückwärts-Primer von 10 µM. | |

Da in der Chiptechnologie i.d.R. sehr kleine Reaktionsvolumina verwendet werden, kann auch der obige Reaktionsansatz bei konstanten Konzentrationen reduziert werden. Eventuell ist auch ein Anpassung der PCR-Zykluszeiten erforderlich. Für die Konsensus-PCR kann zunächst ein ribosomales DNA-Fragment amplifiziert werden. Dieser Vorgang kann spezifisch für größere taxonomische Einheiten sein, wie in Beispiel 1 beschrieben, wobei die dort beschriebenen Primer verwendet werden. Alternativ kann ein ribosomales DNA-Fragment von allen Bakterien amplifiziert werden. Ribosomale DNA eines sehr breiten taxonomischen Spektrums von Bakterien bietet z.B. die Verwendung der Primerkombination SEQ ID 211 + SEQ ID 212.

Die amplifizierte DNA wird nach Standardmethoden denaturiert und dadurch in einzelsträngige DNA überführt. Diese Form ist dazu geeignet an eine DNA, RNA oder PNA-Sonde zu binden. Entsprechend der Ausführungsform des Chips wird dann die Hybridisierung des Amplifikats mit der Sonde nachgewiesen. Alternativ kann ein Nachweis mittels eines ELISAs erfolgen. Die Sonde ist so beschaffen, daß sie eine den Anforderungen entsprechende Spezifität aufweist. Dementsprechend können Stämme, Gattungen oder größere taxonomische Einheiten nachgewiesen werden.

Tabelle 3 exemplifiziert den Nachweis einer Gruppe von Gattungen der Familie der Enterobakterien unter der Verwendung der Sonde GTTCCGAGATTGGTT als Teilsequenz von SEQ ID 164. Ein solcher Gruppennachweis ist in der Chiptechnologie besonders sinnvoll, wenn verschiedene Gruppennachweise miteinander überschneiden. In der Schnittmenge kann dann der Nachweis einer einzelnen Art oder von Gruppen von Arten, die z.B. für Lebensmitteluntersuchungen relevant sind, erfolgen.

### 3c) Verwendung der Konsensus-PCR zum Nachweis aller Bakterien

Zum Nachweis aller Bakterien werden in einer ersten Amplifikationsrunde Konsensus-Primer verwendet, die stark konserviert sind. Geeignet zur Sequenzauswahl sind Regionen, die peripher in dem ribosomalen Abschnitt gemäß Abb. 2 liegen. Sie ist demzufolge homolog zu den Bereichen der SEQ ID 1 beginnend bei Position 2571 bzw. endend bei Position 3112. Besonders geeignet aus dieser Region für eine generelle Amplifikation sind z.B. die Primer SEQ ID 211 (z.B. als Primer A1a) und SEQ ID 212 (z.B. als Primer A2a). Außerdem lassen sich leicht weitere Primer (A1b, A1c, .... bzw. A2b, A2c, .....) ableiten, die in einer Multiplex-PCR einen beliebig großen taxonomischen Bereich der Eubacteria erfassen. In dieser Nomenklatur sind die Primer A1 und A2 Primerpaare, B und C...... verschachtelte Primer bzw. A1a und A1b homologe oder ähnliche Primer.

Durch die Verwendung verschachtelter Primer (B, C, D, .....), kann eine erste Differenzierung erfolgen. Dieses kann zudem unterstützt werden, indem der primäre PCR-Ansatz geteilt wird, wobei dann in jedem separaten PCR-Ansatz z.B. ein Primerpaar B, bzw. C bzw. D usw. eingesetzt wird. Diese Verschachtelung ist deshalb besonders vorteilhaft, weil der ribosomale Bereich gemäß Abb. 8 von außen nach Innen zunehmend variabel ist, wie es auch in Tab. 6 beschrieben ist. Sonden können dann vorzugsweise zur abschließenden Differenzierung und Identifizierung genutzt werden. Wenn z.B. Stämme oder Arten nachgewiesen werden sollen, dann sollte die Sonde zentral in Bereich 7 gemäß Abb. 2 hybridisieren.

In Tabelle 8 werden viele Polynukleotide zum Nachweis von Gattungen und Arten oder Stämmen in einer Konsensus-PCR zur Verfügung gestellt. Die Verwendung der Primer Nr. 1 aus Tabelle 8 wurde bereits ausführlich in Beispiel 1 beschrieben.

Die Polynukleotide folgen in ihren Eigenschaften der Charakterisierung aus Tabelle 6, bzw. Abb. 2. Das bedeutet, daß Primer A1 dem Bereich 1 aus Tabelle 6, bzw. Abb. 2 zugeordnet werden können, Primer A2 dem Bereich 2 ..... , Primer B2 dem Bereich 8 und Primer A2 dem Bereich 9. Entsprechend diesem Konzept können die Primer A1-G1 aus Tabelle 8 als Vorwärtsprimer verwendet werden während die Primer B2 und A2 als Rückwärtsprimer genutzt werden können. Die Sequenzen für die letzteren beiden Primertypen müssen zu diesem Zweck konvertiert werden (Ausnahme Nr. 1 Tabelle 8). Als art- oder gattungspezifische Sonden lassen sich besonders die "Primer H1" verwenden.

Das hierzu beschriebene Schema einer Konsensus-PCR ist nicht zwingend notwendig für einen erfolgreichen Nachweis. Im Prinzip können die in Tabelle 8 aufgelisteten Polynukleotide in jeder beliebigen Kombination eingesetzt werden. In der Praxis ist zunächst zu klären, welche Bakterien als "unerwünscht" im Nachweis ausgegrenzt werden sollen. Je nach Problemstellung kann dann abweichend von dem gezeigten Schema eine einfachere PCR-Version gewählt werden. Die einfachste Form der Konsensus-PCR besteht dementsprechend aus nur zwei Primern entsprechend den Sequenzen aus Tabelle 8, bzw. komplementären Sequenzen dazu.

Viele der in Tabelle 8 aufgelisteten konservierten Primer haben das Potential die DNA von höheren taxonomischen Einheiten, wie Klassen, Abteilungen oder Familien nachzuweisen. Wie Tabelle 6 zu entnehmen ist trifft dies insbesondere auf die peripheren Primer A oder homologe Sequenzen von SEQ ID 211 + SEQ ID 212 zu. In Tabelle 8 wird ein breiteres Nachweispotential für eine oder mehrere Gattungen oder Arten insbesondere durch die redundante Aufzählung der Sequenzen angezeigt. Falls nur eine Sequenz für eine Gattung explizit aufgezählt wird, so können zum Nachweis zwei Primer aus dieser Sequenz gewählt werden. Auch ist es möglich allgemeine Primer, z.B. Primer A von verwandten Gattungen, für die betreffende Bakterienklasse zu wählen und aus einer spezifischen Sequenz, z.B. "Primer hl" eine Sonde zu entwerfen. Soweit die Sequenzen sehr lang sind können Nukleotidfragmente von mindestens 15 Basen Länge aus diesen gewählt werden.

### 3d) Ausführung der Konsensus-PCR für die Chiptechnologie

Die konkrete Ausführung der Konsensus-PCR wird im wesentlichen bestimmt durch die erwartete Zahl der nachzuweisenden taxonomischen Einheiten. Da die Konsensus-PCR in ihrer komplexesten Form auch eine Multiplex-PCR darstellt, kann in einem Reaktionsansatz nur eine limitierte Zahl von Bakterien bestimmt werden. Erfahrungsgemäß liegt diese Zahl unter 20. Aus diesem Grunde kann es vorteilhaft sein verschiedene PCR-Ansätze mit der gleichen Probe und unterschiedlichen Primern A, B etc. (Nomenklatur nach Abb. 8) durchzuführen.

Aus natürlichen Proben werden Bakterien zunächst angereichert oder mit an sich bekannten Standardverfahren wird genomische DNA direkt aus ihnen isoliert. Je ca. 1 bis 100 ng dieser Präparationen können in einer PCR eingesetzt werden. Der Reaktionsansatz kann folglich die nachfolgende Zusammensetzung haben:

| | |
|---|---|
| genomische DNA | - 1 µl |
| H₂O | - 19,8 µl |
| Puffer (10x) *¹ | - 2,5 µl |
| dNTP (10 mM)*² | - 0,25 µl |
| forwärts-Primer (10 mM)*³ | - 0,20 µl |
| rückwärts-Primer (10 mM)*³ | - 0,20 µl |
| MgCl₂ | - 0,75 µl |
| Taq-Polymerase (5 U/µl)*¹ | - 0,30 µl |

| | |
|---|---|
| *¹ Puffer und Enzym von Biomaster *² Nukleotide von Boehringer Mannheim oder einem beliebigen anderen Lieferanten *³ equimolare Mengen von Primern, falls Gemische in einer Gesamtendkonzentration der Primer 10 µM. Primer können z.B. entworfen und kombiniert werden wie unter 3c beschrieben. | |

Da in der Chiptechnologie i.d.R. sehr kleine Reaktionsvolumina verwendet werden, kann auch der obige Reaktionsansatz bei konstanten Konzentrationen reduziert werden. Eventuell ist auch ein Anpassung der PCR-Zykluszeiten erforderlich.

Nach den Amplifikationsrunden wird die DNA vereinigt. Auf einem Chip werden Sonden, in einer spezifischen Ausführungsform aus den Sequenzen der Spalte "Primer H1" der Tabelle 8 ausgewählt werden können, immobilisiert. Technische Verfahren hierzu sind dem Fachmann bekannt. Die vereinigte DNA wird 1:1 mit Denaturierungspuffer (Bsp. 4) verdünnt und eine Stunde bei Raumtemperatur inkubiert. Anschließend wird das zehnfache Volumen Hybridisierungspuffer (Bsp. 4) dazugegeben und die Lösung langsam bei 37-60° C über den Chip, das heißt die mit Sonden behaftete Oberfläche, geleitet. Die so behandelte Chipoberfläche wird anschließend mit Waschpuffer (Bsp. 4) mindestens 2 min bei 37-60° C dreimal gewaschen. Abschließend kann die Detektion erfolgen. Hierzu können Primer verwendet werden, die mit einem Fluoreszensfarbstoff gekoppelt sind. Die Fluoreszenz kann dann mit einem Detektor, z.B. einer CCD-Kamera wahrgenommen werden. Es gibt jedoch viele verschiedene alternative Detektionsmöglichkeiten. So ist es z.B. auch möglich die Bindung der einzelsträngigen Amplifikatationsprodukte an die Sonden durch Oberflächen-Plasmon-Resonanz-Spektroskopie (SPR) zu verfolgen und zu quantifizieren. Letztere Methode hat den Vorteil, daß keine Farbstoffe zur Detektion verwendet werden müssen. Bei Nutzung der SPR sollte selbige so ausgelegt sein, daß die Detektion simultan auf den Bereichen der Oberfläche erfolgt, die die gleichen Sonden besitzen. Eine besonders vorteilhafte Ausführung liegt dann vor, wenn viele, d.h. mehr als 100 oder 1000 separate Detektionsflächen auf dem Chip angeordnet sind. Ein Ansteigen des SPR-Signals, verursacht duch die Nukleinsäure-Hybridisierung auf diesen Flächen, stellt ein positives Ergebnis dar. Auf diese Weise lassen sich mit den in Tabelle 8 aufgelisteten Primern die dazugehörigen Bakterien, oder im Prinzip alle Bakterien nachweisen und gegebenenfalls quantifizieren.

### Beispiel 4) Nachweis von Mikroorganismen mit Sonden

Sonden sind als Polynukleotide, d.h. als DNA, RNA, PNA oder in einer ähnlichen Ausführungsform, die dem Fachmann geläufig ist, grundsätzlich geeignet die Konzentrierung und den Nachweis von DNA oder RNA zu leisten. Sie liegen als einzelsträngige Moleküle vor oder werden durch Denaturieren, was z.B. durch Erhitzen oder mit Natronlauge entsprechend publizierten Standartprotokollen erreicht werden kann, in diese Form überführt.

Zum Nachweis von Mikroorganismen muß die DNA oder RNA derselben aus diesen isoliert und eventuell gereinigt werden. Eine hohe Effizienz der Nukleinsäureausbeute kann durch verschiedene Maßnahmen erreicht werden:
1) die Mikroorganismen können mit physikalischen Methoden, z.B. mit an magnetischen Partikeln gekoppelten Antikörpern oder durch Zentrifugieren ankonzentriert werden;
2) die DNA oder RNA der Mikroorganismen kann in einer PCR oder vergleichbaren Amplifikationsrekation amplifiziert werden;
3) die eventuell amplifizierte DNA oder RNA der Mikroorganismen wird im Verlaufe der Reinigung mit kommerziell erhältlichem Material beim Aufreinigen ankonzentriert.

Die Verbesserung der Effizienz der Nukleinsäureausbeute, insbesondere durch Amplifikation, kann selbst bedeutend zur Spezifität des Nachweises der Bakterien beitragen.

Es folgt anschließend ein Inkubationschritt, in welchem die Sonden mit den nachzuweisenden Nukleinsäuren (sofern die nachzuweisenden Mikroorganismen vorhanden waren) ein Hybridmolekül bilden. Die Bildung des Hybridmoleküls erfolgt unter kontrollierten Bedingungen. Ebenso folgen Waschschritte mit Puffern unter Bedingungen (pH-Wert, Temperatur, Ionenkonzentration), die die spezifische Hybridisierung von Nukleinsäuren erlauben, während weniger spezifische und unerwünschte Hybridmoleküle dissoziieren. Abschließend erfolgt die Detektion von Hybridmolekülen. Zur Detektion gibt es eine Vielzahl von Ausführungsformen, die dem Fachmann im Einzelnen bekannt sind. Zum Einsatz kommen Farbstoffe, eventuell fluoreszierende, die direkt oder indirekt an die Sonden oder die nachzuweisende DNA gekoppelt werden oder in diese inkorporiert werden. Dieses kann insbesondere auch in der Chiptechnologie oder der Lightcyclertechnologie der Fall sein. Darüber hinaus gibt es andere physikalische Verfahren, wie z.B. verstärkte Totalreflektion des Lichts (attenuated total reflection) an Grenzoberflächen mit zwei unterschiedlichen Dichten, die zur Anwendung bei der Detektion der Hybridmoleküle kommen können.

Die Auswertung der Detektion kann auf verschiedene Weisen erfolgen. In einem "alles oder nichts"-Nachweis kann das Hybridmolekül nur nachgewiesen werden, wenn die gesuchten Mikroorganismen vorhanden waren. Wenn also die zuvor erwähnte Amplifikationsreaktion mit den Nukleinsäuren der Mikroorganismen zu keiner Vermehrung der Nukleinsäuren geführt hat, dann werden auch keine Hybridmoleküle nachweisbar sein. Wurden jedoch "unerwünschte" Nukleinsäuren amplifiziert oder waren diese in großer Menge vorhanden, dann kann durch die Stringenzbedingungen bei der Hybridisierung ein Ausschluß dieser Nukleinsäuren erfolgen. Außerdem ermöglicht die Quantifizierung der Hybridmoleküle eine Feinabstimmung der Spezifität des Nachweises, indem ein Grenzwert für den positiven Nachweis festgelegt wird.
- Alle in diesem Patent spezifizierten Nukleinsäuren sind grundsätzlich als Sonde verwendbar. Ein Extrakt möglicher Sonden ist insbesondere in Tabelle 3 aufgelistet. Die Nukleinsäuren leisten den Nachweis der in der Tabelle genannten Gattungen und die Abgrenzung gegen alle anderen Gattungen der Eubakterien.

Nachfolgend sei exemplifiziert wie die hierzu spezifizierten DNA-Bereiche als Sonden zum Nachweis von Mikroorganismen eingesetzt werden können. In diesem Beispiel wird ein ELISA-Verfahren zur Detektion verwendet. Dabei werden Nukleinsäuren mittels einer enzymatischen Farbreaktion, die in Mikrotiterplatten abläuft, nachgewiesen.

Im vorliegenden Beispiel wird die DNA zunächst in einer PCR-Reaktion amplifiziert. Dabei werden Primer verwendet, die mit Digoxygenin gekoppelt sind. Anschließend wird eine mit Streptavidin beschichtete Mikrotiterplatte mit einer biotinmarkierten Sonde beladen, so daß es zu einer Kopplung der Sonden an die Plattenoberfläche kommt. Die alkalisch denaturierten PCR-Amplifikate hybridisieren in einer 30-minütigen Reaktion mit den Sonden. Das 5'-Digoxigenin markierte Ende der Amplifikate dient nun als Antigen für einen spezifischen Antikörper, welcher wiederum an das Enzym Peroxidase gekoppelt ist. Nach Zugabe von Tetramethylbenzidin kommt es zur Bildung eines blauen Farbstoffs. Die Entstehung dieses Farbstoffes wird mit 0,5 molarer Schwefelsäure gestoppt. Gleichzeitig schlägt die Farbe aufgrund der Verschiebung des pH-Wertes nach gelb um. Die Intensität der Absorption wird im ELISA-Reader bei 450 nm gemessen.

Zur Durchführung des ELISA werden die nachfolgenden Reagenzien angesetzt:
- Hybridisierungspuffer (2,5x SSC)

| | |
|---|---|
| 2,5 x SSC | 62,5 ml von 20 x SSC (s. unten) |
| 2 x Denhardts | 20 ml von 50 x Denhardts (s. unten) |
| 10 mM Tris (Gibco, Nr. 15504-038) | 5 ml von 1 M Tris |
| 1 mM EDTA (Fluka, Nr. 03699) | 1 ml von 0,5 M EDTA |
| mit bidest. Wasser auf 0,51 auffüllen und pH 7,5 einstellen | |

- Waschpuffer 1

| | |
|---|---|
| 1 x SSC | 50 ml von 20 x SSC (s. unten) |
| 2 x Denhardts | 40 ml von 50 x Denhardts (s. unten) |
| 10 mM Tris (Gibco, Nr. 15504-038) | 10 ml von 1 M Tris |
| 1 mM EDTA (Fluka, Nr. 03699) | 2 ml von 0,5 M EDTA |
| mit bidest. Wasser auf 11 auffüllen und pH 7,5 einstellen | |

- Waschpuffer 2

| | |
|---|---|
| 100 mM Tris (Gibco, Nr. 15504-038) | 12,15 g |
| 150 mM NaCl (Merck, Nr. 6404.5000) | 8,78 g |
| 0,05% Tween 20 (Serva, Nr. 37470) | 0,5 g |

0,5% Blocking Reagenz (Boehringer) 5g in D1 (s. unten) bei 60°C lösen 10 µg/ml Heringsperma 10 ml von der Stammlösung mit 10mg/ml mit bidest. Wasser auf 11 auffüllen und pH 7,5 einstellen
- Denaturierungspuffer

| | |
|---|---|
| 125 mM NaOH (Fluka, Nr. 71690) | 0,5 g |
| 20 mM EDTA (Fluka, Nr. 03699) | 0,745 g |
| mit bidest. Wasser auf 0,11 auffüllen | |

- Kopplungspuffer

| | |
|---|---|
| 10 mM Tris (Gibco, Nr. 15504-038) | 10 ml von 1 M Tris |
| 1 mM EDTA (Fluka, Nr. 03699) | 2 ml von 0,5 M EDTA |
| 100 mM NaCl (Merck, Nr. 6404.5000) | 5,88 g |
| 0,15% Triton X 100 (Chemikalienlager) | 15 ml |
| mit bidest. Wasser auf 11 auffüllen und pH 7,5 einstellen | |

- Stoppreagenz (0,5M H₂SO₄)

| | |
|---|---|
| 95%ige H₂SO₄ | 14 ml |
| mit bidest. Wasser auf 0,51 auffüllen | |

- 50 x Denhardts

| | |
|---|---|
| Ficoll 400 (Pharmacia Biotech, Nr. 17-0400-01) | 5 g |
| Polyvinylpyrrolidon (Sigma, Nr. P-2307) | 5 g |
| Rinder-Serumalbumin | 5 g |
| mit bidest. Wasser auf 0,51 auffüllen | |

- 20 x SSC

| | |
|---|---|
| NaCl (Merck, Nr. 106404.1000) | 350,36 g |
| Natriumcitrat (Trinatriumcitratdihydrat, Fluka, Nr. 71404) | 176,29 g |
| mit bidest. Wasser auf 21 auffüllen und pH 7,0 einstellen | |

- D1

| | |
|---|---|
| 100 mM Maleinsäure (Fluka, Nr. 63190) | 11,62 g |
| 150 mM NaCl (Merck, Nr. 106404.1000) | 8,76 g |
| NaOH (Fluka, Nr. 71690) ca. | 7,5 g |
| mit bidest. Wasser auf 2 auffüllen und pH 7,0 einstellen | |

### Durchführung des ELISA:

Pro Kavität werden 200 µl Bindungspuffer und 1 µl Sonde aufgetragen. Die Mikrotiterplatte wird mit einer Klebefolie abgedeckt und zwei Stunden bei Raumtemperatur stehen gelassen. Die zu untersuchenden PCR-Amplifikate werden bei Raumtemperatur aufgetaut und im Verhältnis 1:1 mit Denaturierungspuffer versetzt und 10 min bei Raumtemperatur inkubiert. Anschließend werden 10 ml dieser Probe in die zwischenzeitlich entleerten Kavitäten gefüllt. Zusätzlich werden in jede Kavität je 100 µl Hybridisierungspuffer gegeben und 30 min bei 37-60° C inkubiert. Zum Waschen werden die Kavitäten entleert und mit 200 ml Waschpuffer 1, der auf 37-60° C vorgeheizt wurde, gefüllt und 2 min bei der gleichen Temperatur inkubiert. Dieser Waschschritt wird dreimal durchgeführt.

Nachdem der Waschpuffer sorgfältig entfernt wurde, wird der Anti-Dig-POD-Antikörper (DAKO) 1:3000 verdünnt (1 ml in 3 ml Waschpuffer 2) und jeweils 100 ml dieser Lösung in die trockenen Kavitäten gefüllt. Diese Anordung wird bei 37°C im Brutschrank für 30 Minuten inkubiert.

Anschließend wird die Mikrotiterplatte dreimal mit 200 ml Waschpuffer 2 je Vertiefung gewaschen. Pro Kavität werden nun 100 ml des Farbstoffs BM blue (Boehringer) hinzugegeben. Nach 15 min wird die Reaktion durch Zugabe von 100 ml 0,5 M H₂SO₄ gestoppt. Die Extinktion der Proben wird im ELISA-Reader quantifiziert.

In dem oben beschriebenen Verfahren lassen sich die in Tabelle 4 zusammengefaßten Sonden zum Nachweis der aufgeführten Arten verwenden.

### Beispiel 5): Allgemeine Nutzbarkeit der in diesem Patent spezifizierten DNA-Regionen zum Nachweis von Bakterien

Die hierin spezifizierten ribosomalen DNA-Regionen, insbesondere wenn sie mit dem 23S-5S ribosomalen Spacer kombiniert werden, sind geeignet Eubakterien nachzuweisen. Der Fachmann ist in der Lage sehr schnell mit Hilfe der Sequenzen unter SEQ ID 1-530 oder unter Fokussierung auf die genannte ribosomale DNA-Region bakterielle taxonomische Einheiten seiner Wahl schnell zu identifizieren. Nachfolgend ist ein möglicher Weg exemplifiziert, der die generelle Nutzbarkeit für alle eubakteriellen Spezies eröffnet.

Der hier beschriebene Weg umfaßt im wesentlichen 3 Schritte. Im ersten wird eine ribosomale Region, ungefähr umfassend die letzten 330-430 Nukleotide des 23S-Gens, der nachfolgend transkribierten Spacer und das ribosomale 5S-Gen amplifiziert. Da diese Region bei den verschiedenen eubakteriellen Spezies längenvariabel ist, hat sie eine Ausdehnung von insgesamt 400 bis ca. 750 Nukleotiden. Soweit die DNA-Sequenz noch nicht bekannt ist, kann es vorteilhaft sein, diese für die nachzuweisende und einige nahe verwandte abzugrenzende Spezies zu bestimmen. Aus einem Sequenzvergleich kann der Fachmann leicht die besten Oligonukleotide bestimmen, die den gewünschten Nachweis, z.B. als PCR-Primer oder Sonde leisten. Im vorliegenden Beispiel werden auf diese Weise sowohl Primer als auch Sonden ausgewählt. Alternativ können auch die hierin genannten Sequenzen direkt für ein breites Spektrum von Bakterien genutzt werden, insbesondere wenn die Stringenzbedingungen der PCR und/oder der Hybridisierung geeignet gewählt werden.

### A) Amplifikation ribosomaler DNA

Der zu verwendende DNA-Abschnitt läßt sich aus genomischer bakterieller DNA der Proteobakterien und vieler anderer bakterieller Klassen mit den Primern SEQ ID 211 und 212 amplifizieren. Sollte es bei der Amplifikation von DNA anderer Klassen Probleme geben, so werden Primer, die aus DNA-Regionen, welche der SEQ ID 211 und 212 entsprechen, abgeleitet sind, zum Erfolg führen.

Aus Reinkulturen der in Tabelle 5 aufgeführten Bakterien wird in an sich bekannten Standardverfahren genomische DNA isoliert. Je ca. 1 bis 100 ng dieser Präparationen wird dann in eine PCR eingesetzt. Der Reaktionsansatz hat die nachfolgende Zusammensetzung:

| | |
|---|---|
| genomische DNA | - 1 µl |
| H₂O | - 19,8 µl |
| Puffer (10x) *¹ | - 2,5 µl |
| dNTP (10 MM)*² | - 0,25 µl |
| vorwärts-Primer (10 mM)*³ | - 0,20 µl |
| rückwärts-Primer (10 mM)*³ | - 0,20 µl |
| MgCl₂ | - 0,75 µl |
| Taq-Polymerase (5 U/gl)*¹ | - 0,3 µl |

| | |
|---|---|
| *¹ Puffer und Enzym von Biomaster oder einem beliebigen anderen Lieferanten *² Nukleotide von Boehringer Mannheim oder einem beliebigen anderen Lieferanten *³ equimolare Mengen von Primern, falls Gemische in einer Gesamtendkonzentration vorwärts-Primer bzw. rückwärts-Primer von je 10 µM | |

Die PCR wird in einem Perkin Elmer 9600 Thermocycler mit dem nachfolgend aufgeführten Thermoprofil durchgeführt:

| | | |
|---|---|---|
| initiale Denaturierung | 95 °C | 5 min |
| Amplifikation (35 Zyklen) | 92 °C | 1 min |
| | 52 °C | 1 min |
| | 72 °C | 30 s |
| finale Synthese | 72 °C | 5 min |

Genomische DNA, die zur Amplifikation verwendet werden kann, ist in Tabelle 5 beispielhaft aufgelistet.

### B) Gattungs- und speziesspezifische Amplifikation eines Unterbereichs des Produkts von A)

Das unter A) amplifizierte DNA-Produkt kann direkt zum Nachweis von Bakterien, insbesondere unter Verwendung spezifischer Sonden, genutzt werden. Es kann vorteilhaft sein, einen Teilbereich dieser Sequenz primär zu amplifizieren, wenn durch diesen Vorgang eine Beschränkung auf eine kleinere systematische Einheit der Bakterien, wie Arten, Gattungen oder Familien erreicht werden soll. Zumindest ein Teil der Differenzierungsleistung kann dann bereits durch die Amplifikationsprimer erbracht werden. Der unter A) amplifizierte Bereich bietet eine Vielzahl von Subregionen, die spezifische Differenzierungsleistungen erbringen. Der Fachmann wird leicht diese Regionen durch einen Vergleich der Sequenzen von zu identifizierenden Bakterien mit nahe verwandten Bakterien erkennen.

Im vorliegenden Beispiel wurden als Regionen für spezifische Primer der Beginn des 23S-5S transkribierten Spacers und das Ende desselben ausgewählt. Die konkreten Sequenzen und die Herkunft der Primer ist in Tabelle 5 zusammengefaßt. Aus einem Vergleich der Sequenzen ist zu erkennen, daß sie im wesentlichen einen speziesspezifischen Nachweis leisten. Eine Ausnahme bilden die Primer für die Vibrio-Spezies, die auch schon einen gattungsspezifischen Nachweis erlauben. In den vorwärts-Primern ist insbesondere für Enterobakterien die Sequenz CGAAG...TTTT und in den rückwärts-Primern die Sequenz AACAGAATTT konserviert. Es gibt nun zwei Möglichkeiten die Spezifität der Primer auf Gattungen und Gruppen von Gattungen, z.B. aus den Enterobakterien, zu erweitern: erstens können die Annealingtemperaturen der PCR erniedrigt werden. Zweitens können die Sequenzen der vorwärts-Primer in Richtung 23S-Gen und der rückwärtsPrimer in Richtung 5S-Gen verschoben werden. Das Resultat sind Primer, deren Sequenzen weniger speziesvariabel sind. Die konkrete Ausführung kann dabei an die Anforderungen des Nachweises ausgerichtet werden. Hier sei der speziesspezifische Nachweis mit den Primern der Tabelle 5 durch PCR-Amplifikation exemplifiziert.

Aus Reinkulturen der in Tabelle 5 aufgeführten Bakterien wird in an sich bekannten Standardverfahren genomische DNA isoliert. Je ca. 1 bis 100 ng dieser Präparationen wird dann in eine PCR eingesetzt. Der Reaktionsansatz hat die nachfolgende Zusammensetzung:

| | |
|---|---|
| genomische DNA | - 1 µl |
| H₂O | - 19,8 µl |
| Puffer (10x)*¹ | - 2,5 µl |
| dNTP (10 mM)*² | - 0,25 µl |
| vorwärts-Primer (10 mM)*³ | - 0,20 µl |
| rückwärts-Primer* (10 mM)*³ | - 0,20 µl |
| MgCl₂ | - 0,75 µl |
| Taq-Polymerase (5 U/ml)*¹ | - 0,3 µl |

| | |
|---|---|
| *¹ Puffer und Enzym von Biomaster oder einem beliebigen anderen Lieferanten *² Nukleotide von Boehringer Mannheim oder einem beliebigen anderen Lieferanten *³ vorwärts-Primer A und rückwärts-Primer* sind in Tabelle 5 aufgelistet, equimolare Mengen von Primern, falls Gemische in einer Gesamtendkonzentration der Primer von je 10 zum; rückwärts-Primer* weisen die komplementäre Sequenz zu rückwärts-Primern nach Tabelle 5 auf | |

Die PCR wird in einem Perkin Elmer 9600 Thermocycler mit dem nachfolgend aufgeführten Thermoprofil durchgeführt:

| | | |
|---|---|---|
| initiale Denaturierung | 95 °C | 5 min |
| Amplifikation (35 Zyklen) | 92 °C | 1 min |
| | * 45-72 °C | 1 min |
| | 72°C | 30 s |
| finale Synthese | 72 °C | 5 min |

| | | |
|---|---|---|
| * Die Annealingtemperatur kann nach den allgemein verwendeten Formeln für PCR-Primer bestimmt werden. | | |

Das Ergebnis der Amplifikation ist in Tabelle 5 aufgelistet, d.h. der speziesspezifische Nachweis von Bakterien unter Verwendung der Primer der Tabelle 5 führt zur Identifikation der den Primern in dieser Tabelle zugeordneten Bakterien. Die Verwendung von allgemeineren Primern hingegen, deren Entwurf zuvor beschrieben wurde, kann zum Nachweis aller enterobakteriellen Gattungen oder oder zum Nachweis aller Gattungen des γ-Zweiges der Proteobakterien führen.

### C) Weitere Spezifizierung des Nachweises durch Verwendung von Primern oder Sonden aus dem 23S-5S ribosomalen Spacer

Soweit nach den Schritten A) und/oder B) eine Amplifikation von DNA höherer taxonomischer Einheiten erfolgte kann anschließend durch die Auswahl von Sonden eine weitere Differenzierung des Nachweises erfolgen. Zum artspezifischen Nachweis kann ein variabler DNA-Bereich, z.B. ein zentraler Bereich des 23S-5S transkribierten Spacers verwendet werden. Die Sonden können dabei z.B. in einen Chip integriert sein oder im Rahmen der Lightcyclertechnologie oder in einem ELISA verwendet werden. In letzterem Fall kann das ELISA-Protokoll von Beispiel 4 Anwendung finden. Die Resultate des speziesspezifischen Nachweises von Baktieren entsprechen dabei der Auswahl des 23S-5S transkribierten Spacers, da dieser zum Großteil speziesspezifische Sequenzbereiche aufweist. Bei Verwendung der Primer aus Tabelle 5 und Nutzung der entsprechenden Spacer (Spalte SEQ ID aus Tabelle 5) ist somit die Identifikation der in dieser Tabelle aufgelisteten Arten zu erreichen.

### Erläuterungen verwendeter Begriffe:

### Ableiten von DNA-Sequenzen:

Um ein Poly- oder Oligonukleotid, das zum Nachweis von taxonomischen Einheiten verwendet werden soll, zu finden und zu entwickeln, kann es von einer oder mehreren DNA-Sequenzen abgeleitet werden. Im Fall vom mehreren Sequenzen ist dabei ein Allignment der Sequenzen, also ein Vergleich, vorteilhaft. Abgeleitete Oligonukleotide können zur ursprünglichen Sequenz identisch sein. Sie können außerdem einen Konsensus aus einer Menge von Variablen darstellen. In diesem Fall werden die Nukleotide des Polymers entsprechend den häufigsten oder vorherrschenden Bausteinen an einer bestimmten Position der analysierten Sequenzen ausgewählt. Außerdem ist es möglich in einer zu entwickelnden Sequenz Variablen gemäß der Definition "Nukleotide" zu wählen. Die aus diesen variablen Sequenzen resultierenden DNA- oder RNA-Polymere stellen folglich ein Gemisch von Molekülen dar, das an den Positionen der Variablen alle erlaubten Nukleotide aufweist.

### Analoge DNA-Sequenzen:

Analoge DNA-Bereiche haben die gleiche Funktion oder eine ähnliche Lokalisation wie eine vorgegebene Sequenz, sind jedoch nicht auf den gleichen phylogenetischen Ursprung zurückzuführen. Ein Beispiel ist gegeben mit dem transkribierten Spacer zwischen 5 S rDNA und 23 S rDNA, wenn er keine Ähnlichkeit mit einem zu vergleichenenden transkribierten Spacer gleicher Lokalisation aufweist. Das ist möglich, weil er bei entfernt verwandten Organismen häufig so variabel ist, daß eine stammesgeschichtliche Abstammung oder Homologie nicht mehr feststellbar ist. Der obige transkribierte Spacer ist jedoch als DNA-Sequenz und in seiner Funktion als transkribierter Spacer oder in seiner Lokalisation eindeutig definierbar, da er am Ende des kodierenden Bereiches der 23 S rDNA beginnt und am Anfang der 5 S rDNA endet.

### Benachbarte Gene:

Gene sind benachbart, wenn sie durch kein anderes Gen getrennt sind oder wenn dieses bei zwei bestimmten Genen für den größten Teil der untersuchten Spezies zutrifft. Eine Trennung liegt nur dann vor, wenn ein weiteres Gen zwischen zwei anderen Genen liegt.

### Enterobakterien

Die Enterobakterien sind eine Familie des γ-Zweiges der Proteobacteria. Der Begriff involviert alle taxonomischen Einheiten der Familie, insbesondere die Gattungen Alterococcus, Aquamonas, Aranicola, Arsenophonus, Brenneria, Budvicia, Cedecea, Calymmatobacterium, Citrobacter, Edwardsiella, Enterobacter, Erwinia, Escherichia, Ewingella, Hafnia, Klebsiella, Kluyvera, Koserella, Leclercia, Moellerella, Morganella, Pantoea, Phlomobacter, Photorhabdus, Plesiomonas, Proteus, Providencia, Rahnella, Salmonella, Serratia, Shigella, Wigglesworthia, Xenorhabdus, Yersinia, Yokenella.

### Eubakterien

Die Eubakterien bilden neben den Archaebakterien ein Reich der Prokaryonten. Hierzu wurden "Bakterien" oder "Eubakterien" synonym verwendet. Mit dem Begriff sind alle taxonomischen Einheiten innerhalb dieses Reiches gemeint. Zu den Eubakterien gehören z.B. die Aquificales, Aquificaceae, Desulfurobacterium-Gruppe, Chlamydiales, Verrumicrobia-Gruppe, Chlamydiaceae, Simkaniaceae, Waddliaceae, Verrumicrobia, Verrumicrobiales, Coprothermobacter-Gruppe, Cyanobacteria, Chroococcales, Nostocales, Oscillatoriales, Pleurocapsales, Prochlorophytes, Stigonematales, Cytophagales, Gruppe der grünen Schwefelbakterien, Bacteroidaceae, Cytophagaceae, Flavobacteriaceae, Flexibacter-Gruppe, Hymenobacter-Gruppe, Rhodothermus-Gruppe, Saprospira-Gruppe, Sphingobacteriaceae, Succinovibrionaceae, Grüne Schwefelbakterien, Fibrobacter, Acidobacterium-Gruppe, Fibrobacter-Gruppe, Firmicutes, Actinobacteria, Acidomicrobidae, Actinobacteridae, Coriobacteridae, Rubrobacteridae, Sphaerobacteridae, Bacillus-Gruppe, Clostridium-Gruppe, Lactobacillus-Gruppe, Streptococcus-Gruppe, Clostridiaceae, Haloanaerobiales, Heliobacterium-Gruppe, Mollicutes, Sporomusa-Zweig, Syntrophomonas-Gruppe, Thermoanaerobacter-Gruppe, Flexistipes-Gruppe, Fusobacteria, Grüne Nicht-Schwefelbakterien, Chloroflexaceae-Gruppe, Chloroflexaceae, photosynthetische Flexibakterien, Holophaga-Gruppe, Nitrospira-Gruppe, Planctomycetales, Planctomycetaceae, Proteobacteria, Purpur-Nichtschwefelbakterien, Alpha-Unterabteilung der Proteobakterien, Beta-Unterabteilung der Proteobakterien, Gamma-Unterabteilung der Proteobakterien, Delta/Epsilon-Unterabteilung der Proteobakterien, Spirochetales, Leptospiraceae, Spirochaetaceae, Synergistes-Gruppe, Thermodesulfobacterium-Gruppe, Thermotogales, Thermus-Gruppe oder Deinococcus-Gruppe.

### Gen:

Das Gen umfaßt den offenen Leserahmen oder kodierenden Bereich einer DNA. Es kodiert somit ausschließlich für ein Protein. Auch das Cistron ist ein Gen, das zusammen mit anderen Cistrons jedoch auf einer mRNA liegt. DNA-Regionen, die die Transkription des Gens regulieren, wie der Promotor, Terminator, Enhancer gehören ebenfalls zum Gen. Wenn in diesem Patent vereinfachend vom 23 S rDNA-Gen und 5 S rDNA-Gen die Rede ist, so geschieht dies in Anlehnung an übliche Bezeichnungen. Gemäß unserer Definition sei das 23S-rDNA-Gen oder das 5S-rDNA-Gen jedoch kein Gen, sondern ein eigenständiger funktioneller DNA-Abschnitt, da er nicht für ein Protein kodiert und nicht in Codons unterteilt werden kann.

### Transkribierter Spacer:

Der hierin schwerpunktmäßig behandelte transkribierte Spacer liegt hinter dem kodierenden Bereich des 23 S rDNA-Gens und vor dem kodierenden Bereich des 5 S rDNA-Gens. In seiner systematischen Einordnung nimmt er eine Sonderstellung ein. Da er transkribiert wird, also Bestandteil der mRNA und eines biologisch inaktiven Vorläufermoleküls, der prae-rRNA, ist, gehört er nicht zum intergenischen Bereich. Das Vorläufermolekül wird durch Ausschneiden des transkribierten Spacer in ein im ribosomalen Kontext biologisch aktives Molekül verwandelt. Anderseits läßt er sich funktionell oder phylogenetisch auch nicht eindeutig dem 23 S-Gen oder 5 S-Gen zuordnen. Da der Genbegriff in diesem Fall zur Klassifizierung offensichtlich nicht herangezogen werden kann, sei der "transkribierte Spacer" der ribosomalen Operons gleichberechtigt zu dem "Gen" und der "intergenischen Region" eine eigenständige funktionelle DNA-(RNA-) Klasse.

### Homologe DNA-Sequenzen

DNA oder RNA-Sequenzen sind dann homolog, wenn sie den gleichen phylogenetischen Ursprung haben. Das kann daran zu erkennen sein, daß mindestens 40 % der Nukleotide in einem DNA-Abschnitt identisch sind. In einem größeren DNA-Abschnitt können variable Abschnitte vorliegen. In dem Fall ist es ausreichend, wenn die phylogenetische Verwandschaft angezeigt wird durch das Vorhandensein einer 25 Nukleotide langen Sequenz, die mindestens zu 60 % identisch ist mit einer anderen 25 Nukleotide langen Sequenz der zu vergleichenden DNA. Außerdem können homologe Sequenzen häufig am besten erkannt werden, wenn ein Vergleich mit nahe Verwandten Organismen erfolgt. Zum Erkennen der Homologie von Sequenzen fern verwandter Organismen ist dann ein sequentieller Vergleich mit Sequenzen von Arten erforderlich, die den Abstand zu den fern verwandten phylogenetisch überbrücken.

### Identische DNA-Sequenzen / Prozent Identität

Zur Bestimmung der Identität (im Sinne von vollständiger Übereinstimmung, entsprechend 100 % Identität) von DNA oder RNA-Sequenzen werden Teilsequenzen eines größeren Polynukleotids betrachtet. Diese Teilsequenzen umfassen 10 Nukleotide und sind dann identisch, wenn alle 10 Bausteine bei zwei Vergleichssequenzen identisch sind. Die Nukleotide Thymidin und Uridin seien identisch. Als Teilsequenzen können alle möglichen Fragmente eines größeren Polynukleotids betrachtet werden.

Dabei liegt 90 % Identität vor, wenn in den beiden zu vergleichenden Sequenzen in einem Abschnitt 9 von 10 Nukleotide bzw. 18 von 20 Nukleotide identisch sind.

Als Beispiel seien zwei Polynukleotide betrachtet, die 20 Nukleotide umfassen und sich in dem 5. Baustein unterscheiden. In einem Sequenzvergleich findet man dann sechs 10-er Nukleotide, die identisch sind und 5, die nicht identisch sind, da sie sich in einem Baustein unterscheiden.

Außerdem kann die Identität graduell bestimmt werden, wobei die Einheit in Prozent angegeben wird. Zur Bestimmung des Grades der Identität werden auch Teilsequenzen betrachtet, die minimal die Länge der tatsächlich genutzten Sequenz, z.B. als Primer, oder aber 20 Nukleotide umfassen.

Als Beispiel werden Polynukleotide A mit einer Länge von 100 Nukleotiden und B mit eine Länge von 200 Nukleotiden verglichen. Aus Polynukleotid B wird ein Primer abgeleitet mit einer Länge von 14 Nukleotiden. Zur Bestimmung des Grades der Identität wird Polynukleotid A mit dem Primer in seiner ganzen Länge verglichen. Wenn die Sequenz des Primers in Polynukleotid A vorkommt, wobei sie aber in einem Baustein abweicht, dann gibt es ein Fragment mit einem Identitätsgrad von 13:14 → 92,3 %.

Im zweiten Beispiel werden die zuvor genannten Polynukleotide A und B in ihrer Gesamtheit verglichen. In diesem Fall werden alle möglichen Vergleichsfenster einer Länge von 20 Nukleotiden angelegt und für sie der Identitätsgrad bestimmt. Sind also Nukleotid Nr. 50-69 von Polynukleotid A und B mit Ausnahme von Nukleotid Nr. 55 identisch, dann ergibt sich für diese Fragmente ein Identitätsgrad von 19:20 → 95 %.

### Konservierte und variable Primer

Konservierte Primer sind Nukleotide die an konservierte DNA- oder RNA-Regionen hybridisieren. Der Begriff konserviert charakterisiert die evolutionäre Veränderlichkeit einer Nukleotidsequenz für Spezies verschiedener taxonomischer Einheiten. Er ist deshalb ein vergleichendes Maß. Je nachdem welche Sequenz zum Vergleich herangezogen werden kann ein Bereich bzw. Primer konserviert oder variabel sein. Die Charakterisierung des Primers als "konserviert" oder "variabel" erfolgt anhand unmittelbar angrenzender oder überlappender Regionen bezüglich des Hybridisierungsziels, die die gleiche Länge haben wie der Primer. Es können also Vergleichssequenzen vom gleichen Organismus oder homologe oder ähnliche Sequenzen von anderen Organismen gewählt werden. Beim Vergleich zweier Sequenzen ist eine konserviert, wenn sie mit der Vergleichssequenz zu mindestens 95% identisch ist und variabel, wenn sie zu weniger als 95 % identisch ist.

### Verschachtelte Primer

Verschachtelte Primer werden insbesondere in der Konsensus-PCR verwendet. Es handelt sich dabei um Primer, die ein Fragment eines bereits amplifizierten Polynukleotids amplifizieren. Verschachtelte Primer hybridisieren also mit einem Bereich innerhalb eines bereits vermehrten DNA- oder RNA-Zielmoleküls. Die Amplifikation mit verschachtelten Primern kann dabei beliebig häufig geschehen, so daß sukzessive kleinere Amplifikationsprodukte entstehen.

### Hybridisierung von DNA oder RNA

Zwei identische oder ähnliche Nukleotidframente können miteinander zu einem Doppelstrang hybridisieren. Eine solche Hybridisierung kann nicht nur stattfinden zwischen DNA-, RNA- oder PNA-Einzelsträngen, sondern es können auch Hybridmoleküle zwischen DNA und RNA, DNA und PNA, RNA und PNA usw. gebildet werden. Es gibt eine Reihe von Faktoren, die bestimmen ob zwei Polynukletide hybridisieren. Eine Hybridisierung kann stattfinden in einem Temperaturrahmen von bevorzugt bei 37-60°C. Außerdem kann eine Hybridisierung unter diskreten Hybridisierungs- und Waschschritten ablaufen. Experimentelle Parameter zur Spezifizierung der Hybridisierungsbedingungen sind in Beispiel 4) gegeben. Dabei ist eine spezifische Hybridisierung dann gegeben, wenn mit der eingesetzten Sonde nur eine Hybridisierung mit der gewünschten Zielsequenz erfolgt und nicht mit einer anderen DNA, die ebenfalls in der Probe vorliegt.

### Kombinationen in der Nutzung von Nukleotiden

Primer, Sonden, DNA-Fragmente, Unterbereiche von Polynukleotiden oder Oligonukleotiden können in vielen Kombinationen genutzt werden. Möglich sind z.B. die beliebige Kombination zweier Primer aus einer Gruppe von Primern, die beliebige Auswahl einer Sonde aus einer Gruppe von Sequenzen und die Auswahl von Primern aus der gleichen Gruppe von Sequenzen. In letzterem Fall können die Primer und Sonde(n) identisch oder verschieden sein. Primer oder Sonden können auch aus zwei oder mehreren DNA-Fragmenten zusammengesetzt sein, wobei alle möglichen Variationen der Zusammensetzung in Betracht kommen. Kombinationen sind auch möglich in der Abfolge von distinkten PCR-Schritten mit verschiedenen Primern und dem Einsatz von Sonden.

### Konsensus PCR

Eine Konsensus-PCR wird mit Konsenusprimern durchgeführt. Diese sind in der Lage die DNA von mindestens 2 taxonomischen Einheiten, im Idealfall von allen taxonomischen Einheiten, zu amplifizieren. In nachfolgenden Analyseschritten wird die Identität der amplifizierten DNA bestimmt. Zu diesem Zwecke werden entweder weitere PCR-Schritte durchgeführt, die gegebenenfalls mit variablen, verschachtelten Primern bezüglich kleinerer taxonomischer Einheiten diskriminieren. Die finale Bestimmung einer taxonomischen Einheit kann außer mit variablen Primern auch mit spezifischen Sonden durchgeführt werden.

### Nukleotide

Nukleotide sind die Bausteine der DNA oder RNA. Dabei bedeuten die Abkürzungen: G = Guanosin, A = Adenosin, T = Thymidin, C = Cytidin, R = G oder A, Y = C oder T, K = G oder T, W = A oder T, S = C oder G, M = A oder C, B = C, G oder T, D = A, G oder T, H = A, C oder T, V = A, C oder G, N = A, C, G oder T, I = Inosin.

### Taxonomische Einheiten

Taxonomische Einheiten der Bakterien sind alle bekannten bekannten taxonomischen Unterteilungen, wie z.B. Reiche, Klassen, Abteilungen, Ordnungen, Familien, Gattungen, Arten, Stämme, Zwischeneinheiten dieser taxonomischen Einheiten, wie Unterklassen, Unterordnungen, Unterfamilien etc. oder Gruppen dieser taxonomischen Einheiten.

### Ausführliche Beschreibung der Erfindung

Beschrieben sind im wesentlichen 5 Teilaspekt
- Strategische Auswahl von DNA-Zielregionen unter Nutzung benachbarter Gene
- Beschreibung der Nutzung einer ribosomalen DNA-Region aus dem Ende der 23 S rDNA, dem transkribierten Spacer und Teilen der 5 S rDNA zum Nachweis aller Bakterien
- Bereitstellung von Primern und Sonden für eine Vielzahl von Bakterien
- Nachweis der Familie der Enterobakterien und deren Mitglieder
- Anwendung einer Konsensus-PCR zum Nachweis aller Bakterien

### Strategische Auswahl von DNA-Zielregionen unter Nutzung benachbarter Gene

Die Erfindung besteht in der Nutzung von Anteilen benachbarter Gene zum Nachweis der taxonomischen Einheit der Enterobacteriaceae . Der Vorteil der Erfindung liegt darin, daß DNA-Bereiche, die zwei Gene überspannen bezüglich der Variabilität sehr heterogen zusammengesetzt sind, wie am Beispiel der ribosomalen Operons, insbesondere dem 23S/5S rDNA Abschnitt, gefunden wurde. Durch das Vorhandensein von sehr stark und sehr wenig konservierten Bereichen ist der Fachmann in die Lage versetzt, alle möglichen nahe und auch fern verwandten Organismen nachzuweisen.

### Beschreibung der Nutzung einer ribosomalen DNA-Region aus dem Ende der 23 S rDNA. dem transkribierten Spacer und Teilen der 5 S rDNA zum Nachweis aller Bakterien

Insbesondere ein 23 S-5 S rDNA Bereich umfassend ca. 400-750 Nukleotide kann zum Nachweis von Bakterien genutzt werden. Letztere Region besteht aus ca. 330-430 Nukleotiden des terminalen Bereichs der 23 S rDNA, dem anschließenden transkribierten Spacer und dem 5 S rDNA-Gen. In einzelnen Fällen kann zudem ein t-RNA-Gen in den Spacer insertiert sein und wird für den Nachweis mitgenutzt. Die beschriebene Region enspricht somit den Nukleotiden 2571-3112 der SEQ ID 1, welche die 23 S und 5 S rDNA-Gene von Escherichia coli darstellt. Durch einen dem Fachmann vertrauten Sequenzvergleich lassen sich die homologen und dem obigen Bereich entsprechenden Abschnitte anderer Bakterien bestimmen. Insbesondere bei Angehörigen gleicher Familien oder auch Odnungen oder Abteilungen läßt sich der Beginn des oben skizzierten Bereichs am Terminus der 23 S rDNA-Gens und das Ende des 5 S rDNA-Gens durch einen Vergleich der ribosomalen DNA-Sequenzen zweier Arten A und B leicht bestimmen. Sollte dies für einen Vergleich der Arten A und einer weiter entfernten Art C nicht so leicht möglich sein, so kommt man zu dem gewünschten Ergebnis, indem man einen Vergleich zwischen den Sequenzen der Arten B und C durchführt, wobei B und C miteinander näher verwandt sein sollten. Durch eine Reihe von separaten Sequenzvergleichen können auf diese Weise die der obigen Region entsprechenden homogenen ribosomalen Bereiche der 23 S rDNA, des transkribierten Spacers und der 5 S rDNA aller Eubakterien bestimmt werden. Aufgrund von Variabilität einzelner Subbereiche können dabei durchaus Längenunterschiede von mehreren hundert Nukleotiden auftreten. Des weiteren erlaubt die vorliegende Erfindung die Nutzung von Unterbereichen der oben beschriebenen Region. Ein Großteil dieser Bereiche ist in Tabelle 6 beschrieben.

### Bereitstellung von Primern und Sonden für eine Vielzahl von Bakterien

Neben der generellen Beschreibung des nutzbaren rDNA-Bereichs werden auch Sequenzen (SEQ ID 1-530) bereitgestellt, die zum Zwecke des Nachweises von Bakterien verwendet werden können. Je nach Aufgabenstellung können dabei die in SEQ ID 1-530 spezifizierten Polynukleotide komplett verwendet werden, oder Fragmente aus diesen. Die unter SEQ ID 1-530 spezifizierten Sequenzen stammen dabei aus dem zuvor beschriebenen Bereich des 23 S-rDNA-Gens, transkribierten Spacers und 5 S rDNA-Gens.

In der technischen Ausführung kann der Nachweis von Organismen mit Hilfe der hierzu spezifizierten DNA-Bereiche und Sequenzen durch Sonden und/oder Primer erfolgen. Primer sind Nukleotide, die als Startermoleküle für die Amplifikation dienen. Sie lagern sich dabei an die Zielsequenz an, woraufhin die Region mit Hilfe einer Polymerase neu synthetisiert wird. Durch den Grad der Identität der Primer mit der Zielsequenz läßt sich deren Spezifität einstellen. Die taxonomische Spezifität wird zudem durch die Auswahl der Zielsequenz innhalb des hierin beschriebenen ribosomalen Bereichs determiniert (s. auch Tabelle 6). Dementsprechend können Primer also auf verschiede Weisen genutzt werden: so ist es z.B. möglich den gesamten Bereich entsprechend Abb. 2 oder homolog zu den Nukleotiden Nr. 2571-3112 der SEQ ID 1 (E. coli) mit den Primern SEQ ID 211 und 212 zu amplifizieren. Um die Amplifikation zu optimieren kann auch ein Gemisch von mehr als zwei Primern eingesetzt werden. Außerdem ist es möglich die Primer so zu wählen, daß nur die DNA bestimmter Bakterien amplifiziert wird. In diesem Fall geben sie also zweierlei Informationen: Erstens zeigen sie die Anweisenheit und zweitens die Identität der gesuchten Bakterien im Falle positiver Amplifikation. Durch sequentielle Amplifikationsschritte mit verschachtelten Primern kann der Informationsausstoß am Ende der DNA-Synthese nach den Erfordernissen gelenkt werden.

In einem distinkten Schritt kann die DNA, die idealer Weise zuvor amplifiziert worden ist, mit Sonden gebunden, ankonzentriert und nachgewiesen werden. Sonden sind also Oligonukleotide oder Polynukleotide, die an einzelsträngige DNA-Abschnitte binden können. Die Affinität der Sonden zur Zielsequenz wird duch den Grad der Identität mit dieser bestimmt. Außerdem haben die Hybridisierungsbedingungen einen signifikanten Einfluß, d.h. Salzkonzentration der Puffer, Inkubationszeit und -temperatur müssen optimiert werden. Der Fachmann ist in der Lage diese Parameter mit Hilfe gängiger Methoden schnell zu optimieren. Exemplarische Hybridsierungsbdingungen sind in den Beispielen gegeben. Sonden können ganz analog wie Primer zweierlei leisten: erstens können sie die Anwesenheit von bakterieller DNA oder von Amplifikationsprodukten zeigen; zweitens können sie zur Detektion der DNA bestimmter Bakterien beitragen. In dieser Dualität ihrer Funktion gleichen sie also den Primern. Demzufolge kann es also zwischen Primern und Sonden zu einer Aufgabenteilung bei der Identifizierung von Organismen kommen. Außerdem können die Sonden ebenso wie die Primer aus frei wählbaren Bereichen des terminalen Bereichs der 23 S rDNA, des transkribierten Spacers oder der 5 S rDNA stammen oder auch den gesamten Bereich umfassen.

Ein besonderer Vorteil der vorliegenden Erfindung liegt darin, daß der ausgewählte ribosomale Bereich gemäß Abb. 2 heterogen aus sehr variablen und sehr konservierten Regionen in einem extrem breiten Spektrum zusammengesetzt ist. Da es sehr viele Kombinationen in der Nutzung von Subregionen, z.B. gemäß Tabelle 6 gibt, bietet die vorliegende Erfindung eine Nachweismöglichkeit für die taxonomischen Einheit der Enterobacteriaceae.

### Nachweis der Familie der Enterobakterien und deren Mitglieder

Mit Hilfe der hierin charakterisierten DNA-Zielregion können z.B. bakterielle Familien wie die Enterobacteriaceae nachgewiesen werden (Bsp. 1). Die Enterobakterien sind eine homogene taxonomische Einheit des γ-Zweiges der Proteobakterien oder Purpurbakterien. Sie sind deshalb von besonderem Interesse, weil ihnen viele pathogene Bakterien angehören, wie Escherichia coli (EHEC etc.), Shigella, Salmonella, Yersinia. Sie eignen sich also als Markerorganismen, um den hygienischen Zustand von Lebensmitteln zu überprüfen. In der klinischen Mikrobiologie kann der Nachweis von Enterobakterien, einen ersten Schritt bei der Eingrenzung oder Identifizierung pathogener Keime darstellen. Aus der hierin enthaltenen Auflistung sind z.B. die Primer SEQ ID 2-25 in verschiedenen Kombinationen geeignet die Enterobakterien als Familie zu identifizieren. Viele der aufgelisteten Sequenzen sind außerdem geeignet einzelne Mitglieder der Enterobakterien, d.h. Gattungen, Spezies und Stämmen zu indentifizieren. Weitere Sequenzen werden auch für die übrigen taxonomischen Einheiten der Proteobakterien, insbesondere den gesamten γ-Zweig und außerdem für die Firmicutes bereitgestellt. Mit der Beschreibung der ribosomalen Region gemäß Abb. 2 wird ein weiterer Weg aufgezeigt, wie der Fachmann leicht weitere Sequenzen gewinnen kann, um alle Eubakterien nachzuweisen.

### Anwendung einer Konsensus-PCR zum Nachweis aller Bakterien

Ein besonderer Vorteil liegt darin, daß die DNA-Zielregion, wie sie in Abb. 2 beschrieben ist, sich in idealer Weise in einer Konsensus-PCR nachweisen läßt. Eine wesentliche Voraussetzung für die experimentelle Anwendbarkeit dieser Methode ist, daß die Sequenzen innerhalb einer zu amplifizierenden Zielregion zunehmend variabel werden. Diese Konfiguration ist in dem von uns charakterisierten Bereich des ribosomalen Operons für alle untersuchten Spezies erfüllt.

Das Schema der Konsensus-PCR ist in Abb. 8 umrissen. In der Regel wird zunächst ein "Masterfragment" amplifiziert. Dieses kann dem Gesamtfragment entsprechend Abb. 2 gleichen oder ein Teil davon sein. Wenn nun in einer Probe verschiedene zu identifizierende Keime vorliegen, so wird für alle dieses Fragment amplifiziert. Die einzelnen Keime werden schließlich mit spezifischen Sonden und/oder in Kombination mit weiteren PCR-Schritten identifiziert. Der Nachweis mit Sonden kann auch miniaturisiert sein und auf Chips erfolgen. Alternativ kann ein Nachweis im klassischen ELISA-Verfahren erfolgen. Die Komponenten des Bakteriennachweises können in Form eines Kits bereitgestellt werden.

Vorteilhaft zur Detektion sind insbesondere fluoreszierende Farbstoffe. Diese können an die Primer oder die Sonden gekoppelt werden. Insbesondere im ELISA oder in Southern Blot-Verfahren werden jedoch häufig auch nicht-fluoreszierende Farbstoffe verwendet. Eine weitere Möglichkeit des Nachweises besteht mit der Genetrak- und LightcyclerTechnologie. Im Prinzip bieten alle diese Verfahren die Option eines quantitativen Nachweises. Es ist also möglich durch Auswertung des Detektionssignals letztendlich auf die Zahl der in einer Probe vorhandenen Bakterien rückzuschließen.

Der Nachweis von Bakterien kann in einem experimentellen Kontext erfolgen, der dem Fachmann durchaus bekannt ist. So ist es möglich Bakterien vor dem Nachweis zunächst in einem geeigneten Medium anzureichern. Beim Arbeiten mit Lebensmitteln können physikalische Abtrennungsschritte, wie Zentrifugation der Sedimentation, eine vorteilhafte Ausführung darstellen. Es ist auch möglich die Bakterien so anzureichern, daß nachträglich Rückschlüsse auf die Ausgangskeimzahl möglich sind. Des weiteren können Grenzwertbestimmungen bezüglich der Keimzahl durchgeführt werden. Alles in allem ist also ein quantitativer oder semiquantitativer Keimnachweis möglich.

Zur Isolierung genomischer DNA werden die (angereicherten) Bakterien aufgeschlossen. Physikalische (Glasperlen, Hitze) und chemische (NaOH) Einflüsse liegen häufig den dem Fachmann bekannten Protokollen zum Zellaufschluß zugrunde. Es ist jedoch auch möglich Zellen direkt in eine PCR zum DNA-Nachweis einzusetzen. Außerdem kann es vorteilhaft sein, die genomische DNA, insbesondere wenn sie in Lebenmittelmatrizes verteilt ist, aufzureinigen. Auch diese Verfahren sind dem Fachmann bekannt. DNA-Reinigungskits sind zudem kommerziell erhältlich.

**Tabelle 1. Nachweis von Enterobakterien unter Ausgrenzung von anderen Bakterien (Beisp. 1)**

| **Nr.** | **Arten** | **Stamm** | **Nachweis** |
|---|---|---|---|
| 1 | Budvicia aquatilis | DSM 5025 | + |
| 2 | Buttiauxella agrestis | DSM 4586 | + |
| 3 | Cedecea davisae | DSM 4568 | + |
| 4 | Citrobacter koser | DSM 4595 | + |
| 5 | Erwinia carotovora | DSM 30168 | + |
| 6 | Erwinia chrysanthemi | DSM 4610 | + |
| 7 | Ewingella americana | DSM 4580 | + |
| 8 | Enterobacter agglomerans | B-5081-i | + |
| 9 | Enterobacter aerogenes | DSM 30053 | + |
| 10 | Enterobacter sakazakii | DSM 4485 | + |
| 11 | Enterobacter intermedius | DSM 4581 | + |
| 12 | Enterobacter cloacae | DSM 30054 | + |
| 13 | E. coli | BC 7883 | + |
| 14 | E. coli | H123 | + |
| 15 | E. coli | BC 7884 | + |
| 16 | E. coli | BC 7885 | + |
| 17 | E. hermanii | B-4943a | + |
| 18 | E. coli | ATCC 8739 | + |
| 19 | Hafnia alvei | DSM 30163 | + |
| 20 | Klebsiella pneumoniae | ATCC 13883 | + |
| 21 | Klebsiella pneumoniae | DSM 2026 | + |
| 22 | Klebsiella planticola | DSM 4617 | + |
| 23 | Klebsiella oxytoca | DSM 5175 | + |
| 24 | Kluyvera cryocrescens | DSM 4583 | + |
| 25 | Morganella morganii | DSM 30164 | + |
| 26 | Plesiomonas shigelloides | DSM 8224 | + |
| 27 | Pantoea ssp. | B-5200 | + |
| 28 | Pantoea dispersa | DSM 30073 | + |
| 29 | Proteus rettgeri | DSM 1131 | + |
| 30 | Proteus rettgeri | ATCC 14505 | + |
| 31 | Providencia stuartii | DSM4539 | + |
| 32 | Rahnella aquatilis | DSM 4594 | + |
| 33 | Rahnella aquatilis | DSM 4594 | + |
| 34 | Serratia proteamaculans | DSM 4487 | + |
| 35 | Serratia ficaria | DSM 4509 | + |
| 36 | Serratia plymutica | DSM49 | + |
| 37 | Serratia rubidea | DSM 4480 | + |
| 38 | Serratia marcescens | DSM 1636 | + |
| 39 | Salmonella bongori | DSM 7952 | + |
| 40 | Yersinia pseudotuberculosis | DSM 8992 | + |
| 41 | Yersinia pseudotuberculosis | DSM 8992 | + |
| 42 | Yersinia enterolytica | DSM 4790 | + |
| 43 | Acinetobacter calcoaceticus | DSM 590 | - |
| 44 | Aeromonas hydrophila | DSM 6173 | - |
| 45 | Aeromonas enteropelogenes | DSM 6394 | - |
| 46 | Fransilla tularensis Isolat | F16 | - |
| 47 | Franzisella philomiragia | DSM 7535 | - |
| 48 | Moraxella catarrhalis | DSM 9143 | - |
| 49 | Pasteurella pneumotropica | B-2397 A 13 | - |
| 50 | Pseudomonas beyjerinkii | DSM 7218 | - |
| 51 | Vibrio fischeri | DSM 507 | - |
| 52 | Vibrio alginolyticus | DSM2171 | - |
| 53 | Vibrio proteolyticus | DSM 30189 | - |
| 54 | Vibrio paramaemolytiucs | DSM 10027 | - |
| 55 | Vibrio harveyi | DSM 6104 | - |
| 56 | Xanthomonas maltophila | BC 4273 | - |
| 57 | Achromobacter xylosa | DSM 2402 | - |
| 58 | Alcaligenes spp | DSM 2625 | - |
| 59 | Alcaligenes latus | DSM 1122 | - |
| 60 | Brucella neotomae | ATCC 25840 | - |
| 61 | Brucella ovis | ATCC 23459 | - |
| 62 | Enterococcus casseliflavus | DSM 20680 | - |
| 63 | Flavobacterium sp. | ATCC 27551 | - |
| 64 | Flavobacterium resinovorum | DSM 7438 | - |
| 65 | Flavobacterium johnsonü | DSM 2064 | - |
| 66 | Flavobacterium flavense | DSM 1076 | - |
| 67 | Lactobacillus bifennentans | BC 8463 | - |
| 68 | Pseudomonas paucimobilis | DSM 1098 | - |
| 69 | Pseudomonas cepacia | DSM 3134 | - |
| 70 | Sphingobacterium multivorans | DSM 6175 | - |

**Tabelle 2. Nachweis von Pantoea dispersa unter Ausgrenzung von anderen Bakterien (Beisp. 2)**

| **Nr.** | **Art** | **Nachweis** |
|---|---|---|
| 1 | Pantoea dispersa | + |
| 2 | Budvicia aquatica | - |
| 3 | Buttiauxella agrestis | - |
| 4 | Enterobacter agglomerans | - |
| 5 | Erwinia carotovora | - |
| 6 | Erwinia crysanthemi | - |
| 7 | Escherichia coli | - |
| 8 | Escherichia vulneris | - |
| 9 | Escherichia hermannii | - |
| 10 | Hafnia alvei | - |
| 11 | Klebsiella oxytoca | - |
| 12 | Kluyvera cryoescens | - |
| 13 | Morganella morganii | - |
| 14 | Proteus mirabilis | - |
| 15 | Proteus rettgeri | - |
| 16 | Proteus stuartii | - |
| 17 | Providencia stuartii | - |
| 18 | Rahnella aquatilis | - |
| 19 | Serratia ficaria | - |
| 20 | Serratia fonticola | - |
| 21 | Serratia marcescens | - |
| 22 | Scrratia plymuthica | - |
| 23 | Serratia proteamaculans | - |
| 24 | Serratia rubidea | - |
| 25 | Yersinia enterolytica | - |
| 26 | Yersinia peudotuberculosis | - |
| 27 | Acinetobacter calcoaceticus | - |
| 28 | Aeromonas enteropelogenes | - |
| 29 | Aeromonas hydrophila | - |
| 30 | Cedecea davisae | - |
| 31 | Haemophilus influenzae | - |
| 32 | Moraxella catarrhalis | - |
| 33 | Pasteurella pneumotropica | - |
| 34 | Stenotrophomonas multophila | - |
| 35 | Vibrio alginolyticus | - |
| 36 | Vibrio fisheri | - |
| 37 | Vibrio harveyi | - |
| 38 | Vibrio parahaemolyticus | - |
| 39 | Alcaligenes sp. | - |
| 40 | Bacillus subtilis | - |
| 41 | Brucella abortus | - |
| 42 | Brucella ovis | - |
| 43 | Flavobacterium resinovorum | - |
| 44 | Pseudomonas paucimobilis | - |
| 45 | Pseudomonas cepacia | - |
| 46 | Ralstonia pickettü | - |
| 47 | Sphingobacterium multivorum | - |
| 48 | Sphingomonas paucimobilis | - |
| 49 | Streptococcus faecalis | - |

**Tabelle 3: Nachweis einer Gruppe von Gattungen mit der Sonde GTTCCGAGATTGGTT**

| **Nr.** | **Art** | **Nachweis** |
|---|---|---|
| 1 | Rahnella aquatilis | + |
| 2 | Serratia ficaria | + |
| 3 | Serratia fonticola | + |
| 4 | Serratia marcescens | + |
| 5 | Serratia plymuthica | + |
| 6 | Serratia proteamaculans | + |
| 7 | Serratia rubidea | + |
| 8 | Yersinia enterolytica | + |
| 9 | Yersinia peudotuberculosis | + |
| 10 | Budvicia aquatica | - |
| 11 | Buttiauxella agrestis | - |
| 12 | Enterobacter agglomerans | - |
| 13 | Erwinia carotovora | - |
| 14 | Erwinia crysanthemi | - |
| 15 | Escherichia coli | - |
| 16 | Escherichia vulneris | - |
| 17 | Escherichia hermannii | - |
| 18 | Hafnia alvei | - |
| 19 | Klebsiella oxytoca | - |
| 20 | Kluyvera cryoescens | - |
| 21 | Morganella morganii | - |
| 22 | Pantoea dispersa | - |
| 23 | Proteus mirabilis | - |
| 24 | Proteus rettgeri | - |
| 25 | Proteus stuartii | - |
| 26 | Providencia stuartü | - |
| 27 | Acinetobacter calcoaceticus | - |
| 28 | Aeromonas enteropelogenes | - |
| 29 | Aeromonas hydrophila | - |
| 30 | Cedecea davisae | - |
| 31 | Haemophilus influenzae | - |
| 32 | Moraxella catarrhalis | - |
| 33 | Pasteurella pneumotropica | - |
| 34 | Stenotrophomonas multophila | - |
| 35 | Vibrio alginolyticus | - |
| 36 | Vibrio fisheri | - |
| 37 | Vibno harveyi | - |
| 38 | Vibrio parahaemolyticus | - |
| 39 | Alcaligenes sp. | - |
| 40 | Bacillus subtilis | - |
| 41 | Brucella abortus | - |
| 42 | Brucella ovis | - |
| 43 | Flavobacterium resinovorum | - |
| 44 | Pseudomonas paucimobilis | - |
| 45 | Pseudomonas cepacia | - |
| 46 | Ralstonia pickettii | - |
| 47 | Sphingobacterium mulüvorum | - |
| 48 | Sphingomonas paucimobilis | - |
| 49 | Streptococcus faecalis | - |

**Tabelle 4: Spezifische Sonden zum Nachweis von bakterieller Gattungen und Arten**

| **Nr.** | **Sonde SEQ ID** | **Nachweis Gattung/Art** |
|---|---|---|
| 1 | 96 | Budvicia aquatica |
| 2 | 97 | Buttiauxella agrestis |
| 3 | 98 | Enterobacter agglomerans |
| 4 | 99 | Erwinia carotovora |
| 5 | 100 | Erwnia chrysanthemi |
| 6 | 101 | Escherichia coli |
| 7 | 102 | Escherichia hermannii |
| 8 | 103 | Escherichia vulneris |
| 9 | 104 | Hafnia alvei |
| 10 | 105 | Klebsiella oxytoca |
| 11 | 106 | Kluyvera cryoescens |
| 12 | 107 | Morganella morganii |
| 13 | 108, 109 | Pantoea |
| 14 | 110 | Proteus mirabilis |
| 15 | 111 | Proteus rettgeri |
| 16 | 112 | Providencia stuartii |
| 17 | 113 | Rahnella aquatilis |
| 18 | 114 | Serratia ficaria |
| 19 | 115 | Serratia fonticola |
| 20 | 116 | Serratia marcescens |
| 21 | 117 | Serratia plymuthica |
| 22 | 118 | Serratia proteamaculans |
| 23 | 119 | Serratia rubidea |
| 24 | 120 | Yersinia enterolytica |
| 25 | 121 | Yersinia pseudotuberculosis |
| 26 | 122 | Acinetobacter calcoaceticus |
| 27 | 123 | Acromonas enteropelogenes |
| 28 | 124 | Aeromonas hydrophila |
| 29 | 125 | Ccdecea davisae |
| 30 | 126 | Haemophilus influenzae |
| 31 | 127 | Moraxella catharralis |
| 32 | 128 | Pasteurella pneumotropica |
| 33 | 129 | Stenotrophomonas multophila |
| 34 | 130 | Vibrio alginolyticus |
| 35 | 131 | Vibrio fisheri |
| 36 | 132 | Vibrio harveyi |
| 37 | 133 | Vibrio parahaemolyticus |
| 38 | 134 | Vibrio proteolyticus |
| 39 | 432 | Salmonella typhi |
| 40 | 433 | Buchnera aphidocola |
| 41 | 434 | Pseudomonas stutzeri |
| 42 | 435 | Thiobacillus ferrooxidans |
| 43 | 436 | Agrobactenum vitis |
| 44 | 437 | Adalia bipunctata |
| 45 | 438 | Amycocalatopsis orientalis |
| 46 | 439 | Brucella |
| 47 | 440 | Bradyrhyzobium japonicum |
| 48 | 441 | Pseudomonas paucimobilis |
| 49 | 442 | Rhodobacter sphaeroides |
| 50 | 443 | Rickettsia prowazekü |
| 51 | 444 | Pseudomonas cepacia |
| 52 | 445 | Ralstonia pickettii |
| 53 | 446 | Campylobacter jejuni |
| 54 | 447 | Helicobacter pylori |
| 55 | 448 | Actinoplanes utahensis |
| 56 | 449 | Bacillus halodurans |
| 57 | 450 | Bacillus subtilis |
| 58 | 451 | Clostridium tyrobutyricum |
| 59 | 452 | Frankia |
| 60 | 453 | Microbispora bispora |
| 61 | 454 | Mycobactenum leprae |
| 62 | 455 | Mycobacterium smegmatis |
| 63 | 456 | Mycobacterium tuberculosis |
| 64 | 457 | Mycoplasma gallisepticum |
| 65 | 458 | Propionibacterium freudenreichii |
| 66 | 459 | Rhodococcus erythropolis |
| 67 | 460 | Rhodococcus fascians |
| 68 | 461 | Staphylococcus aureus |
| 69 | 462 | Streptococcus faecalis |
| 70 | 463 | Streptomyces ambifaciens |
| 71 | 464 | Streptomyces galbus |
| 72 | 465 | Streptomyces griseus |
| 73 | 466 | Streptomyces lividans |
| 74 | 467 | Streptomyces mashuensis |
| 75 | 468 | Flavobacterium resmovorum |
| 76 | 469 | Sphmgobacterium multivorans |
| 77 | 470 | Synechococcus |
| 78 | 471 | Synechocystis |
| 79 | 472 | Borrelia burgdorferi |
| 80 | 473 | Chlamydia trachomatis |
| 81 | 474 | Azotobacter vinelandii |
| 82 | 475 | Cowdria ruminantium |
| 83 | 476 | Mycobacterium intracellulare |
| 84 | 477 | Mycobacterium lufu |
| 85 | 478 | Mycobacterium simiae |
| 86 | 479 | Mycobacterium smegmatis |
| 87 | 480 | Saccharomonospora azurea |
| 88 | 481 | Saccharomonospora caesia |
| 89 | 482 | Saccharomonospora cyanea |
| 90 | 483 | Saccharomonospora glauca |
| 91 | 484 | Saccharomonospora viridis |
| 92 | 485 | Wolbachia pipientis |
| 93 | 525 | Sphmgomonas paucimobilis |
| 94 | 526 | Zymomonas mobilis |
| 95 | 527 | Alcaligenes |
| 96 | 528 | Borrelia burgdorferi |
| 97 | 529 | Xanthomonas campestris |
| 98 | 530 | Cowduria ruminantium |

**Tabelle 5: Primer zum Nachweis von Bakterienarten oder Gattungen**

| **Nr.** | **verwendete Spezies** | **SEQ ID** | **vorwärts-Primer** | **rückwärts-Primer (rückwärts- Primer* = komplementär)** |
|---|---|---|---|---|
| 1 | Budvicia aquatica | 96 | CGAGGTGTTTTAAGGAAAGTT | CGGTCAATAGACAGAATAT |
| 2 | Buttiauxellis agrestis | 97 | CGAAGGTGTTTTGGTTGAGAG | GGTTGATGAAACAGAATAT |
| 4 | Enterobacter agglomerans | 98 | CGAAGATGTTTTGGCGGATTG | GTITCTGGCAACAGAATTT |
| 5 | Erwinia carotovora | 99 | CGAAGGTGTTTTGAGAGTGAC | TTGGGATGAAACAGAATTT |
| 6 | Erwinia chrysanthemi | 100 | CGAAGGTGTTTTAGAGAGATT | TCGGGATGAAACAAAATTT |
| 7 | Escherichia coli | 101 | CGAAGCTGTTTTGGCGGATGA | GTCTGATAAAACAGAATTT |
| 8 | Escherichia hermannii | 102 | CAGAGTGGTTTTGGTGTTGCG | CAGCAGGTGAACAGAATTT |
| 9 | Escherichia vlneris | 103 | CGAAGATGTn7GGCGGATTT | CGTCAGACAGACAGAATTT |
| 10 | Hafnia alvei | 104 | CGAAGGTGTTTTAAGACGCAG | GGTACAAATAACAGAATAT |
| 11 | Klebsiella oxytoca | 105 | CGAAGATGTTTTGGCGATTTG | GTTTCTGACAACAGAATTT |
| 12 | Kluyvera cryoescens | 106 | CAAAGATGTTTTGGTGAAAAG | CGGGTTAATAACAGAATTT |
| 13 | Morganella morganii | 107 | CGAAGGTGTTTTGAGTTGAGA | TTTGGATTGAAATGAATTT |
| 14 | Pantoea dispersa | 108 | CAGAGGCGTTTTGGTCTGAGA | GCGGTNTAAAACAAAATTT |
| 15 | Pantoea ssp. | 109 | CGAAGATGTTTTGGCGGAATG | GTTTCTGGCAACAGAATTT |
| 16 | Proteus mirabilis | 110 | CGAAAGTGTTTTGTCAGAGAG | AGTGATTAAAACCGAATTT |
| 17 | Proteus rettgeri | 111 | CGAAGGTGTTTfAGAGAGATA | CGGGAACAAAACAGAATIT |
| 18 | Providencia stuartii | 112 | CGAAGGTGTTTTAGAGAGACG | ACGGGAACGAACCGAATTT |
| 19 | Rahnella aquatilis | 113 | CGAAGGTGTTTTTGATTTGAG | TATGAATGAAACAGAATTT |
| 20 | Salmonella typhi | 432 | CGAAGGTGTTTTGGAGGATAA | GATAAAAGAAACAGAATTT |
| 21 | Serratia ficaria | 114 | CGAAGGTGTTTTAGAGAGACG | CAAGAATGAAACAGAATTT |
| 22 | Serratia fonticola | 115 | CCAAGGTGTTTTGAAGAGATT | TTGAAATGAAACAGAATTT |
| 23 | Setratia marcescens | 116 | CGAAGGTGTTTTTAGAGAGAT | TTGGAATGAAACAGAATTT |
| 24 | Serratia plymuthica | 117 | CGAAGGTGTTTTAGAGAGATT | TTGGAATGAAACAGAATTT |
| 25 | Serratia proteamaculans | 118 | CAAAGGTGTTITAGAGAGATI | TTGGAATGAAACANAATTT |
| 26 | Serratia rubidea | 119 | CGAAGGTGTTTTAGAGAGATT | TCGGGATGAAACAGAATTT |
| 27 | Yersinia enterolytica | 120 | CAAAGGTGTTTTGTATTTGAG | G7rAGTTTAGACAGAATIT |
| 28 | Acinetobacter calcoaceticus | 122 | CCAAGCAGTTGTATATAAAGC | GCAACCAATAAGACCAATG |
| 29 | Aeromonas enteropelogenes | 123 | CCAAGAAGTGTTTNTGGTGCT | TTCCAAGATTGAAGATTTT |
| 30 | Aeromonas hydrophila | 124 | CCAAGAAGTGTTCTAAGGCTT | TTCTCAGATTGAAGAATTT |
| 31 | Buchnera aphidocola | 433 | CCAGAGGTGTTTTTTATAAAA | ATCTTGTTTTACTGAATTT |
| 32 | Haemophilus influenzae | 126 | GCTCAAGTGTTTTTGGGAGCT | CGGTCAGTAAACAGAATTT |
| 33 | Moraxella catarrhalis | 127 | ACCCAAGTGGTTTACCACTGA | GTAATAAACAGACTCATAC |
| 34 | Pasteurella pneumotropica | 128 | ACCAAATTTGTTTATCGTAAC | AGTTGTTATAATAAAACAT |
| 35 | Vibrio alginolyticus | 130 | CCMGGGGITITGATGGACTC | TTTCCAGATTAAAGAATTT |
| 36 | Vibrio fisheri | 131 | CCAAGTGGTTTGTATCAAGCA | TTAAGTAAAACAAACACAG |
| 37 | Vibrio harveyi | 132 | CCAAGGGGTTTTGATGGACTC | TTTCCAAATTAAAGAATTT |
| 38 | Vibrio parahaemolyticus | 133 | CCAAGGGGTTTTGATGGACTC | TTTCCGAATTAAAGAATTT |
| 39 | Vibrio proteolyticus | 134 | CCAAGGGGTTTTGATGGACTC | TTGTTCCAGACAAAATTTT |

**Tabelle 6: Nachweispotentiale und Spezifikation der Lokalisation von DNA-Fragmenten aus dem rDNA-Operon**

| **Nr. in** **Abb. 2** | **DNA-Bereich** | **Position in SEQ ID 1** | **Nachweispotentiale** |
|---|---|---|---|
| 1. | terminaler Bereich des 23 S rDNA-Gens | 2667-2720 | Abteilungen, Klassen, Ordnungen, Familien |
| 2. | terminaler Bereich des 23 S rDNA-Gens | 2727-2776 | Abteilungen, Klassen, Ordnungen, Familien |
| 3. | terminaler Bereich des 23 S rDNA-Gens | 2777-2800 | Abteilungen, Klassen, Ordnungen, Familien |
| 4: | terminaler Bereich des 23 S rDNA-Gens | 2801-2838 | Klassen, Ordnungen, Familien |
| 5. | Ende des 23 S rDNA-Gens | 2857-2896 | Abteilungen, Klassen, Ordnungen, Familien |
| 6. | Beginn des 23 S-5 S transkribierten Spacers | 2897-2938 | Ordnungen, Familien, Gattungen, Arten, Stämme |
| 7. | 23 S-5 S transkribierter Spacer | 2939-2983 | Gattungen, Arten, Stämme |
| 8. | Ende des 23 S-5 S transkribierten Spacers | 2984-2999 | Familien, Gattungen, Arten, Stämme |
| 9. | Beginn des 5 S rDNA-Gens | 3000-3032 | Abteilumgen, Klassen, Ordnungen, Familien |

**Tabelle 7: Primer aus Beispiel 1**

| **vorwärts-Primer** | **rückwärts-Primer** | **Annealing-temperatur (°C)** | **abbildung** |
|---|---|---|---|
| SEQ ID 2 | SEQ ID 7-22 | 62 | 3 |
| SEQ ID 2 | SEQ ID 23-24 | 62 | 4 |
| SEQ ID 2 | SEQ ID 25 | 67 | 5 |
| SEQ ID 3-6 | SEQ ID 23-24 | 62 | 6 |
| SEQ ID 3-6 | SEQ ID 25 | 67 | 7 |

**Tabelle 8: Konsensus-PCR zum Nachweis von Bakterien**

| **Nr.** | **taxonomische Einheit** | **Primer A1 SEQ iD** | **Primer B1 SEQ ID** | **Primer C1 SEQ ID** | **Primer D1 SEQ ID** | **Primer E1 SEQ ID** | **Primer F1 SEQ ID** | **Primer G1 SEQ ID** | **Primer H1 SEQ ID** | **Primer B2 SEQ ID** | **Primer A2 SEQ ID** |
|---|---|---|---|---|---|---|---|---|---|---|---|
| 1 | Enterobakterien | 1 | 7-22 | | | | | | | 4 | 5 |
| 2 | Enterobakterien | 26 | 34 | 42 | 54 | 66 | 78 | 85 | | | 135 |
| 3 | Acinetobacter | 27 | 35 | 43 | 55 | 67 | 79 | | | | |
| 4 | Aeromonas | 28 | 36 | 44 | 56 | 68 | 80 | 87 | | | 155 |
| 5 | Haemophilus | 29 | 37 | 45 | 57 | 69 | 81 | | | | |
| 6 | Moraxella | 30 | 38 | 46 | 58 | 70 | 82 | | | | |
| 7 | Pasteurella | 31 | 39 | 47 | 59 | | | | | | |
| 8 | Stenotrophomonas | 32 | 40 | 48 | 60 | 72 | | 90 | | | |
| 9 | Vibrio | 33 | 41 | | | | | | | | |
| 10 | Vibrio alginolyticus | | | 49 | 61 | 73 | | 91 | 130 | | 160 |
| 11 | Vibrio fisheri | | | 50 | 62 | 74 | | 92 | 131 | | 161 |
| 12 | Vibrio harveyi | | | 51 | 63 | 75 | | 93 | 132 | | 162 |
| 13 | Vibrio parahaemolyticus | | | 52 | 64 | 76 | | 94 | 133 | | 163 |
| 14 | Vibrio proteolyticus | | | 53 | 65 | 77 | | 95 | 134 | | 163 |
| 15 | Pasteurella pneumotropica | | | | | 71 | 83 | | 128 | | 158 |
| 16 | Acinetobacter calcoaceticus | | | | | | | 86 | 122 | | 154 |
| 17 | Haemophilus influenzae | | | | | | | 88 | 126 | | 156 |
| 18 | Moraxella catarrhalis | | | | | | | 89 | 127 | | 157 |
| 19 | Budvicia aquatica | | | | 166 | | | | 96 | | 135 |
| 20 | Buttiauxella agrestis | | | 187 | 167 | | | | 97 | | 136 |
| 21 | Enterobacter agglomerans | | | 188 | 168 | | | | 98 | | |
| 22 | Erwinia carotovora | | | 189 | 169 | | | | 99 | | |
| 23 | Erwinia chrysanthemi | | | 190 | 170 | | | | 100 | | 138 |
| 24 | Escherichia coli | | | 187 | 171 | | | | 101 | | 139 |
| 25 | Escherichia hermannii | | | 191 | 172 | | | | 102 | | 140 |
| 26 | Escherichia vulneris | | | 192 | 173 | | | | 103, 165 | | 141 |
| 27 | Hafnia alvei | | | 193 | 174 | | | | 104 | | 142 |
| 28 | Klebsiella oxytoca | | | 187 | 175 | | | | 105, 165 | | 143 |
| 29 | Kluyvera cryoescens | | | 187 | 175 | | | | 106 | | 144 |
| 30 | Morganella morganii | | | 194 | 176 | | | | 107 | | 145 |
| 31 | Pantoea dispersa | | | 187 | 177 | | | | 108, 165 | | 146 |
| 32 | Pantoea | | | 188 | 178 | | | | 109, 165 | | 147 |
| 33 | Proteus mirabilis | | | 195 | 179 | | | | 110 | | |
| 34 | Proteus rettgeri | | | 196 | 180 | | | | 111 | | 148 |
| 35 | Providencia stuartii | | | 197 | 181 | | | | 112 | | 149 |
| 36 | Rahnella aquatilis | | | 198 | 182 | | | | 113, 164 | | 149 |
| 37 | Serratia ficaria | | | | | | | | 114, 164 | | 150 |
| 38 | Serratia fonticola | | | | | | | | 115, 164 | | |
| 39 | Serratia marcescens | | | | | | | | 116, 164 | | |
| 40 | Serratia plymuthica | | | | | | | | 117, 164 | | |
| 41 | Serratia proteamaculans | | | | | | | | 118, 164 | | |
| 42 | Serratia rubidea | | | | | | | | 119, 164 | | |
| 43 | Yersinia enterolytica | | | 199 | 184 | | | | 120, 164 | | 152 |
| 44 | Yersinia pseuaotuberculosis | | | 200 | 185 | | | | 121, 164 | | 153 |
| 45 | Aeromonas enteropelogenes | | | | | | | | 123 | | |
| 46 | Aeromonas hydrophila | | | | | | | | 124 | | |
| 47 | Stenotrophomonas multophila | | | 201 | 186 | | | | 125 | | |
| 48 | Stenotrophomonas multophila | | | | | | | | 129 | | 159 |
| 49 | Enterobacter agglomerans | | | | | | | | 137, 165 | | |
| 50 | Serratia | | | | 183 | | | | | | 151 |
| 51 | Citrobacter | | | | | | | | 202,203 | | |
| 52 | Salmonella | | | | | | | 204-210 | | | |
| 53 | Pseudomonas stutzeri | 213 | 252 | 289 | 326 | 361 | 403 | | 434 | | 488 |
| 54 | Thiobacillus ferrooxidans | 214 | 253 | 290 | 327 | 362 | 404 | | 435 | | 489 |
| 55 | Agrobacterium vitis | 215 | 254 | 291 | 328 | 363 | | | 436 | | 490 |
| 56 | Adalia bipunctata | 216 | 255 | 292 | 329 | 364 | | | 437 | | 491 |
| 57 | Amycolatopsis orientalis | 217 | 256 | 293 | 330 | | | | 438 | | |
| 58 | Brucella ovis | 218 | 257 | 294 | 331 | 365 | | | 439 | | 492 |
| 59 | Bradyrizobium japonicum | 219 | 258 | 295 | 331 | 366 | | | 440 | | 493 |
| 60 | Pseudomonas paucimobilis | 220 | 259 | 296 | 332 | 367 | | | 441 | | 494 |
| 61 | Rhodobacter sphaeroides | 221 | 260 | 297 | 333 | 368 | | | 442 | | 495 |
| 62 | Rickettsia prowazekii | 222 | 261 | 298 | 333 | 369 | | | 443 | | 496 |
| 63 | Sphingomonas paucimobilis | 223 | 262 | 299 | 334 | 370 | 405 | | 525 | | 499 |
| 64 | Zymomonas mobilis | 224 | 263 | 300 | 335 | 371 | | | 526 | | 500 |
| 65 | Alcaligenes | 225 | 264 | 301 | 336 | 372 | 406 | | 527 | | 501 |
| 66 | Pseudomonas cepacia | 226 | 265 | 302 | 337 | | 407 | | 444 | | 502 |
| 67 | Ralstonia pickettii | 227 | 266 | 303 | 338 | 373 | 408 | | 445 | | 503 |
| 68 | Campylobacter jejuni | 228 | 267 | 304 | 339 | 374 | 409 | | 446 | | |
| 69 | Helicobacter pylori | 229 | 268 | 305 | 340 | 375 | 410 | | 447 | | 504 |
| 70 | Actinoplanes utahensis | 230 | 269 | 306 | 341 | | 411 | | 448 | | |
| 71 | Bacillus halodurans | 231 | 270 | 307 | 342 | 376 | 412 | | 449 | | 505 |
| 72 | Bacillus subtilis | 232 | | | 343 | 377 | 413 | | 450 | | 506 |
| 73 | Clostridium tyrobutyricum | 233 | 271 | 308 | 344 | 378 | 414 | | 451 | | 507 |
| 74 | Frankia | 234 | 272 | 309 | 345 | 379 | 415 | | 452 | | 508 |
| 75 | Microbispora bispora | 235 | 273 | 310 | 346 | 380 | 416 | | 453 | | 509 |
| 76 | Mycobacterium leprae | 236 | 274 | 311 | 347 | 381 | 417 | | 454 | | 510 |
| 77 | Mycobacterium smegmatis | 237 | 275 | 312 | 348 | 382 | 418 | | 455 | | 511 |
| 78 | Mycobacterium tuberculosis | 238 | 276 | 313 | 349 | 383 | 419 | | 456 | | 512 |
| 79 | Mycobacterium gallisepticum | 239 | 277 | 314 | | 384 | 420 | | 457 | | |
| 80 | Propionibacterium freudenreich | 240 | 278 | 315 | 350 | 385 | 421 | | 458 | | |
| 81 | Rhodococcus erythropolis | 241 | 279 | 316 | 351 | 386 | 422 | | 459 | | 513 |
| 82 | Rhodococcus fascians | 242 | | | | 387 | 423 | | 460 | | 514 |
| 83 | Staphylococcus aureus | 243 | 280 | 317 | 352 | 388 | 424 | | 461 | | 515 |
| 84 | Streptococcus faecalis | 244 | 281 | 318 | 353 | 389 | 425 | | 462 | | 516 |
| 85 | Streptomyces ambifaciens | 245 | 282 | 319 | 354 | 390 | 426 | | 463 | | 517 |
| 86 | Flavobacterium resinovorum | 246 | 283 | 320 | 355 | 395 | 428 | | 468 | | 519 |
| 87 | Sphingobacterium multivorans | 247 | 284 | 321 | 356 | 396 | | | 469 | | 520 |
| 88 | Synechococcus | 248 | 285 | 322 | 357 | 397 | 429 | | 470 | | 521 |
| 89 | Synechocystis | 249 | 286 | 323 | 358 | 398 | 430 | | 471 | | 522 |
| 90 | Borrelia burgdorferi | 250 | 287 | 324 | 359 | 399 | | | 472,428 | | 523 |
| 91 | Chlamydia trachomatis | 251 | 288 | 325 | 360 | 400 | 431 | | 473 | | 524 |
| 92 | Streptomyces galbus | | | | | 391 | 426 | | 464 | | |
| 93 | Streptomyces griseus | | | | | 392 | 426 | | 465 | | 518 |
| 94 | Streptomyces lividans | | | | | 393 | 426 | | 466 | | 518 |
| 95 | Streptomyces mashuensis | | | | | 394 | 427 | | 467 | | |
| 96 | Salmonella typhi | | | | | | 401 | | 432 | | 486 |
| 97 | Buchnera aphidocola | | | | | | | | 433 | | 487 |
| 98 | Brucetia orientalis | | | | | | | | 439 | | 492 |
| 99 | Brucella abortus | | | | | | | | 439 | | 492 |
| 100 | Azotobacter vinelandii | | | | | | | | 474 | | |
| 101 | Cowduria ruminantium | | | | | | | | 475,530 | | |
| 102 | Mycobacterium intracellulare | | | | | | | | 476 | | |
| 103 | Mycobacterium lufu | | | | | | | | 477 | | |
| 104 | Mycobactenum simiae | | | | | | | | 478 | | |
| 105 | Mycobacterium smegmatis | | | | | | | | 479 | | |
| 106 | Saccharomonospora azurea | | | | | | | | 480 | | |
| 107 | Saccharomonospora caesia | | | | | | | | 481 | | |
| 108 | Saccharomonospora cyanea | | | | | | | | 482 | | |
| 109 | Saccharomonospora glauca | | | | | | | | 483 | | |
| 110 | Saccharomonospora viridis | | | | | | | | 484 | | |
| 111 | Wolbachia pipientis | | | | | | | | 485 | | |
| 112 | Rickettsia bellis | | | | | | | | | | 497 |
| 113 | Rickettsia rickettsii | | | | | | | | | | 498 |
| 114 | Xanthomonas campestris | | | | | | | | 529 | | |

## Patentansprüche

1. Nukleinsäuremoleküle als Sonde und/oder Primer geeignet zum Nachweis der taxonomischen Einheit der Enterobacteriaceae, ausgewählt aus
a) einem Nukleinsäuremolekül bestehend aus einer Sequenz mit einer der SEQ ID Nrn: 2-26, 34, 42, 54, 66, 78, 85, bzw. 135;
b) einem Nukleinsäuremolekül, das spezifisch mit einer Nukleinsäure nach a) hybridisiert;
c) einem Nukleinsäuremolekül, das mindestens 90% Identität zu einer Nukleinsäure nach a) aufweist;
d) einem Nukleinsäuremolekül, das komplementär zu einer Nukleinsäure nach a) bis
e) ist;
wobei das Nukleinsäuremolekül mindestens 14 Nukleotide lang ist.

2. Nukleinsäuremolekül nach einem der vorhergehenden Ansprüche, **dadurch gekennzeichnet, dass** das Nukleinsäuremolekül dadurch modifiziert ist, dass bis zu 20 % der Nukleotide in 10 aufeinanderfolgenden Nukleotiden, durch Nukleotide ersetzt sind, die in Bakterien nicht natürlich vorkommen.

3. Nukleinsäuremolekül nach einem der vorhergehenden Ansprüche, **dadurch gekennzeichnet, dass** das Nukleinsäuremolekül so modifiziert bzw. markiert ist, dass es in an sich bekannten analytischen Nachweisverfahren ein Signal erzeugen kann, wobei die Modifikation ausgewählt wird aus (i) radioaktiven Gruppen (ii) farbigen Gruppen, (iii) fluoreszierenden Gruppen, (iv) Gruppen zur Immobilisierung von einer festen Phase und (v) Gruppen, die eine indirekte oder direkte Reaktion mit Hilfe von Antikörpern, Antigenen, Enzymen und/oder Substanzen mit Affinität zu Enzymen oder Enzymkomplexen, erlauben.

4. Kombination aus mindestens 2 Nukleinsäuremolekülen gemäß Anspruch 1, ausgewählt von
a) SEQ ID NO: 2 mit SEQ ID NO: 7-22
b) SEQ ID NO: 2 mit SEQ ID NO: 23-24,
c) SEQ ID NO: 2 mit SEQ ID NO: 25
d) SEQ ID NO: 3-6 mit SEQ ID NO: 23-24,
e) SEQ ID NO: 3-6 mit SEQ ID NO: 25 und
f) SEQ ID NO: 26, 34, 42, 54, 66, 78, 85 mit SEQ ID NO: 135.

5. Kit, enthaltend ein Nukleinsäuremolekül oder eine Kombination an Nukleinsäuremolekülen nach einem der vorhergehenden Ansprüche.

6. Verfahren zum Nachweis der taxonomischen Einheit der Enterobakteriaceae, in einer Analysenprobe, umfassend den Schritt Inkontaktbringen der Analysenprobe mit einer Nukleinsäure oder einer Kombination an Nukleinsäuren nach einem der Ansprüche 1 bis 5 und Nachweis geeigneter Hybridnukleinsäuren, umfassend die eingebrachte Nukleinsäure und bakterielle Nukleinsäure.

7. Verfahren zur Amplifikation bakterieller DNA einer Vielzahl verschiedener taxonomischer Einheiten, unter Verwendung von Primern nach einem der Ansprüche 1 - 4, wobei
in einem ersten Amplifikationsschritt die DNA für die hohe taxonomische Einheit der Enterobacteriaceae mit konservierten Primern amplifiziert wird und in mindestens einem weiteren Amplifikationsschritt mit verschachtelten, zunehmend variablen Primern, Teile des ersten Amplifikationsfragmentes, die spezifisch für Gattungen, Arten oder Spezies sind, vermehrt werden können.

8. Verfahren nach einem der vorhergehenden Ansprüche, **dadurch gekennzeichnet, dass** das Verfahren eine PCR-Amplifikation der nachzuweisenden Nukleinsäure umfasst.

9. Verfahren nach einem der vorhergehenden Ansprüche, **dadurch gekennzeichnet, dass** das Verfahren eine Southernblothybridisierung umfasst.

10. Verwendung eines Nukleinsäuremoleküls nach einem der Ansprüche 1 bis 5 zum Nachweis der Familie der Enterobacteriaceae oder eines beliebigen Bakteriums der Familie der Enterobacteriaceae.

11. Verwendung eines Nukleinsäuremoleküls nach Anspruch 11, **dadurch gekennzeichnet, dass** der Nachweis eine Polymerasen-Kettenreaktion (PCR) umfasst.

12. Verwendung eines Nukleinsäuremoleküls nach Anspruch 11, **dadurch gekennzeichnet, dass** der Nachweis eine Ligase-Kettenreaktion umfasst.

13. Verwendung eines Nukleinsäuremoleküls nach Anspruch 11, **dadurch gekennzeichnet, dass** der Nachweis eine isotherme Nukleinsäureamplifikation umfasst.

14. Verwendung eines Nukleinsäuremoleküls nach einem der Ansprüche 1 bis 5 zum Identifizieren und/oder Charakterisieren von Bakterien.

## Claims

1. Nucleic acid molecules as a probe and/or primer suitable for the detection of the taxonomic unit of enterobacteriaceae, selected from
a) a nucleic acid molecule consisting of a sequence with one of the SEQ ID nos.: 2-26, 34, 42, 54, 66, 78, 85 or 135;
b) a nucleic acid molecule that hybridises specifically with a nucleic acid according to a);
c) a nucleic acid molecule that has at least 90% identity with a nucleic acid according to a);
d) a nucleic acid molecule that is complementary to a nucleic acid according to a) to c);
wherein the nucleic acid molecule is at least 14 nucleotides long.

2. Nucleic acid molecule according to one of the preceding claims, **characterised in that** the nucleic acid molecule is modified **in that** up to 20% of the nucleotides in 10 successive nucleotides are replaced by nucleotides that do not occur naturally in bacteria.

3. Nucleic acid molecule according to one of the preceding claims, **characterised in that** the nucleic acid molecule is so modified and labelled that it can
produce a signal in analytical methods of detection known in themselves, wherein the modification is selected from (i) radioactive groups (ii) coloured groups, (iii) fluorescent groups, (iv) groups for immobilising a solid phase and (v) groups that allow an indirect or direct reaction by means of antibodies, antigens, enzymes and/or substances with affinity to enzymes or enzyme complexes.

4. Combination of at least 2 nucleic acid molecules according to claim 1, selected from
a) SEQ ID NO: 2 with SEQ ID NO: 7-22
b) SEQ ID NO: 2 with SEQ ID NO: 23-24
c) SEQ ID NO: 2 with SEQ ID NO: 25
d) SEQ ID NO: 3-6 with SEQ ID NO: 23-24
e) SEQ ID NO: 3-6 with SEQ ID NO: 25 and
f) SEQ ID NO: 26, 34, 42, 54, 66, 78, 85 with SEQ ID NO: 135

5. Kit containing a nucleic acid molecule or a combination of nucleic acid molecules according to one of the preceding claims.

6. Method for the detection of the taxonomic unit of enterobacteriaceae in an analysis sample, comprising the step of bringing into contact the analysis sample with a nucleic acid or a combination of nucleic acids according to one of claims 1 to 5 and detection of suitable hybrid nucleic acids, comprising the introduced nucleic acid and bacterial nucleic acid.

7. Method for the amplification of bacterial DNA of a multiplicity of different taxonomic units using primers according to one of claims 1 - 4, wherein
in a first amplification step, the DNA for the high taxonomic unit of the enterobacteriaceae is amplified with preserved primers and in at least one further amplification step with nested, increasingly variable primers, parts of the first amplification fragment which are specific for genera, varieties or species can be multiplied.

8. Method according to one of the preceding claims, **characterised in that** the method comprises a PCR amplification of the nucleic acid to be detected.

9. Method according to one of the preceding claims, **characterised in that** the method comprises a southern blot hybridisation.

10. Use of a nucleic acid molecule according to one of claims 1 to 5 for the detection of the family of enterobacteriaceae or any bacterium of the family of enterobacteriaceae.

11. Use of a nucleic acid molecule according to claim 11, **characterised in that** the detection comprises a polymerase chain reaction (PCR).

12. Use of a nucleic acid molecule according to claim 11, **characterised in that** the detection comprises a ligase chain reaction.

13. Use of a nucleic acid molecule according to claim 11, **characterised in that** the detection comprises an isothermal nucleic acid amplification.

14. Use of a nucleic acid molecule according to one of claims 1 to 5 for identifying and/or characterising bacteria.

## Revendications

1. Molécules d'acide nucléique servant de sondes et/ou d'amorces appropriées pour la détection du motif taxonomique d'entérobactériacées, choisies parmi :
a) une molécule d'acide nucléique consistant en une séquence comportant l'un des numéros de SEQ ID : 2 à 26, 34, 42, 54, 66, 78, 85 ou 135 ;
b) une molécule d'acide nucléique qui s'hybride spécifiquement à un acide nucléique selon le point a) ;
c) une molécule d'acide nucléique qui présente au moins 90 % d'identité avec un acide nucléique selon le point a) ;
d) une molécule d'acide nucléique qui est complémentaire à l'un des acides nucléiques selon les points a) à c) ;
la molécule d'acide nucléique ayant une longueur d'au moins 14 nucléotides.

2. Molécule d'acide nucléique selon l'une des revendications précédentes, **caractérisée en ce que** la molécule d'acide nucléique est modifiée par le fait que jusqu'à 20 % des nucléotides dans 10 nucléotides consécutifs sont remplacés par des nucléotides qui ne sont pas présents à l'état naturel dans les bactéries.

3. Molécule d'acide nucléique selon l'une des revendications précédentes, **caractérisée en ce que** la molécule d'acide nucléique est modifiée ou marquée de telle sorte qu'elle puisse générer un signal dans un procédé de détection analytique en lui-même connu, la modification étant choisie parmi (i) des groupes radioactifs, (ii) des groupes colorés, (iii) des groupes fluorescents, (iv) des
groupes pour l'immobilisation d'une phase solide et (v) des groupes qui permettent une réaction indirecte ou directe à l'aide d'anticorps, d'antigènes, d'enzymes et/ou de substances présentant une affinité avec des enzymes ou des complexes enzymatiques.

4. Combinaison d'au moins 2 molécules d'acide nucléique selon la revendication 1, choisies parmi
a) SEQ ID N° : 2 avec SEQ ID N° : 7-22
b) SEQ ID N° : 2 avec SEQ ID N° : 23-24
c) SEQ ID N° : 2 avec SEQ ID N° : 25
d) SEQ ID N° : 3-6 avec SEQ ID N° : 23-24
e) SEQ ID N° : 3-6 avec SEQ ID N° : 25 et
f) SEQ ID N° : 26, 34, 42, 54, 66, 78, 85 avec SEQ ID N° : 135.

5. Kit contenant une molécule d'acide nucléique ou une combinaison de molécules d'acide nucléique selon l'une des revendications précédentes.

6. Procédé de détection du motif taxonomique d'entérobactériacées dans un échantillon d'analyse, comprenant l'étape de mise en contact de l'échantillon d'analyse avec un acide nucléique ou une combinaison d'acides nucléiques selon l'une des revendications 1 à 5, et détection d'acides nucléiques hybrides appropriés, comprenant l'acide nucléique intégré et un acide nucléique bactérien.

7. Procédé d'amplification d'ADN bactérien d'une multiplicité de différents motifs taxonomiques en utilisant des amorces selon l'une des revendications 1 à 4, dans lequel
dans une première étape d'amplification, l'ADN pour le motif taxonomique élevé d'entérobactériacées est amplifié avec des amorces conservées et dans au moins une autre étape d'amplification avec des amorces emboîtées de plus en plus variables, des parties du premier fragment d'amplification qui sont spécifiques des genres, sortes ou espèces, peuvent être multipliées.

8. Procédé selon l'une des revendications précédentes, **caractérisé en ce que** le procédé comprend une amplification par PCR de l'acide nucléique à détecter.

9. Procédé selon l'une des revendications précédentes, **caractérisé en ce que** le procédé comprend une hybridation par southern blot.

10. Utilisation d'une molécule d'acide nucléique selon l'une des revendications 1 à 5 pour la détection de la famille des entérobactériacées ou d'une bactéries quelconque de la famille des entérobactériacées.

11. Utilisation d'une molécule d'acide nucléique selon la revendication 11, **caractérisée en ce que** la détection comprend une réaction en chaîne polymérase (PCR).

12. Utilisation d'une molécule d'acide nucléique selon la revendication 11, **caractérisée en ce que** la détection comprend une réaction en chaîne ligase.

13. Utilisation d'une molécule d'acide nucléique selon la revendication 11, **caractérisée en ce que** la détection comprend une amplification d'acide nucléique isotherme.

14. Utilisation d'une molécule d'acide nucléique selon l'une des revendications 1 à 5, pour l'identification et/ou la caractérisation de bactéries.


### SEQUENZPROTOKOLL

<110> BioteCon Diagnostics GmbH
<120> Nukleinsäuremoleküle zum Nachweis von Bakterien und
   phylogenetischen Einheiten von Bakterien
<130> PCT1217-066
<140>
   <141>
<160> 530
<170> PatentIn Ver. 2.1
<210> 1
   <211> 3118
   <212> DNA.
   <213> Escherichia coli
<400> 1
   ggttaagcga ctaagcgtac acggtggatg ccctggcagt cagaggcgat gaaggacgtg 60
   ctaatctgcg ataagcgtcg gtaaggtgat atgaaccgtt ataaccggcg atttccgaat 120
   ggggaaaccc agtgtgtttc gacacactat cattaactga atccataggt taatgaggcg 180
   aaccggggga actgaaacat ctaagtaccc cgaggaaaag aaatcaaccg agattccccc 240
   agtagcggcg agcgaacggg gagcagccca gagcctgaat cagtgtgtgt gttagtggaa 300
   gcgtctggaa aggcgtgcga tacagggtga cagccccgta cacaaaaatg cacatgctgt 360
   gagctcgatg agtagggcgg gacacgtggt atcctgtctg aatatggggg gaccatcctc 420
   caaggctaaa tactcctgac tgaccgatag tgaaccagta ccgtgaggga aaggcgaaaa 480
   gaaccccggc gaggggagtg aaaaagaacc tgaaaccgtg tacgtacaag cagtgggagc 540
   acgcttaggc gtgtgactgc gtaccttttg tataatgggt cagcgactta tattctgtag 600
   caaggttaac cgaatagggg agccgaaggg aaaccgagtc ttaactgggc gttaagttgc 660
   agggtataga cccgaaaccc ggtgatctag ccatgggcag gttgaaggtt gggtaacact 720
   aactggagga ccgaaccgac taatgttgaa aaattagcgg atgacttgtg gctgggggtg 780
   aaaggccaat caaaccggga gatagctggt tctccccgaa agctatttag gtagcgcctc 840
   gtgaattcat ctccgggggt agagcactgt ttcggcaagg gggtcatccc gacttaccaa 900
   cccgatgcaa actgcgaata ccggagaatg ttatcacggg agacacacgg cgggtgctaa 960
   cgtccgtcgt gaagagggaa acaacccaga ccgccagcta aggtcccaaa gtcatggtta 1020
   agtgggaaac gatgtgggaa ggcccagaca gccaggatgt tggcttagaa gcagccatca 1080
   tttaaagaaa gcgtaatagc tcactggtcg agtcggcctg cgcggaagat gtaacggggc 1140
   taaaccatgc accgaagctg cggcagcgac actatgtgtt gttgggtagg ggagcgttct 1200
   gtaagcctgt gaaggtgtgc tgtgaggcat gctggaggta tcagaagtgc gaatgctgac 1260
   ataagtaacg ataaagcggg tgaaaagccc gctcgccgga agaccaaggg ttcctgtcca 1320
   acgttaatcg ggcagggtgt agtcgacccc taaggcgagg ccgaaaggcg tagtcgatgg 1380
   gaaacaggtt aatattcctg tacttggtgt tactgcgaag gggggacgga gaaggctatg 1440
   ttggccgggc gacggttgtc ccggtttaag cgtgtaggct ggttttccag gcaaatccgg 1500
   aaaatcaagg ctgaggcgtg atgacgaggc actacggtgc tgaagcaaca aatgccctgc 1560
   ttccaggaaa agcctctaag catcaggtaa catcaaatcg taccccaaac cgacacaggt 1620
   ggtcaggtag agaataccaa ggcgcttgag agaactcggg tgaaggaact aggcaaaatg 1680
   gtgccgtaac ttcgggagaa ggcacgctga tatgtaggtg aagcgacttg ctcgtggagc 1740
   tgaaatcagt cgaagatacc agctggctgc aactgtttat taaaaacaca gcactgtgca 1800
   aacacgaaag tggacgtata cggtgtgacg cctgcccggt gccggaaggt taattgatgg 1860
   ggttagccgc aaggcgaagc tcttgatcga agccccggta aacggcggcc gtaactataa 1920
   cggtcctaag gtagcgaaat tccttgtcgg gtaagttccg acctgcacga atggcgtaat 1980
   gatggccagg ctgtctccac ccgagactca gtgaaattga actcgctgtg aagatgcagt 2040
   gtacccgcgg caagacggaa agaccccgtg aacctttact atagcttgac actgaacatt 2100
   gagccttgat gtgtaggata ggtgggaggc tttgaagtgt ggacgccagt ctgcatggag 2160
   ccgaccttga aataccaccc tttaatgttt gatgttctaa cgttgacccg taatccgggt 2220
   tgcggacagt gtctggtggg tagtttgact ggggcggtct cctcctaaag agtaacggag 2280
   gagcacgaag gttggctaat cctggtcgga catcaggagg ttagtgcaat ggcataagcc 2340
   agcttgactg cgagcgtgac ggcgcgagca ggtgcgaaag caggtcatag tgatccggtg 2400
   gttctgaatg gaagggccat cgctcaacgg ataaaaggta ctccggggat aacaggctga 2460
   taccgcccaa gagttcatat cgacggcggt gtttggcacc tcgatgtcgg ctcatcacat 2520
   cctggggctg aagtaggtcc caagggtatg gctgttcgcc atttaaagtg gtacgcgagc 2580
   tgggtttaga acgtcgtgag acagttcggt ccctatctgc cgtgggcgct ggagaactga 2640
   ggggggctgc tcctagtacg agaggaccgg agtggacgca tcactggtgt tcgggttgtc 2700
   atgccaatgg cactgcccgg tagctaaatg cggaagagat aagtgctgaa agcatctaag 2760
   cacgaaactt gccccgagat gagttctccc tgactccttg agagtcctga aggaacgttg 2820
   aagacgacga cgttgatagg ccgggtgtgt aagcgcagcg atgcgttgag ctaaccggta 2880
   ctaatgaacc gtgaggctta accttacaac gccgaaggtg ttttggcgga ttgagagaag 2940
   attttcagcc tgatacagat taaatcagaa cgcagaagcg gtctgataaa acagaatttg 3000
   cctggcggca gtagcgcggt ggtcccacct gaccccatgc cgaactcaga agtgaaacgc 3060
   cgtagcgccg atggtagtgt ggggtctcct catgcgagag tagggaactg ccaggcat   3118
<210> 2
   <211> 20
   <212> DNA
   <213> Künstliche Sequenz
<220>
   <223> Beschreibung der künstlichen Sequenz: abgeleitetet
   von Gattungen der Enterobakterien
<400> 2
   ttcgggttgt catgccaatg 20
<210> 3
   <211> 26
   <212> DNA
   <213> Künstliche Sequenz
<220>
   <223> Beschreibung der künstlichen Sequenz: abgeleitetet
   von Gattungen der Enterobakterien
<400> 3
   ctgaaagcat ctaagcgcga aacttg 26
<210> 4
   <211> 26
   <212> DNA
   <213> Künstliche Sequenz
<220>
   <223> Beschreibung der künstlichen Sequenz: abgeleitetet
   von Gattungen der Enterobakterien
<400> 4
   ctgaaagcat ctaagcggga aacttg 26
<210> 5
   <211> 26
   <212> DNA
   <213> Künstliche Sequenz
<220>
   <223> Beschreibung der künstlichen Sequenz: abgeleitetet
   von Gattungen der Enterobakterien
<400> 5
   ctgaaagcat ctaagcacga aacttg 26
<210> 6
   <211> 26
   <212> DNA
   <213> Künstliche Sequenz
<220>
   <223> Beschreibung der künstlichen Sequenz: abgeleitetet
   von Gattungen der Enterobakterien
<400> 6
   ctgaaagcat ctaagcagga aacttg 26
<210> 7
   <211> 25
   <212> DNA
   <213> Künstliche Sequenz
<220>
   <223> Beschreibung der künstlichen Sequenz: abgeleitetet
   von Gattungen der Enterobakterien
<400> 7
   gggaggactc atctcgaggc aagtt 25
<210> 8
   <211> 25
   <212> DNA
   <213> Künstliche Sequenz
<220>
   <223> Beschreibung der künstlichen Sequenz: abgeleitetet
   von Gattungen der Enterobakterien
<400> 8
   gggaggactc atctcggggc aagtt 25
<210> 9
   <211> 25
   <212> DNA
   <213> Künstliche Sequenz
<220>
   <223> Beschreibung der künstlichen Sequenz: abgeleitetet
   von Gattungen der Enterobakterien
<400> 9
   gggaggactc atctcaaggc aagtt 25
<210> 10
   <211> 25
   <212> DNA
   <213> Künstliche Sequenz
<220>
   <223> Beschreibung der künstlichen Sequenz: abgeleitetet
   von Gattungen der Enterobakterien
<400> 10
   gggaggactc atctcagggc aagtt 25
<210> 11
   <211> 25
   <212> DNA
   <213> Künstliche Sequenz
<220>
   <223> Beschreibung der künstlichen Sequenz: abgeleitetet
   von Gattungen der Enterobakterien
<400> 11
   gggaggactc atcttgaggc aagtt 25
<210> 12
   <211> 25
   <212> DNA
   <213> Künstliche Sequenz
<220>
   <223> Beschreibung der künstlichen Sequenz: abgeleitetet
   von Gattungen der Enterobakterien
<400> 12
   gggaggactc atcttggggc aagtt 25
<210> 13
   <211> 25
   <212> DNA
   <213> Künstliche Sequenz
<220>
   <223> Beschreibung der künstlichen Sequenz: abgeleitetet
   von Gattungen der Enterobakterien
<400> 13
   gggaggactc atcttaaggc aagtt 25
<210> 14
   <211> 25
   <212> DNA
   <213> Künstliche Sequenz
<220>
   <223> Beschreibung der künstlichen Sequenz: abgeleitetet
   von Gattungen der Enterobakterien
<400> 14
   gggaggactc atcttagggc aagtt 25
<210> 15
   <211> 25
   <212> DNA
   <213> Künstliche Sequenz
<220>
   <223> Beschreibung der künstlichen Sequenz: abgeleitetet
   von Gattungen der Enterobakterien
<400> 15
   gggagaactc atctcgaggc aagtt 25
<210> 16
   <211> 25
   <212> DNA
   <213> Künstliche Sequenz
<220>
   <223> Beschreibung der künstlichen Sequenz: abgeleitetet
   von Gattungen der Enterobakterien
<400> 16
   gggagaactc atctcggggc aagtt 25
<210> 17
   <211> 25
   <212> DNA
   <213> Künstliche Sequenz
<220>
   <223> Beschreibung der künstlichen Sequenz: abgeleitetet
   von Gattungen der Enterobakterien
<400> 17
   gggagaactc atctcaaggc aagtt 25
<210> 18
   <211> 25
   <212> DNA
   <213> Künstliche Sequenz
<220>
   <223> Beschreibung der künstlichen Sequenz: abgeleitetet
   von Gattungen der Enterobakterien
<400> 18
   gggagaactc atctcagggc aagtt 25
<210> 19
   <211> 25
   <212> DNA
   <213> Künstliche Sequenz
<220>
   <223> Beschreibung der künstlichen Sequenz: abgeleitetet
   von Gattungen der Enterobakterien
<400> 19
   gggagaactc atcttgaggc aagtt 25
<210> 20
   <211> 25
   <212> DNA
   <213> Künstliche Sequenz
<220>
   <223> Beschreibung der künstlichen Sequenz: abgeleitetet
   von Gattungen der Enterobakterien
<400> 20
   gggagaactc atcttggggc aagtt 25
<210> 21
   <211> 25
   <212> DNA
   <213> Künstliche Sequenz
<220>
   <223> Beschreibung der künstlichen Sequenz: abgeleitetet
   von Gattungen der Enterobakterien
<400> 21
   gggagaactc atcttaaggc aagtt 25
<210> 22
   <211> 25
   <212> DNA
   <213> Künstliche Sequenz
<220>
   <223> Beschreibung der künstlichen Sequenz: abgeleitetet
   von Gattungen der Enterobakterien
<400> 22
   gggagaactc atcttagggc aagtt 25
<210> 23
   <211> 18
   <212> DNA
   <213> Künstliche Sequenz
<220>
   <223> Beschreibung der künstlichen Sequenz: abgeleitetet
   von Gattungen der Enterobakterien
<400> 23
   ccgccaggca aattcggt 18
<210> 24
   <211> 17
   <212> DNA
   <213> Künstliche Sequenz
<220>
   <223> Beschreibung der künstlichen Sequenz: abgeleitetet
   von Gattungen der Enterobakterien
<400> 24
   tcaggtggga ccaccgc 17
<210> 25
   <211> 18
   <212> DNA
   <213> Künstliche Sequenz
<220>
   <223> Beschreibung der künstlichen Sequenz: abgeleitetet
   von Gattungen der Enterobakterien
<400> 25
   ccgccaggca aattctgt 18
<210> 26
   <211> 54
   <212> DNA
   <213> Künstliche Sequenz
<220>
   <223> Beschreibung der künstlichen Sequenz: abgeleitetet
   von Arten der Gattung Enterobakterien
<400> 26
   ccggagtgga cgcaccactg gtgttcgggt tgtcatgcca atggcattgc ccgg 54
<210> 27
   <211> 54
   <212> DNA
   <213> Künstliche Sequenz
<220>
   <223> Beschreibung der künstlichen Sequenz: abgeleitet
   von von Arten der Gattung Acinetobacter
<400> 27
   ccagagtgga cgaacctctg gtgtaccggt tgtgacgcca gtcgcatcgc cggg 54
<210> 28
   <211> 54
   <212> DNA
   <213> Künstliche Sequenz
<220>
   <223> Beschreibung der künstlichen Sequenz:abgeleitet
   von von Arten der Gattung Aeromonas
<400> 28
   ccggagtgaa cgaacctctg gtgttcgggt tgtcacgcca gtggcactgc ccgg 54
<210> 29
   <211> 54
   <212> DNA
   <213> Künstliche Sequenz
<220>
   <223> Beschreibung der künstlichen Sequenz:abgeleitet
   von von Arten der Gattung Haemophilus
<400> 29
   ccggagtgga cgcatcactg gtgttccggt tgtgtcgcca gacgcattgc cggg 54
<210> 30
   <211> 54
   <212> DNA
   <213> Künstliche Sequenz
<220>
   <223> Beschreibung der künstlichen Sequenz:abgeleitet
   von von Arten der Gattung Moraxella
<400> 30
   ccggagtgga cgcatcactg gtgttccggt tgtgtcgcca gacgcattgc cggg 54
<210> 31
   <211> 54
   <212> DNA
   <213> Künstliche Sequenz
<220>
   <223> Beschreibung der künstlichen Sequenz:abgeleitet
   von von Arten der Gattung Pasteurella
<400> 31
   ccgggatgga cacaccgctg gtgtaccagt tgttctgcca agagcatcgc tggg 54
<210> 32
   <211> 54
   <212> DNA
   <213> Künstliche Sequenz
<220>
   <223> Beschreibung der künstlichen Sequenz:
   abgeleitetet von Arten der
   Gattung Stenotrophomonas
<400> 32
   ccggagtgga cgaacctctg gtgtaccggt tgtcacgcca gtggcattgc cggg 54
<210> 33
   <211> 54
   <212> DNA
   <213> Künstliche Sequenz
<220>
   <223> Beschreibung der künstlichen Sequenz:
   abgeleitetet von Arten der Gattungt Vibrio
<400> 33
   ccggagtgga cgaacctctg gtgttcgggt tgtgtcgcca gacgcattgc ccgg 54
<210> 34
   <211> 41
   <212> DNA
   <213> Künstliche Sequenz
<220>
   <223> Beschreibung der künstlichen Sequenz: abgeleitetet
   von Gattungen der Enterobakterien
<400> 34
   gagataaccg ctgaaagcat ctaagcggga aacttgcctc g 41
<210> 35
   <211> 41
   <212> DNA
   <213> Künstliche Sequenz
<220>
   <223> Beschreibung der künstlichen Sequenz: abgeleitet
   von von Arten der Gattung Acinetobacter
<400> 35
   gggataaccg ctgaaagcat ctaagcggga agcctacctc a 41
<210> 36
   <211> 41
   <212> DNA
   <213> Künstliche Sequenz
<220>
   <223> Beschreibung der künstlichen Sequenz:abgeleitet
   von von Arten der Gattung Aeromonas
<400> 36
   tcgataaccg ctgaaagcat ctaagcggga agcgagccct g 41
<210> 37
   <211> 41
   <212> DNA
   <213> Künstliche Sequenz
<220>
   <223> Beschreibung der künstlichen Sequenz:abgeleitet
   von von Arten der Gattung Haemophilus
<400> 37
   gagataagtg ctgaaagcat ctaagcacga aacttgccaa g 41
<210> 38
   <211> 42
   <212> DNA
   <213> Künstliche Sequenz
<220>
   <223> Beschreibung der künstlichen Sequenz:abgeleitet
   von von Arten der Gattung Moraxella
<400> 38
   gggataaccg ctgaaagcat ctaagcggga agcccacctt aa 42
<210> 39
   <211> 42
   <212> DNA
   <213> Künstliche Sequenz
<220>
   <223> Beschreibung der künstlichen Sequenz:abgeleitet
   von von Arten der Gattung Pasteurella
<400> 39
   gggataagtg ctgaaagcat ctaagcacga agcccccctc aa 42
<210> 40
   <211> 42
   <212> DNA
   <213> Künstliche Sequenz
<220>
   <223> Beschreibung der künstlichen Sequenz:
   abgeleitetet von Arten der Gattung
   Stenotrophomonas
<400> 40
   gagataaccg ctgaaagcat ctaagcggga aacttgcctt ga 42
<210> 41
   <211> 42
   <212> DNA
   <213> Künstliche Sequenz
<220>
   <223> Beschreibung der künstlichen Sequenz:
   abgeleitetet von Arten der Gattung Vibrio
<400> 41
   tcgataaccg ctgaaagcat ctaagcggga agcgagcctt ga 42
<210> 42
   <211> 24
   <212> DNA
   <213> Künstliche Sequenz
<220>
   <223> Beschreibung der künstlichen Sequenz: abgeleitetet
   von Gattungen der Enterobakterien
<400> 42
   agatgagtct tccctgggcc ttta 24
<210> 43
   <211> 24
   <212> DNA
   <213> Künstliche Sequenz
<220>
   <223> Beschreibung der künstlichen Sequenz: abgeleitet
   von von Arten der Gattung Acinetobacter
<400> 43
   agataagatt tccctaggac ttta 24
<210> 44
   <211> 24
   <212> DNA
   <213> Künstliche Sequenz
<220>
   <223> Beschreibung der künstlichen Sequenz:abgeleitet
   von von Arten der Gattung Aeromonas
<400> 44
   agatgagtca tccctgaccc cttg 24
<210> 45
   <211> 21
   <212> DNA
   <213> Künstliche Sequenz

<220>
   <223> Beschreibung der künstlichen Sequenz:abgeleitet
   von von Arten der Gattung Haemophilus
<400> 45
   agatgagtca tccctgactt t 21
<210> 46
   <211> 13
   <212> DNA
   <213> Künstliche Sequenz
<220>
   <223> Beschreibung der künstlichen Sequenz:abgeleitet
   von von Arten der Gattung Moraxella
<400> 46
   agataagatt tcc 13
<210> 47
   <211> 21
   <212> DNA
   <213> Künstliche Sequenz
<220>
   <223> Beschreibung der künstlichen Sequenz:abgeleitet
   von von Arten der Gattung Pasteurella
<400> 47
   agatgagatt tcccattacg c 21
<210> 48
   <211> 23
   <212> DNA
   <213> Künstliche Sequenz
<220>
   <223> Beschreibung der künstlichen Sequenz:
   abgeleitetet von Arten der Gattung
   Stenotrophomonas
<400> 48
   agatgagatt tcccggagcc ttg 23
<210> 49
   <211> 24
   <212> DNA
   <213> Vibrio alginolyticus
<400> 49
   agatgagttc tccctgatac ttta 24
<210> 50
   <211> 13
   <212> DNA
   <213> Vibrio fisheri

<400> 50
   agattagatt tcc 13
<210> 51
   <211> 24
   <212> DNA
   <213> Vibrio harbeyi
<400> 51
   agatgagtct tccctgggcc ttta 24
<210> 52
   <211> 24
   <212> DNA
   <213> Vibrio parahaemolyticus
<400> 52
   agatgagtct tccctgatac ttta 24
<210> 53
   <211> 24
   <212> DNA
   <213> Vibrio proteolyticus
<400> 53
   agatgagtct tccctggcac ttta 24
<210> 54
   <211> 32
   <212> DNA
   <213> Künstliche Sequenz
<220>
   <223> Beschreibung der künstlichen Sequenz: abgeleitetet
   von Gattungen der Enterobakterien
<400> 54
   agggtcctga agggacgttg aagactacga cg 32
<210> 55
   <211> 32
   <212> DNA
   <213> Künstliche Sequenz
<220>
   <223> Beschreibung der künstlichen Sequenz: abgeleitet
   von von Arten der Gattung Acinetobacter
<400> 55
   tgtcctctaa agagccgttc gagactagga cg 32
<210> 56
   <211> 32
   <212> DNA
   <213> Künstliche Sequenz
<220>
   <223> Beschreibung der künstlichen Sequenz:abgeleitet
   von von Arten der Gattung Aeromonas
<400> 56
   tgtcctctaa agagccgttc gagactagga cg 32
<210> 57
   <211> 31
   <212> DNA
   <213> Künstliche Sequenz
<220>
   <223> Beschreibung der künstlichen Sequenz:abgeleitet
   von von Arten der Gattung Haemophilus
<400> 57
   aagtcagtaa gggttgttgt agactacgac g 31
<210> 58
   <211> 26
   <212> DNA
   <213> Künstliche Sequenz
<220>
   <223> Beschreibung der künstlichen Sequenz:abgeleitet
   von von Arten der Gattung Moraxella
<400> 58
   ctaaagagcc gttgtagacg acgacg 26
<210> 59
   <211> 31
   <212> DNA
   <213> Künstliche Sequenz
<220>
   <223> Beschreibung der künstlichen Sequenz:abgeleitet
   von von Arten der Gattung Pasteurella
<400> 59
   aagtaagtaa gatccctcaa agacgatgag g 31
<210> 60
   <211> 32
   <212> DNA
   <213> Künstliche Sequenz
<220>
   <223> Beschreibung der künstlichen Sequenz:
   abgeleitetet von Arten der Gattung
   Stenotrophomonas
<400> 60
   agctccttga agggtcgttc gagaccagga cg 32
<210> 61
   <211> 32
   <212> DNA
   <213> Vibrio alginolyticus
<400> 61
   agtatcctaa agggttgtcg tagmtacgac gt 32
<210> 62
   <211> 27
   <212> DNA
   <213> Vibrio fisheri
<400> 62
   ctaaagagcc gttcaagact aggacgt 27
<210> 63
   <211> 33
   <212> DNA
   <213> Vibrio harbeyi
<400> 63
   agtatcctaa agggttgttc gagactagaa cgt 33
<210> 64
   <211> 33
   <212> DNA
   <213> Vibrio parahaemolyticus
<400> 64
   agtatcctaa agggttgttc gagactagaa cgt 33
<210> 65
   <211> 33
   <212> DNA
   <213> Vibrio proteolyticus
<400> 65
   agtgtcctga agggttgttc gagactagaa cgt 33
<210> 66
   <211> 40
   <212> DNA
   <213> Künstliche Sequenz
<220>
   <223> Beschreibung der künstlichen Sequenz: abgeleitetet
   von Gattungen der Enterobakterien
<400> 66
   agcgatgcgt tgagctaacc agtactaatg acccgtgagg 40
<210> 67
   <211> 40
   <212> DNA
   <213> Künstliche Sequenz
<220>
   <223> Beschreibung der künstlichen Sequenz: abgeleitet
   von von Arten der Gattung Acinetobacter
<400> 67
   agtgatatgt gaagctgacc aatactaatt gctcgtgagg 40
<210> 68
   <211> 40
   <212> DNA
   <213> Künstliche Sequenz
<220>
   <223> Beschreibung der künstlichen Sequenz: abgeleitet
   von von Arten der Gattung Aeromonas
<400> 68
   ggcgacgtgt tgagctaacc catactaatt acccgtgagg 40
<210> 69
   <211> 40
   <212> DNA
   <213> Künstliche Sequenz
<220>
   <223> Beschreibung der künstlichen Sequenz:abgeleitet
   von von Arten der Gattung Haemophilus
<400> 69
   tgtgagtcat tgagctaacc aatactaatt gcccgagagg 40
<210> 70
   <211> 40
   <212> DNA
   <213> Künstliche Sequenz
<220>
   <223> Beschreibung der künstlichen Sequenz:abgeleitet
   von von Arten der Gattung Moraxella
<400> 70
   agtgatacat gtagctaacc aatactaatt gctcgtttgg 40
<210> 71
   <211> 47
   <212> DNA
   <213> Pasteurella pneumotropica
<400> 71
   tggcgacacg tgcagctgac gaatactaat cgatcgagga cttaacc 47
<210> 72
   <211> 40
   <212> DNA
   <213> Künstliche Sequenz
<220>
   <223> Beschreibung der künstlichen Sequenz:
   abgeleitetet von Arten der Gattung
   Stenotrophomonas
<400> 72
   agtaatgcat taagctaacc agtactaatt gcccgtacgg 40
<210> 73
   <211> 40
   <212> DNA
   <213> Vibrio alginolyticus
<400> 73
   tgtgaggcgt tgagctaacc tgtactaatt gcccgtgagg 40
<210> 74
   <211> 40
   <212> DNA
   <213> Vibrio fisheri
<400> 74
   agtgatgcgt gtagctaacc tgtactaatt gctcgtttgg 40
<210> 75
   <211> 40
   <212> DNA
   <213> Vibrio harveyi
<400> 75
   tgtgaggcgt tgagctaacc tgtactaatt gcccgtgagg 40
<210> 76
   <211> 40
   <212> DNA
   <213> Vibrio paramaemolyticus
<400> 76
   tgtgaggcat tgagctaact gatactaatt gcccgtgagg 40
<210> 77
   <211> 40
   <212> DNA
   <213> Vibrio proteolyticus
<400> 77
   tgtgaggcgt tgagctaacc tgtactaatt gcccgtgagg 40
<210> 78
   <211> 30
   <212> DNA
   <213> Künstliche Sequenz
<220>
   <223> Beschreibung der künstlichen Sequenz: abgeleitetet
   von Gattungen der Enterobakterien
<400> 78
   acccgtgagg cttaacctta caacaccgaa 30
<210> 79
   <211> 30
   <212> DNA
   <213> Künstliche Sequenz
<220>
   <223> Beschreibung der künstlichen Sequenz: abgeleitet
   von von Arten der Gattung Acinetobacter
<400> 79
   gctcgtgagg cttgactata caacacccaa 30
<210> 80
   <211> 30
   <212> DNA
   <213> Künstliche Sequenz
<220>
   <223> Beschreibung der künstlichen Sequenz: abgeleitet
   von von Arten der Gattung Aeromonas
<400> 80
   acccgtgagg cttaaccata caacacccaa 30
<210> 81
   <211> 30
   <212> DNA
   <213> Künstliche Sequenz
<220>
   <223> Beschreibung der künstlichen Sequenz:abgeleitet
   von von Arten der Gattung Haemophilus
<400> 81
   gcccgagagg cttaactata caacgctcaa 30
<210> 82
   <211> 30
   <212> DNA
   <213> Künstliche Sequenz
<220>
   <223> Beschreibung der künstlichen Sequenz:abgeleitet
   von von Arten der Gattung Moraxella
<400> 82
   gctcgtttgg cttgaccata caacacccaa 30
<210> 83
   <211> 33
   <212> DNA
   <213> Pasteurella pneumotropica
<400> 83
   gctgacgaat actaatcgat cgaggactta acc 33
<210> 84
   <211> 30
   <212> DNA
   <213> Künstliche Sequenz
<220>
   <223> Beschreibung der künstlichen Sequenz: abgeleitet
   von Arten der Gattung Stenotrophomonas
<400> 84
   gcccgtacgg cttgtcccta taaccttggt 30
<210> 85
   <211> 27
   <212> DNA
   <213> Künstliche Sequenz
<220>
   <223> Beschreibung der künstlichen Sequenz: abgeleitetet
   von Gattungen der Enterobakterien
<400> 85
   caacaccgaa ggtgttttgg aggaatc 27
<210> 86
   <211> 27
   <212> DNA
   <213> Acinetobacter calcoaceticus
<400> 86
   caacacccaa gcagttgtat ataaagc 27
<210> 87
   <211> 27
   <212> DNA
   <213> Künstliche Sequenz
<220>
   <223> Beschreibung der künstlichen Sequenz: abgeleitet
   von von Arten der Gattung Aeromonas
<400> 87
   caacacccaa gaagtgttct aaggctt 27
<210> 88
   <211> 27
   <212> DNA
   <213> Haemophilus influenzae
<400> 88
   caacgctcaa gtgtttttgg gagctaa 27
<210> 89
   <211> 27
   <212> DNA
   <213> Moraxella catarrhalis
<400> 89
   caacacccaa gtggtttacc actgact 27
<210> 90
   <211> 27
   <212> DNA
   <213> Künstliche Sequenz
<220>
   <223> Beschreibung der künstlichen Sequenz:
   abgeleitetet von Arten der Gattung
   Stenotrophomonas
<400> 90
   taaccttggt agtccaaggt cgagtac 27
<210> 91
   <211> 27
   <212> DNA
   <213> Vibrio alginolyticus
<400> 91
   caacacccaa ggggttttga tggactc 27
<210> 92
   <211> 27
   <212> DNA
   <213> Vibrio fisheri
<400> 92
   caacacccaa gtggtttgta tcaagca 27
<210> 93
   <211> 27
   <212> DNA
   <213> Vibrio harveyi
<400> 93
   caacacccaa ggggttttga tggactc 27
<210> 94
   <211> 27
   <212> DNA
   <213> Vibrio paramaemolyticus
<400> 94
   caacacccaa ggggttttga tggactc 27
<210> 95
   <211> 36
   <212> DNA
   <213> Vibrio proteolyticus
<400> 95
   caacacccaa ggggttttga tggactcaat gaaaga 36
<210> 96
   <211> 118
   <212> DNA
   <213> Budvicia aquatica
<400> 96
   caacatccga ggtgttttaa ggaaagttga agagacgaaa gaataagtag aattccagct 60
   tgaaccgaga ttgagttgat ggttgtgtga atgacacgac ggtcaataga cagaatat   118
<210> 97
   <211> 111
   <212> DNA
   <213> Buttiauxella agrestis
<400> 97
   caacaccgaa ggtgttttgg ttgagagact aagatattga attttcagct tgaaccgaga 60
   ttttaagtcg atggttgtgt gaacagcatg acggttgatg aaacagaata t          111
<210> 98
   <211> 193
   <212> DNA
   <213> Enterobacter aglomerans
<400> 98
   caacgccgaa gatgttttgg cggattgaga agattttcag cattgattac agattttcgg 60
   gaacgaaaga ttttacgctg aggcaaggcg gcaaatgaag taaaggaagg agcatacatg 120
   agtatgtgac tgactttgcg aatgcagcca acgcagccac agtgaaaaag attcgtttct 180
   ggcaacagaa ttt                                                    193
<210> 99
   <211> 123
   <212> DNA
   <213> Erwinia carotovora
<400> 99
   caacaccgaa ggtgttttga gagtgactca aagagatgtt gataatcagc ttgttttagg 60
   attggttctg atggttatgc gagagcgaaa gcgaagcatg acggttggga tgaaacagaa 120
   ttt                                                               123
<210> 100
   <211> 101
   <212> DNA
   <213> Erwinia chrysanthemi
<400> 100
   caacaccgaa ggtgttttag agagattggt ttgaattttc agtgaagttc cgagattggt 60
   tctgatggct acggagtagc ggtcgggatg aaacaaaatt t                     101
<210> 101
   <211> 92
   <212> DNA
   <213> Escherichia coli
<400> 101
   caacgccgaa gctgttttgg cggatgagag aagattttca gcctgataca gattaaatca 60
   gaacgcagaa gcggtctgat aaaacagaat tt                               92
<210> 102
   <211> 104
   <212> DNA
   <213> Escherichia hermannii
<400> 102
   caacgccaga gtggttttgg tgttgcggtg tgagagacga ttttcagctt gaccggatag 60
   acatctgtgg cggcgcgcga gcacgcagca ggtgaacaga attt                  104
<210> 103
   <211> 92
   <212> DNA
   <213> Escherichia vulneris
<400> 103
   caacgccgaa gatgttttgg cggatttgaa agacgatttt cagctgatac agattaagtc 60
   tgccgcctga cggcgtcaga cagacagaat tt                               92
<210> 104
   <211> 119
   <212> DNA
   <213> Hafnia alvei
<400> 104
   caacaccgaa ggtgttttaa gacgcagaga cgcgaaaaca caaagagtaa gcttgttgaa 60
   cagattggtt tgtatggcta gctgtagaaa tacagaaagc ggtacaaata acagaatat  119
<210> 105
   <211> 195
<212> DNA
   <213> Klebsiella oxytoca
<400> 105
   cgccgaagat gttttggcga tttgagaaga caacaatttc agcattgatt acagattttc 60
   gggaacgaaa gattttacgc tgaggcaagg cggcaaatga aggaaaggaa ggagcatact 120
   gaagtatgtg actgacttta cgaatgcagc caacgcagca tcggtgtaaa agattcgttt 180
   ctgacaacag aattt                                                  195
<210> 106
   <211> 90
   <212> DNA
   <213> Kluyvera cryoescens
<400> 106
   cgccaaagat gttttggtga aaagagacat caataatcag cttgatacag ataaattaac 60
   tggccgaaag gcgggttaat aacagaattt                                  90
<210> 107
   <211> 105
   <212> DNA
   <213> Morganella morganii
<400> 107
   caccgaaggt gttttgagtt gagagacgat taaagagatt tttcagcaca gtgaagaggc 60
   agaagtcatt cactgtgaaa gcttattttg gattgaaatg aattt                 105
<210> 108
   <211> 192
   <212> DNA
   <213> Pantoea dispersa
<400> 108
   cgccagaggc gttttggtct gagagaccna aagaattttc agcattgttc accggattac 60
   ntccagtgga ttttgtgctg tgacaaggcg gcacgcgaga cgacgggaag gagcatacac 120
   gagtatgtga ctgagcggcg cgagcggggc aacgcagtca gagcgcaaaa gacgcggtnt 180
   aaaacaaaat tt                                                     192
<210> 109
   <211> 190
   <212> DNA
   <213> Künstliche Sequenz
<220>
   <223> Beschreibung der künstlichen Sequenz: abgeleitet
   von Arten der Gattung Pantoea
<400> 109
   cgccgaagat gttttggcgg aatgagaaga ttttcagcat tgattacaga ttttcgggaa 60
   cgaaagattt tacgctgagg caaggcggca aatgaagtaa aggaaggagc atacatgagt 120
   atgtgactga ctttkcggat gcagccaacg cagccacagt gaaaaagatt cgtttctggc 180
   aacagaattt                                                        190
<210> 110
   <211> 111
   <212> DNA
   <213> Proteus mirabilis
<400> 110
   caacaccgaa agtgttttgt cagagagacg aaacgatgaa gtcagcttgt tcaanattga 60
   attactggcg acttaccgaa aggaaagaag cgagtgatta aaaccgaatt t 111
<210> 111
   <211> 139
   <212> DNA
   <213> Proteus rettgeri
<400> 111
   caacaccgaa ggtgttttag agagatagag ttgttttcaa gaaagagtga gaagccaaaa 60
   ggtgaaggac acgcagcttg tttgagattg aggttctggt ttagtgaaga aaaaactaaa 120
   cgggaacaaa acagaattt                                              139
<210> 112
   <211> 137
   <212> DNA
   <213> Providencia stuartii
<400> 112
   caacaccgaa ggtgttttag agagacgaag agacgaattg ttgaagcgca cgagatagag 60
   tggtgcgaaa aaatcagctt gttcaagatt gcagttctgg tttgcggtgt agacgcgaac 120
   gggaacgaac cgaattt 137
<210> 113
   <211> 135
   <212> DNA
   <213> Rahnella aquatilis
<400> 113
   caacaccgaa ggtgtttttg atttgagaga cagactcgag agagtagatt ttcagcgaat 60
   tgttccggta ttggttcgta tggcggcgtg tgatgagaaa ttatgacacg acgcggtatg 120
   aatgaaacag aattt 135
<210> 114
   <211> 100
   <212> DNA
   <213> Serratia ficaria
<400> 114
   caacaccgaa ggtgttttag agagacgaat aattttcagc gaagttctta gattggttct 60
   ggtggttacg cgagtaacgg ccaagaatga aacagaattt 100
<210> 115
   <211> 106
   <212> DNA
   <213> Serratia fonticola
<400> 115
   caacacccaa ggtgttttga agagattgaa gtagattttc agcgaagttc cgagattggt 60
   ttcaatggcg acacgagagt gaagcggttg aaatgaaaca gaattt 106
<210> 116
   <211> 97
   <212> DNA
   <213> Serratia marcescens
<400> 116
   caacaccgaa ggtgttttta gagagatttt cagcgaagtt ccgagattgg ttctgatggc 60
   gacacgaaag tgaagcggtt ggaatgaaac agaattt 97
<210> 117
   <211> 99
   <212> DNA
   <213> Serratia plymuthica
<400> 117
   caacaccgaa ggtgttttag agagattaca gtagattttc agcgacgttc cgagattggt 60
   ttcaatggcc caaaaggcgg ttggaatgaa acagaattt                        99
<210> 118
   <211> 100
   <212> DNA
   <213> Serratia proteamaculans
<400> 118
   caacaccaaa ggtgttttag agagattgta gagattttca gcgagttccg agattggttt 60
   caatggctgc gagagtagcg gttggaatga aacanaattt                       100
<210> 119
   <211> 101
   <212> DNA
   <213> Serratia rubidea
<400> 119
   caacaccgaa ggtgttttag agagattggt ttgaattttc agtgaagttc cgagattggt 60
   tctgatggct acggagtagc ggtcgggatg aaacagaatt t                     101
<210> 120
   <211> 116
   <212> DNA
   <213> Yersinia enterolytica
<400> 120
   caacaccaaa ggtgttttgt atttgagaga tagatattga ttttcagcga atgttccgag 60
   attgggctgg ctggctgtgt gaaagattgc atagcgggtt agtttagaca gaattt     116
<210> 121
   <211> 104
   <212> DNA
   <213> Yersinia pseudotuberculosis
<400> 121
   caacaccgaa gtcttgaatt gagagagatt ttcagcgtcg ttccgagatt ggattgactg 60
   gcgtcacaag cgctgtttgt gtgcgggtta attaaaacag attt                  104
<210> 122
   <211> 179
   <212> DNA
   <213> Acinetobacter calcoaceticus
<400> 122
   caacacccaa gcagttgtat ataaagcatc aatcgattca ttaatatgca aagcaacttg 60
   atttagttat acgcttagct aaaatgaaca aaatatagta agactcaatc agcccatctg 120
   taaagatttg gaaaacgcat cggcaaccaa taagaccaat gcaagtatcc ataccagtt 179
<210> 123
   <211> 118
   <212> DNA
<213> Aeromonas enteropelogenes
<400> 123
   caacacccaa gaagtgtttn tggtgcttgt agcgaatgaa cgaactacgc attcagtgat 60
   aacgacaagc cacgagcaac atcgttattc acgtcagctt tccaagattg aagatttt 118
<210> 124
   <211> 81
   <212> DNA
   <213> Aeromonas hydrophila
<400> 124
   caacacccaa gaagtgttct aaggcttgta gcagataccg agaacgaaca acaaaatcag 60
   ctttctcaga ttgaagaatt t 81
<210> 125
   <211> 96
   <212> DNA
   <213> Cedecea davisae
<400> 125
   caacaccaaa ggtgttttgc gagacgcaat tttaattttc agcgaagttc aggattagac 60
   tgatggtcac aaagtgacgg tcagtaaaca gaattt 96
<210> 126
   <211> 217
   <212> DNA
   <213> Haemophilus influenzae
<400> 126
   caacgctcaa gtgtttttgg gagctaagtg aagtaagaga tgaaaagcga agcaaataaa 60
   agcagagcga aagagaagta aaagactaaa caaagaaaag taaatataga agacttaata 120
   gaaagaaaat cggattcagc ttgtgaccaa taagaacgag tgaaaggtag aggaaagact 180
   gagtaacgag agataaaaga gacgagagat aaaagag 217
<210> 127
   <211> 90
   <212> DNA
   <213> Moraxella catarrhalis
<400> 127
   caacacccaa gtggtttacc actgactgtg ttgattggta atatataaga tgaaccttaa 60
   tcttgatttg gtaataaaca gactcataca 90
<210> 128
   <211> 134
   <212> DNA
   <213> Pasteurella pneumotropica
<400> 128
   cgaggactta accaaatttg tttatcgtaa caatgtcgtt tatccagttt tgaaagaata 60
   aatttttatt aaataactct tgcattattc tacagagttg ttataataaa acatgtcctt 120
   caaaagtatt caag 134
<210> 129
   <211> 141
   <212> DNA
   <213> Stenotrophomonas multophila
<400> 129
   taaccttggt agtccaaggt cgagtacaac tgctcgatac aaaagctaca acccnactta 60
   cttcttccag attcatggcc acgctgaaca aagcgtaggg tgggcggctg tnccgcccac 120
   gcgtaactca agcgtagcca g 141
<210> 130
   <211> 100
   <212> DNA
   <213> Vibrio alginolyticus
<400> 130
   caacacccaa ggggttttga tggactcaat gaaagaacat tgaatgtgta agaacgagaa 60
   ttaaaaaaca gctttccaga ttaaagaatt tgcttggcga 100
<210> 131
   <211> 122
   <212> DNA
   <213> Vibrio fisheri
<400> 131
   caacacccaa gtggtttgta tcaagcatta tatcgatatc accgttatcc ttgattcagt 60
   taggataagt gatacttaag tcattaagta aaacaaacac agactcatat ctaaccccct 120
   tt 122
<210> 132
   <211> 122
   <212> DNA
   <213> Vibrio harveyi
<400> 132
   caacacccaa gtggtttgta tcaagcatta tatcgatatc accgttatcc ttgattcagt 60
   taggataagt gatacttaag tcattaagta aaacaaacac agactcatat ctaaccccct 120
   tt 122
<210> 133
   <211> 89
   <212> DNA
   <213> Vibrio paramaemolyticus
<400> 133
   caacacccaa ggggttttga tggactcgaa gcaagaacag aattgaatgt gtagagaaca 60
   caaaaacagc tttccgaatt aaagaattt 89
<210> 134
   <211> 169
   <212> DNA
   <213> Vibrio proteolyticus
<400> 134
   caacacccaa ggggttttga tggactcaat gaaagaacat tgaatgtgta agaacgagaa 60
   ttaaaaaaca gctttccgaa tttaggaatt gaatttatta acgacatcca tgtcgttaac 120
   ccttcgggcc gcactgaagt gcgttaaatt ttgttccaga caaaatttt 169
<210> 135
   <211> 33
   <212> DNA
   <213> Künstliche Sequenz
<220>
<223> Beschreibung der künstlichen Sequenz: abgeleitetet
   von Gattungen der Enterobakterien
<400> 135
   gcctggcggc actagcgcgg tggtcccacc tga 33
<210> 136
   <211> 33
   <212> DNA
   <213> Buttiauxella agrestis
<400> 136
   gcctggcggc agtagcgcgg tggtcccacc tga 33
<210> 137
   <211> 33
   <212> DNA
   <213> Enterobacter agglomerans
<400> 137
   gcctggcggc tttagcgcgg tggtcccacc tga 33
<210> 138
   <211> 33
   <212> DNA
   <213> Erwinia carotovora
<400> 138
   gcctggcggc gatagcgcgg tggtcccacc tga 33
<210> 139
   <211> 33
   <212> DNA
   <213> Erwinia chrysanthemi
<400> 139
   gcctggcggc ggtagcgcgg tggtcccacc tga 33
<210> 140
   <211> 33
   <212> DNA
   <213> Escherichia coli
<400> 140
   gcctggcggc agtagcgcgg tggtcccacc tga 33
<210> 141
   <211> 33
   <212> DNA
   <213> Escherichia hermannii
<400> 141
   gcctggcggc aagagcgcgg tggtcccacc tga 33
<210> 142
   <211> 33
   <212> DNA
   <213> Escherichia vulneris
<400> 142
   gcctggcggc actagcgcgg tggtcccacc tga 33
<210> 143
   <211> 33
   <212> DNA
   <213> Hafnia alvei
<400> 143
   gcctggcggc gatagcgcgg tggtcccacc tga 33
<210> 144
   <211> 32
   <212> DNA
   <213> Klebsiella oxytoca
<400> 144
   gcctggcggc actagcgcgg tggtccacct ga 32
<210> 145
   <211> 33
   <212> DNA
   <213> Kluyvera cryoescens
<400> 145
   gcctggcggc aacagcgcgg tggtcccacc tga 33
<210> 146
   <211> 33
   <212> DNA
   <213> Morganella morganii
<400> 146
   gcctggcggc cgtagcgcgg tggtcccacc tga 33
<210> 147
   <211> 31
   <212> DNA
   <213> Pantoea dispersa
<400> 147
   gcctggcggc aacagccgcg gtggtcccac c 31
<210> 148
   <211> 33
   <212> DNA
   <213> Proteus mirabilis
<400> 148
   gcttggtggc catagcgcgg tggtcccacc tga 33
<210> 149
   <211> 33
   <212> DNA
   <213> Künstliche Sequenz
<220>
   <223> Beschreibung der künstlichen Sequenz: abgeleitet
   von Arten der Gattungen Proteus, Providencia
<400> 149
   gtctggcggc aatagcacgg tggtcccacc tga 33
<210> 150
   <211> 33
   <212> DNA
   <213> Rahnella aquatilis
<400> 150
   gcctggcggc agtagcgcgg tggtcccacc tga 33
<210> 151
   <211> 33
   <212> DNA
   <213> Künstliche Sequenz
<220>
   <223> Beschreibung der künstlichen Sequenz: abgeleitet
   von Arten der Gattung Serratia

<400> 151
   gcctggcggc aatagcgcgg tggtcccacc tga 33
<210> 152
   <211> 33
   <212> DNA
   <213> Yersinia enterolytica
<400> 152
   gcctggcggc catagcgcgg tggacccacc tga 33
<210> 153
   <211> 33
   <212> DNA
   <213> Yersinia pseudotuberculosis
<400> 153
   gtctggcggc catagcgcgg tggtcycacc tga 33
<210> 154
   <211> 51
   <212> DNA
   <213> Acinetobacter calcoaceticus
<400> 154
   aagtatccat accagttgtg ctggcgacca tagcaagagt gaaccacctg a 51
<210> 155
   <211> 33
   <212> DNA
   <213> Künstliche Sequenz
<220>
   <223> Beschreibung der künstlichen Sequenz: abgeleitet
   von Arten der Gattung Aeromonas
<400> 155
   gcctggcggc catagcgccg tggaaccacc tga 33
<210> 156
   <211> 51
   <212> DNA
   <213> Haemophilus influenzae
<400> 156
   aaaagacgag ttatcaaaga attatcctgg cggcgatagt gcggtggacc c 51
<210> 157
   <211> 54
   <212> DNA
   <213> Moraxella catarrhalis
<400> 157
   acagcgttgt taatcctttt acgctgacga caatagcaag atggaaccac ctga 54
<210> 158
   <211> 43
   <212> DNA
   <213> Pasteurella pneumotropica
<400> 158
   tctagtgatg atggcgaaga ggtcacaccc gttcccatac cga 43
<210> 159
   <211> 54
   <212> DNA
   <213> Stenotrophomonas multophila
<400> 159
   acaagtcaaa gcctgatgac catagcaagt cggtcccacc ccttcccatc ccga 54
<210> 160
   <211> 33
   <212> DNA
   <213> Vibrio alginolyticus
<400> 160
   gcttggcgac catagcgttt tggacccacc tga 33
<210> 161
   <211> 51
   <212> DNA
   <213> Vibrio fisheri
<400> 161
   ctcatatcta accccctttg ctgacgacaa tagcacgatg gcaccacctg a 51
<210> 162
   <211> 45
   <212> DNA
   <213> Vibrio harveyi
<400> 162
   gcttggcgac catagcgatt tggacccacc tgacttccat tccga 45
<210> 163
   <211> 33
   <212> DNA
   <213> Vibrio proteolyticus
<400> 163
   gcttggcgac catagcgttt tggacccacc tga 33
<210> 164
   <211> 37
   <212> DNA
   <213> Künstliche Sequenz
<220>
   <223> Beschreibung der künstlichen Sequenz: abgeleitet
   von Arten der Gattungen Rahnella, Serratia,
   Yersinia
<400> 164
   agattttcag cgaagttccg agattggttt caatggc 37
<210> 165
   <211> 18
   <212> DNA
   <213> Künstliche Sequenz
<220>
   <223> Beschreibung der künstlichen Sequenz: abgeleitet
   von Arten der Gattungen Enterobacter, Escherichia,
   Klebsiella, Pantoea
<400> 165
   ggaaggagca taciiiagtat 18
<210> 166
   <211> 32
   <212> DNA
   <213> Budvicia aquatica
<400> 166
   aggtccctga aggaacgttt gagactaaga cg 32
<210> 167
   <211> 32
   <2i2> DNA
   <213> Buttiauxella agrestis

<400> 167
   agggtcctga aggaacgttg aagactacga cg 32
<210> 168
   <211> 32
   <212> DNA
   <213> Enterobacter agglomerans
<400> 168
   aggacactaa aggaacgttg aagacgacga cg 32
<210> 169
   <211> 32
   <212> DNA
   <213> Erwinia carotovora
<400> 169
   atgcccctga agggccgttg aagactacga cg 32
<210> 170
   <211> 32
   <212> DNA
   <213> Erwinia chrysanthemi
<400> 170
   aggcccctga agggacgttt aagacgaaga cg 32
<210> 171
   <211> 29
   <212> DNA
   <213> Escherichia coli
<400> 171
   agggtcctga aggaacgttg aagacgacg 29
<210> 172
   <211> 32
   <212> DNA
   <213> Escherichia hermannii
<400> 172
   agagtcctga aggaacgttg aagacgacga cg 32
<210> 173
   <211> 32
   <212> DNA
   <213> Escherichia vulneris
<400> 173
   agtctcctga aggaacgttg aagacgacga cg 32
<210> 174
   <211> 32
   <212> DNA
   <213> Hafnia alvei
<400> 174
   agtctcctaa aggaacgttt aagactaaga cg 32
<210> 175
   <211> 32
   <212> DNA
   <213> Künstliche Sequenz
<220>
   <223> Beschreibung der künstlichen Sequenz: abgeleitet
   von Arten der Gattungen Klebsiella, Kuyvera
<400> 175
   agggtcctga aggaacgttg aagacgacga cg 32
<210> 176
   <211> 32
   <212> DNA
   <213> Morganella morganii
<400> 176
   agggtcctga aggaacgttt gagactaaga cg 32
<210> 177
   <211> 32
   <212> DNA
   <213> Pantoea dispersa
<400> 177
   agggtcctga agggacgctg aagacgacga cg 32
<210> 178
   <211> 32
   <212> DNA
   <213> Künstliche Sequenz
<220>
   <223> Beschreibung der künstlichen Sequenz: abgeleitet
   von Arten der Gattung Pantoea
<400> 178
   aggacactaa aggaacgtta aagacgatga cg 32
<210> 179
   <211> 32
   <212> DNA
   <213> Proteus mirabilis
<400> 179
   agtgacctaa aggaacgttt aagactaaga cg 32
<210> 180
   <211> 32
   <212> DNA
   <213> Proteus rettgeri
<400> 180
   agggtcctaa aggaacgttt aagactaaga cg 32
<210> 181
   <211> 32
   <212> DNA
   <213> Providencia stuartii
<400> 181
   agggtcctaa aggaacgttt aagacgaaga cg 32
<210> 182
   <211> 32
   <212> DNA
   <213> Rahnella aquatilis
<400> 182
   agccacctga agggacgttt aagactaaga cg 32
<210> 183
   <211> 32
   <212> DNA
   <213> Künstliche Sequenz
<220>
   <223> Beschreibung der künstlichen Sequenz: abgeleitet
   von Arten der Gattung Serratia
<400> 183
   aggcccctga aggaacgttt aagactaaga cg 32
<210> 184
   <211> 32
   <212> DNA
   <213> Yersinia enterolytica
<400> 184
   agccccctga aggaacgtta aagactatga cg 32
<210> 185
   <211> 32
   <212> DNA
   <213> Yersinia pseudotuberculosis
<400> 185
   agccccctga gggaacgtta aagactatga cg 32
<210> 186
   <211> 32
   <212> DNA
   <213> Cedecea davisae
<400> 186
   agacccctga agggacgttg aagactacga cg 32
<210> 187
   <211> 24
   <212> DNA
   <213> Künstliche Sequenz
<220>
   <223> Beschreibung der künstlichen Sequenz: abgeleitet
   von Arten der Gattungen Buttiauxella, Escherichia,
   Klebsiella, Kluyvera, Pantoea
<400> 187
   agatgagttc tccctgaccc ttta 24
<210> 188
   <211> 24
   <212> DNA
   <213> Künstliche Sequenz
<220>
   <223> Beschreibung der künstlichen Sequenz: abgeleitet
   von Arten der Gattungen Enterobacter, Pantoea
<400> 188
   agatgagttc tcccttgtcc ttta 24

<210> 189
   <211> 24
   <212> DNA
   <213> Erwinia carotovora
<400> 189
   agatgagtct tccctgggca ccag 24
<210> 190
   <211> 24
   <212> DNA
   <213> Erwinia chrysanthemi
<400> 190
   agatgagtct tccctgggcc cttg 24
<210> 191
   <211> 24
   <212> DNA
   <213> Escherichia hermannii
<400> 191
   agatgagttc tccctgactc cttg 24
<210> 192
   <211> 24
   <212> DNA
   <213> Escherichia vulneris
<400> 192
   agatgagttc tccctgagac ttta 24
<210> 193
   <211> 24
   <212> DNA
   <213> Hafnia alvei
<400> 193
   agatgagtct tccctgagac cttg 24
<210> 194
   <211> 24
   <212> DNA
   <213> Morganella morganii
<400> 194
   agatgagtct tccctgaccc ttta 24
<210> 195
   <211> 24
   <212> DNA
   <213> Proteus mirabilis
<400> 195
   agatgagtct tccctgtcac ttta 24
<210> 196
   <211> 24
   <212> DNA
   <213> Proteus rettgeri
<400> 196
   agatgagtct tccctgaccc ttta 24
<210> 197
   <211> 24
   <212> DNA
   <213> Providencia stuartii
<400> 197
   agatgagtct tccctgactc ttta 24
<210> 198
   <211> 24
   <212> DNA
   <213> Rahnella aquatilis
<400> 198
   agatgagtct tccctgtggc ttta 24
<210> 199
   <211> 24
   <212> DNA
   <213> Yersinia enterolytica
<400> 199
   agatgagtct tccctggggc ttta 24
<210> 200
   <211> 24
   <212> DNA
   <213> Yersinia pseudotuberculosis
<400> 200
   agatgagtct tccctggggc ttaa 24
<210> 201
   <211> 24
   <212> DNA
   <213> Cedecea davisae
<400> 201
   agatgaattc tccctgggtc cttg 24
<210> 202
   <211> 199
   <212> DNA
   <213> Künstliche Sequenz
<220>
   <223> Beschreibung der künstlichen Sequenz: abgeleitet
   von Arten der Gattung Citrobacter
<400> 202
   caacgccgaa gatgttttgg cggaattgag aagattttca gcattgattc agagtccgaa 60
   ggattttgcg ctgagacaag gcggcawccc caccacggaa ggagcataca aaagtatgtg 120
   actgaggttc gcaagcgcag ccaacgcagt atcagcacaa aagacacagg acagagcaca 180
   aagaatttct ggcggccgt                                              199
<210> 203
   <211> 199
   <212> DNA
   <213> Künstliche Sequenz
<220>
   <223> Beschreibung der künstlichen Sequenz: abgeleitet
   von Arten der Gattung Citrobacter

<400> 203
   caacgccgaa gatgttttgg cggattgaga agattttcag tattgattac agattttgcg 60
   aaaacgaaag attttacgct gaggcaaggc ggcaagtgaa gcgacggaag kggcatacaa 120
   aagtatgtga ctgaggttcg caggcgcagc caacgcagca tcagtggaaa agattcgttt 180
   taagagcaca aagaatttc                                              199
<210> 204
   <211> 199
   <212> DNA
   <213> Künstliche Sequenz
<220>
   <223> Beschreibung der künstlichen Sequenz: abgeleitet
   von Arten der Gattung Salmonella
<400> 204
   caacsccsaa gatgttttgg csgatsagag argattttca gcactgattc ckgattttcg 60
   vgaacgaaag attttacgct gaggcaaggc rgcaavcgaa ggaaaggaag gagcatactg 120
   aagtatgtga ctgactttac gagcgcagcc aacgctagca tcsgtgtaaa agattcgttt 180
   ctggcaacag aatttcctg                                              199
<210> 205
   <211> 201
   <212> DNA
   <213> Künstliche Sequenz
<220>
   <223> Beschreibung der künstlichen Sequenz: abgeleitet
   von Arten der Gattung Salmonella
<400> 205
   caacgccgaa gctgttttgg cggatranaa sacgaacaat tttcagcact gattcagagt 60
   tgagtacgca ataatttgcg cagcagcaag gcggcaagcg aaggaaagga aggagcatac 120
   agaagtatgt gactgacttt acgagcgcag ccaacgccgc tgatgcgata aagaattgcg 180
   tacagagcac aaaagaatat t                                           201
<210> 206
   <211> 193
   <212> DNA
   <213> Künstliche Sequenz
<220>
   <223> Beschreibung der künstlichen Sequenz: abgeleitet
   von Arten der Gattung Salmonella
<400> 206
   caacgccgaa gatgttttgg csgttgagaa gacgattttc agcagtgatt ccgrgttgag 60
   trcgcmrtaa tttkcgcmgc wgcarggcgg cargcgaagg arrggaggga gcatccwgaa 120
   gtatktgact gagttttcgr gcgcwggcam cgccgctgat gcgataaaga attgcgtach 180
   gmgcacamag aat                                                    193
<210> 207
   <211> 199
   <212> DNA
   <213> Künstliche Sequenz
<220>
   <223> Beschreibung der künstlichen Sequenz: abgeleitet
   von Arten der Gattung Salmonella
<400> 207
   caacgccgaa gatgttttgg cggattgaga gacgattttc agcactgatt ccggattttc 60
   gggaacgaaa gattttacgc tgaggcaagg cggcaaatgr aggaaaggaa ggagcatact 120
   gaagtatgtg actgactttt cgaatgcagc cgacgcagca tcggtgtaaa agattcgttt 180
   ccggcaacag aattgtcct                                              199
<210> 208
   <211> 189
   <212> DNA
   <213> Künstliche Sequenz
<220>
   <223> Beschreibung der künstlichen Sequenz: abgeleitet
   von Arten der Gattung Salmonella
<400> 208
   caacgccgaa gatgttttgg cggatgagag acgattttca gcactgattc agagttgagt 60
   acgcaataat ttgcgcagca gcaaggcggc aagcgaagga aaggaaggag catacagaag 120
   tatgtgactg agtttacgag cgcaggcaac gccgctgatg cgataaagaa ttgcgtactg 180
   agcataaaa                                                         189
<210> 209
   <211> 196
   <212> DNA
   <213> Künstliche Sequenz
<220>
   <223> Beschreibung der künstlichen Sequenz: abgeleitet
   von Arten der Gattung Salmonella
<400> 209
   caacgccgaa gatgttttgg cggattgaga agacaacaat tttcagcyca gattcagagt 60
   ccgaaggatt ttacgctgag acaaggcggc aaacgcagcs mcsgaaggas cmycacagaa 120
   gtatgtgact gacgctcgca agagcagcca acgccgtatc agtgtaaaag acacaggacg 180
   grgcacaaag aaattt                                                 196
<210> 210
   <211> 77
   <212> DNA
   <213> Künstliche Sequenz
<220>
   <223> Beschreibung der künstlichen Sequenz: abgeleitet
   von Arten der Gattung Salmonella
<400> 210
   gagagacgat tttcagcact gattccggat tttcgggaac gaaagataaa agattcgttt 60
   ccggcaacag aatttcc                                                 77
<210> 211
   <211> 24
   <212> DNA
   <213> Künstliche Sequenz
<220>
   <223> Beschreibung der künstlichen Sequenz: abgeleitet
   von Arten und Gattungen der Eubakterien
<400> 211
   ggtacgcgag ctgggtttag aacg 24
<210> 212
   <211> 19
   <212> DNA
   <213> Künstliche Sequenz
<220>
   <223> Beschreibung der künstlichen Sequenz: abgeleitet
   von Arten und Gattungen der Eubakterien
<400> 212
   gbgagagtag gdmayygcc 19
<210> 213
   <211> 54
   <212> DNA
   <213> Pseudomonas stutzeri
<400> 213
   ccggagtgga cgaacctctg gtgttccggt tgtcacgcca gtggcattgc cggg 54
<210> 214
   <211> 53
   <212> DNA
   <213> Thiobacilluc ferrooxidans
<400> 214
   ccggagtgga cgtactctgg tgttccggtt gttctgccaa gggcattgcc ggg 53
<210> 215
   <211> 54
   <212> DNA
   <213> Agrobacterium vitis
<400> 215
   ccgggatgga catatctctg gtggacctgt tgtcgtgcca acggcatagc aggg 54
<210> 216
   <211> 54
   <212> DNA
   <213> Adalia bipunctata
<400> 216
   ccgaggtgga cgtacctctg gtggaccagt tgtcatgcca atggcacagc tggg 54
<210> 217
   <211> 54
   <212> DNA
   <213> Amycolatopsis orientalis
<400> 217
   ccgggacgga cgaacctctg gtgtgccagt tgtcctgcca agggcatggc tggt 54
<210> 218
   <211> 54
   <212> DNA
   <213> Brucella ovis
<400> 218
   ccgggatgga cgtatctntg gtggacctgt tgtggcgcca gccgcatagc aggg 54
<210> 219
   <211> 54
   <212> DNA
   <213> Bradyrhizobium japonicum
<400> 219
   ccggggtgaa cgtacctctg gtggagctgt tgtcgcgcca gcggcagtgc agca 54
<210> 220
   <211> 54
   <212> DNA
   <213> Pseudomonas paucimobilis
<400> 220
   ccgggatgga cgcaccgctg gtgtaccagt tgttctgcca agggcatcgc tggg 54
<210> 221
   <211> 54
   <212> DNA
   <213> Rhodobacter sphaeroides
<400> 221
   ccgggatgga cgcaccgctg gtgtaccagt tgttctgcca agggcatcgc tggg 54
<210> 222
   <211> 57
   <212> DNA
   <213> Rickettsia prowazekii
<400> 222
   ccgaggtgga cgtacccctg gtggaccagt tgtcgtgcca acggcaagct gggtagc 57
<210> 223
   <211> 54
   <212> DNA
   <213> Sphingomonas paucimobilis
<400> 223
   ccggagtgga cgaacctctg gtgtaccggt tgtcacgcca gtggcattgc cggg 54
<210> 224
   <211> 54
   <212> DNA
   <213> Zymomonas mobilis
<400> 224
   ccggggtgaa catgcctctg gtggacctgt cgtggcgcca gccgcgcagc aggg 54
<210> 225
   <211> 54
   <212> DNA
   <213> Künstliche Sequenz
<220>
   <223> Beschreibung der künstlichen Sequenz: abgeleitet
   von Arten der Gattung Alcaligenes
<400> 225
   ccagagtgga cgaacctctg gtgtaccggt tgtgacgcca gtcgcatcgc cggg 54
<210> 226
   <211> 53
   <212> DNA
   <213> Pseudomonas cepacia
<400> 226
   ccgggacgac gaacctctgg tgtgtcagtt gtactgccaa gtgcaccgct gat 53
<210> 227
   <211> 54
   <212> DNA
   <213> Ralstonia pickettii
<400> 227
   ccggagtgga cgaacctctg gtgttccggt tgtcacgcca gtggcattgc cggg 54
<210> 228
   <211> 54
   <212> DNA
   <213> Campylobacter jejuni
<400> 228
   ccgggttgaa caaaccactg gtgtagctgt tgttctgcca agagcatcgc agcg 54
<210> 229
   <211> 53
   <212> DNA
   <213> Helicobacter pylori
<400> 229
   ccgggatgga cgtgtcactg gtgcaccagt tgtctgccaa gagcatcgct ggg 53
<210> 230
   <211> 53
   <212> DNA
   <213> Actinoplanes utahensis
<400> 230
   ccgggacgga cgaacctctg gtgtgccagt tgttctgcca agagcacggc tgg 53
<210> 231
   <211> 54
   <212> DNA
   <213> Bacillus halodurans
<400> 231
   ccgggatgga cacaccgctg gtgtaccagt tgttccgcca ggagcatcgc tggg 54
<210> 232
   <211> 54
   <212> DNA
   <213> Bacillus subtilis
<400> 232
   ccgggatgga cgcaccgctg gtgtaccagt tgttctgcca agggcatcgc tggg 54
<210> 233
   <211> 54
   <212> DNA
   <213> Clostridium tyrobutyricum
<400> 233
   ccgggatgga ctgacctctg gtgtaccagt tgttccgcca ggagcatggc tggg 54
<210> 234
   <211> 54
   <212> DNA
   <213> Künstliche Sequenz
<220>
   <223> Beschreibung der künstlichen Sequenz: abgeleitet
   von Arten der Gattung Frankia
<400> 234
   ccgggacgga cgaacctctg gtgtgccagt tgttctgcca agggcatggc tggt 54
<210> 235
   <211> 54
   <212> DNA
   <213> Microbispora bispora
<400> 235
   ccggaacgga cgaacctctg gtgtgccagt tgtgccgcca ggtgcacggc tggt 54
<210> 236
   <211> 54
   <212> DNA
   <213> Mycobacterium leprae
<400> 236
   ccgggacgga cgaacctctg gtataccagt tgtctcacca ggggcaccgc tgga 54
<210> 237
   <211> 54
   <212> DNA
   <213> Mycobacterium smegmatis
<400> 237
   ccgggacgga cgaacctctg gtataccagt tgtcccacca ggggcacggc tgga 54
<210> 238
   <211> 54
   <212> DNA
   <213> Mycobacterium tuberculosis
<400> 238
   ccgggacgga cgaacctctg gtgcaccagt tgtcccgcca ggggcaccgc tgga 54
<210> 239
   <211> 54
   <212> DNA
   <213> Mycobacterium gallisepticum
<400> 239
   ccggagtgaa gacacctctt gtgctccagt tgtagcgcca actgcaccgc tggg 54
<210> 240
   <211> 58
   <212> DNA
   <213> Propionibacterium freudenreichii
<400> 240
   ccgggacgga ccaacctctg gtgtgccagt tgttccacca ggagcatggc tggttggc 58
<210> 241
   <211> 54
   <212> DNA
   <213> Rhodococcus erythropolis
<400> 241
   ccgggacgga cgaacctctg gtgtgccagt tgttccgcca ggagcaccgc tggt 54
<210> 242
   <211> 57
   <212> DNA
   <213> Rhodococcus fascians
<400> 242
   ccgggacgac gaacctctgg tgtgccagtt gttccaccag gagcaccgct ggttggc 57
<210> 243
   <211> 58
   <212> DNA
   <213> Staphylococcus aureus
<400> 243
   ccgggatgga catacctctg gtgtaccagt tgtcgtgcca acggcatagc tgggtagc 58
<210> 244
   <211> 54
   <212> DNA
   <213> Streptococcus faecalis
<400> 244
   ccgggatgga cttnccgctg gtgtaccagt tgttctgcca agggcattgc tggg 54
<210> 245
   <211> 54
   <212> DNA
   <213> Streptomyces ambifaciens
<400> 245
   ccgggatgga cttnccgctg gtgtaccagt tgttctgcca agggcattgc tggg 54
<210> 246
   <211> 54
   <212> DNA
   <213> Flavobacterium resinovorum
<400> 246
   ccggagtgga cgtaccgctg gtgtacctgt tgtctcgcca gaggcatcgc aggg 54
<210> 247
   <211> 54
   <212> DNA
   <213> Sphingobacterium multivorans
<400> 247
   ccgggttgga cagacctctg gtgaacctgt catnccgcca ggtgtacggc aggg 54
<210> 248
   <211> 54
   <212> DNA
   <213> Künstliche Sequenz
<220>
   <223> Beschreibung der künstlichen Sequenz: abgeleitet
   von Arten der Gattung Synechococcus
<400> 248
   ccggaggaac gcaccgctgg tgtaccagtt atcgtgccaa cggtaaacgc tggg 54
<210> 249
   <211> 55
   <212> DNA
   <213> Künstliche Sequenz
<220>
   <223> Beschreibung der künstlichen Sequenz: abgeleitet
   von Arten der Gattung Synechocystis
<400> 249
   ccgggaagta cgcacctctg gtgtacctgt tatcgtgcca acggtaaacg caggg 55
<210> 250
   <211> 59
   <212> DNA
   <213> Borrelia burgdorferi
<400> 250
   ccgagatgga cgaacctcta gtgtaccagt tatcctgcca agggtaagtg ctgggtagc 59
<210> 251
   <211> 58
   <212> DNA
   <213> Chlamydia trachomatis
<400> 251
   ccggaatgga cgaaccaatg gtgtgtcggt tgttttgcca agggcatagc cgagtagc 58
<210> 252
   <211> 42
   <212> DNA
   <213> Pseudomonas stutzeri
<400> 252
   gagataaccg ctgaaagcat ctaagcggga aacttgcctc aa 42
<210> 253
   <211> 41
   <212> DNA
   <213> Thiobacilluc ferrooxidans
<400> 253
   gggataaccg ctgaaagcat ctaagcggaa gccatcctaa g 41
<210> 254
   <211> 41
   <212> DNA
   <213> Agrobacterium vitis
<400> 254
   tggataaccg ctgaaggcat ctaagcggga aaccaacctg a 41
<210> 255
   <211> 41
   <212> DNA
   <213> Adalia bipunctata
<400> 255
   gggataaccg ctgaatgcat ctaagcagga aactcacctc a 41
<210> 256
   <211> 41
   <212> DNA
   <213> Amycolatopsis orientalis
<400> 256
   aggataaccg ctgaaagcat ctaagcggga agcctgcttc g 41
<210> 257
   <211> 42
   <212> DNA
   <213> Brucella ovis
<400> 257
   gggataaccg ctgaaggcat ntaagcggga aacccacctg aa 42
<210> 258
   <211> 41
<212> DNA
   <213> Bradyrhizobium japonicum
<400> 258
   gggataaccg ctgaaagcat ctaagcggga aacccacctc a 41
<210> 259
   <211> 41
   <212> DNA
   <213> Pseudomonas paucimobilis
<400> 259
   gggataagtg ctgaaagcat ctaagcatga agcccccctc a 41
<210> 260
   <211> 41
   <212> DNA
   <213> Rhodobacter sphaeroides
<400> 260
   aggataaccg ctgaaggcat ctaagcggga agcccccttc a 41
<210> 261
   <211> 40
   <212> DNA
   <213> Rickettsia prowazekii
<400> 261
   gggataactg ctgaatgcat ctaagcagga aacccacctc 40
<210> 262
   <211> 41
   <212> DNA
   <213> Sphingomonas paucimobilis
<400> 262
   gagataaccg ctgaaagcat ctaagcggga aacttgcctt g 41
<210> 263
   <211> 41
   <212> DNA
   <213> Zymomonas mobilis
<400> 263
   gggataaccg ctgaaagcat ctaagcggga agcctccctc a 41
<210> 264
   <211> 41
   <212> DNA
   <213> Künstliche Sequenz
<220>
   <223> Beschreibung der künstlichen Sequenz: abgeleitet
   von Arten der Gattung Alcaligenes
<400> 264
   gggataaccg ctgaaagcat ctaagcggga agcctacctc a 41
   <210> 265
   <211> 41
   <212> DNA
   <213> Pseudomonas cepacia
<400> 265
   gggataaccg ctgaaagcat ctaagcggga agctcgcttc a 41
<210> 266
   <211> 41
   <212> DNA
   <213> Ralstonia pickettii
<400> 266
   gagataaccg ctgaaagcat ctaagcggaa aacttgcctc a 41
<210> 267
   <211> 41
   <212> DNA
   <213> Campylobacter jejuni
<400> 267
   aggataaacg ctgaaagcat ctaagcgtga agccaactct a 41
<210> 268
   <211> 42
   <212> DNA
   <213> Helicobacter pylori
<400> 268
   tgtgataact gctgaaagca tctaagcagg aaccaactcc aa 42
<210> 269
   <211> 41
   <212> DNA
   <213> Actinoplanes utahensis
<400> 269
   gggataaccg ctgaaagcat ctaagcggga agctcgcttc g 41
<210> 270
   <211> 41
   <212> DNA
   <213> Bacillus halodurans
<400> 270
   gggataagtg ctgaaagcat ctaagcatga agcccccctc a 41
<210> 271
   <211> 40
   <212> DNA
   <213> Clostridium tyrobutyricum
<400> 271
   gggataaacg ctgaaagcat ctaagcgtga agcccacctc 40
<210> 272
   <211> 41
<212> DNA
   <213> Künstliche Sequenz
<220>
   <223> Beschreibung der künstlichen Sequenz: abgeleitet
   von Arten der Gattung Frankia
<400> 272
   gggataaccg ctgaaagcat ctaagcggga agcctgcttc g 41
<210> 273
   <211> 41
   <212> DNA
   <213> Microbispora bispora
<400> 273
   gggataaccg ctgaaagcat ctaagcggga agcccgcccc g 41
<210> 274
   <211> 41
   <212> DNA
   <213> Mycobacterium leprae
<400> 274
   aagataaccg ctgaaagcat ctaagcggga aaccttctcc a 41
<210> 275
   <211> 41
   <212> DNA
   <213> Mycobacterium smegmatis
<400> 275
   aggataaccg ctgaaagcat ctaagcggga aacctcttcc a 41
<210> 276
   <211> 41
   <212> DNA
   <213> Mycobacterium tuberculosis
<400> 276
   aggataaccg ctgaaagcat ctaagcggga aaccttctcc a 41
<210> 277
   <211> 41
   <212> DNA
   <213> Mycobacterium gallisepticum
<400> 277
   cggataaacg ctgaaagcat ctaagtgtga aaccgacttt a 41
<210> 278
   <211> 43
   <212> DNA
   <213> Propionibacterium freudenreichü
<400> 278
   agtgataacc gctgaaagca tctaagtggg aagcacgctt caa 43
<210> 279
   <211> 41
   <212> DNA
   <213> Rhodococcus erythropolis
<400> 279
   gggataaccg ctgaaagcat ctaagcggga agcctgttcc a 41
<210> 280
   <211> 41
   <212> DNA
   <213> Staphylococcus aureus
<400> 280
   gggataagtg ctgaaagcat ctaagcatga agcccccctc a 41
<210> 281
   <211> 41
   <212> DNA
   <213> Streptococcus faecalis
<400> 281
   gggataaacg ctgaaagcat ctaagtgtga agcccncctc a 41
<210> 282
   <211> 41
   <212> DNA
   <213> Streptomyces ambifaciens
<400> 282
   gggataaccg ctgaaagcat ctaagcggga agcctgcttc g 41
<210> 283
   <211> 41
   <212> DNA
   <213> Flavobacterium resinovorum
<400> 283
   gagataaccg ctgaaagcat ctaagcggga aactcgcctg a 41
<210> 284
   <211> 41
   <212> DNA
   <213> Sphingobacterium multivorans
<400> 284
   tagataagcg ctgaaagcat ctaagtgcga aactagccac g 41
<210> 285
   <211> 43
   <212> DNA
   <213> Künstliche Sequenz
<220>
   <223> Beschreibung der künstlichen Sequenz: abgeleitet
   von Arten der Gattung Synechococcus
<400> 285
   gtggataacc gctgaaagca tctaagtggg aagcccacct caa 43
<210> 286
   <211> 43
   <212> DNA
   <213> Künstliche Sequenz
<220>
   <223> Beschreibung der künstlichen Sequenz: abgeleitet
   von Arten der Gattung Synechocystis
<400> 286
   gtggataacc gctgaaagca tctaagtggg aagcccacct caa 43
<210> 287
   <211> 41
   <212> DNA
   <213> Borrelia burgdorferi
<400> 287
   aggataaccg ctgaaagcat ctaagtggga agccttcctc a 41
<210> 288
   <211> 41
   <212> DNA
   <213> Chlamydia trachomatis
<400> 288
   aggataagca ttgaaagcat ctaaatgcca agcctccctc a 41
<210> 289
   <211> 24
   <212> DNA
   <213> Pseudomonas stutzeri
<400> 289
   agatgagatc tcactggagc cttg 24
<210> 290
   <211> 19
   <212> DNA
   <213> Thiobacillus ferrooxidans
<400> 290
   atgagatctc ccgggcata 19
<210> 291
   <211> 18
   <212> DNA
   <213> Agrobacterium vitis
<400> 291
   aaacgagtat tccctatc 18
<210> 292
   <211> 18
   <212> DNA
   <213> Adalia bipunctata
<400> 292
   aaactagact tccccatc 18
<210> 293
   <211> 23
   <212> DNA
   <213> Amycolatopsis orientalis
<400> 293
   agatgagggc tcccacctcc ttg 23
<210> 294
   <211> 18
   <212> DNA
   <213> Brucella ovis
<400> 294
   aaacgagtat tccctatc 18
<210> 295
   <211> 17
   <212> DNA
   <213> Bradyrhizobium japonicum
<400> 295
   aaacgagcat tcccttg 17
<210> 296
   <211> 22
   <212> DNA
   <213> Pseudomonas paucimobilis
<400> 296
   agatgagatt tcccattccg ca 22
<210> 297
   <211> 22
   <212> DNA
   <213> Rhodobacter sphaeroides
<400> 297
   agatgagatt tcccattccg ca 22
<210> 298
   <211> 18
   <212> DNA
   <213> Rickettsia prowazekii
<400> 298
   aaactagact tccccatt 18
<210> 299
   <211> 23
   <212> DNA
   <213> Sphingomonas paucimobilis
<400> 299
   agatgagatt tcccggagcc ttg 23
<210> 300
   <211> 14
   <212> DNA
   <213> Zymomonas mobilis
<400> 300
   agataagata tctc 14
<210> 301
   <211> 24
   <212> DNA
   <213> Künstliche Sequenz
<220>
   <223> Beschreibung der künstlichen Sequenz: abgeleitet
   von Arten der Gattung Alcaligenes
<400> 301
   agataagatt tccctaggac ttta 24
<210> 302
   <211> 23
   <212> DNA
   <213> Pseudomonas cepacia
<400> 302
   agatgagatt tccatacacc ttg 23
<210> 303
   <211> 24
   <212> DNA
   <213> Ralstonia pickettii
<400> 303
   agatgagatc tcactggaac cttg 24
<210> 304
   <211> 24
   <212> DNA
   <213> Campylobacter jejuni
<400> 304
   agatgaatct tctctaagct ctct 24
<210> 305
   <211> 13
   <212> DNA
   <213> Helicobacter pylori
<400> 305
   gataaacttt ccc 13
<210> 306
   <211> 23
   <212> DNA
   <213> Actinoplanes utahensis
<400> 306
   agatgaggta tcccaccacc ttg 23
<210> 307
   <211> 22
   <212> DNA
   <213> Bacillus halodurans
<400> 307
   agatgagatt tcccatggag ta 22
<210> 308
   <211> 22
   <212> DNA
   <213> Clostridium tyrobutyricum
<400> 308
   agattagatt tcccacagcg ta 22
<210> 309
   <211> 23
   <212> DNA
   <213> Künstliche Sequenz
<220>
   <223> Beschreibung der künstlichen Sequenz: abgeleitet
   von Arten der Gattung Frankia
<400> 309
   agatgaggtc tcccacaggg tag 23
<210> 310
   <211> 23
   <212> DNA
   <213> Microbispora bispora
<400> 310
   agatgaggtc tccctccggg tta 23
<210> 311
   <211> 22
   <212> DNA
   <213> Mycobacterium leprae
<400> 311
   agatcaggtt tcttacccac tt 22
<210> 312
   <211> 22
   <212> DNA
   <213> Mycobacterium smegmatis
<400> 312
   agaccaggct tctcaccctc ta 22
<210> 313
   <211> 22
   <212> DNA
<213> Mycobacterium tuberculosis
<400> 313
   agatcaggtt tctcacccac tt 22
<210> 314
   <211> 30
   <212> DNA
   <213> Mycobacterium gallisepticum
<400> 314
   agaataatct tcccttccag caatggagta 30
<210> 315
   <211> 21
   <212> DNA
   <213> Propionibacterium freudenreichii
<400> 315
   gatgagggtt cctgcacagt t 21
<210> 316
   <211> 22
   <212> DNA
   <213> Rhodococcus erythropolis
<400> 316
   agatgaggtt tctcaccccc tc 22
<210> 317
   <211> 20
   <212> DNA
   <213> Staphylococcus aureus
<400> 317
   agatgagatt tcccaacttc 20
<210> 318
   <211> 22
   <212> DNA
   <213> Streptococcus faecalis
<400> 318
   agatgagatt tcccatttct tt 22
<210> 319
   <211> 23
   <212> DNA
   <213> Streptomyces ambifaciens
<400> 319
   agatgaggac tcccaccccc ttg 23
<210> 320
   <211> 24
   <212> DNA
   <213> Flavobacterium resinovorum
<400> 320
   agatgaggat tccctggcgg cttg 24
<210> 321 .
   <211> 17
   <212> DNA
   <213> Sphingobacterium multivorans
<400> 321
   agatgagact tccttat 17
<210> 322
   <211> 20
   <212> DNA
   <213> Künstliche Sequenz
<220>
   <223> Beschreibung der künstlichen Sequenz: abgeleitet
   von Arten der Gattung Synechococcus
<400> 322
   gatgagtact ctcatggcat 20
<210> 323
   <211> 21
   <212> DNA
   <213> Künstliche Sequenz
<220>
   <223> Beschreibung der künstlichen Sequenz: abgeleitet
   von Arten der Gattung Synechocystis
<400> 323
   gatgagtact ctcatggtgt t 21
<210> 324
   <211> 16
   <212> DNA
   <213> Borrelia burgdorferi
<400> 324
   agatgagata tccttt 16
<210> 325
   <211> 14
   <212> DNA
   <213> Chlamydia trachomatis
<400> 325
   agataaggta tccc 14
<210> 326
   <211> 32
   <212> DNA
   <213> Pseudomonas stutzeri
<400> 326
   agctccctga agggccgtcg aagactacga cg 32
<210> 327
   <211> 32
   <212> DNA
   <213> Thiobacillus ferrooxidans
<400> 327
   agccccctga agggacgtgg aagactacca cg 32
<210> 328
   <211> 22
   <212> DNA
   <213> Agrobacterium vitis
<400> 328
   agagccgtgg aagacgacca cg 22
<210> 329
   <211> 22
   <212> DNA
   <213> Adalia bipunctata
<400> 329
   agagccgtgg aagaccacca cg 22
<210> 330
   <211> 30
   <212> DNA
   <213> Amycolatopsis orientalis
<400> 330
   aggggttaag gctcccagta gacgactggg 30
<210> 331
   <211> 22
   <212> DNA
   <213> Künstliche Sequenz
<220>
   <223> Beschreibung der künstlichen Sequenz: abgeleitet
   von Arten der Gattungen Brucella, Bradyrhizobium
<400> 331
   agagccgtgg aagaccacca cg 22
<210> 332
   <211> 30
   <212> DNA
   <213> Pseudomonas paucimobilis
<400> 332
   aggaagtaag atccctgaaa gatgatcagg 30
<210> 333
   <211> 22
   <212> DNA
   <213> Künstliche Sequenz
<220>
   <223> Beschreibung der künstlichen Sequenz: abgeleitet
   von Arten der Gattungen Rhodobacter, Rickettsia
<400> 333
   agggccgtgg aagaccacca cg 22
<210> 334
   <211> 26
   <212> DNA
   <213> Sphingomonas paucimobilis
<400> 334
   agctccttga agggtcgttc gagacc 26
<210> 335
   <211> 22
   <212> DNA
   <213> Zymomonas mobilis
<400> 335
   agagccgtcg aagactacga cg 22
<210> 336
   <211> 26
   <212> DNA
   <213> Künstliche Sequenz
<220>
   <223> Beschreibung der künstlichen Sequenz: abgeleitet
   von Arten der Gattung Alcaligenes
<400> 336
   tgtcctctaa agagccgttc gagact 26
<210> 337
   <211> 25
   <212> DNA
   <213> Pseudomonas cepacia
<400> 337
   tgtgtgagag gcccccagcc agacc 25
<210> 338
   <211> 26
   <212> DNA
   <213> Ralstonia pickettii
<400> 338
   agttccctga agggccgtcg aagact 26
<210> 339
   <211> 14
   <212> DNA
   <213> Campylobacter jejuni
<400> 339
   agaagactac tagt 14
<210> 340
   <211> 25
   <212> DNA
   <213> Helicobacter pylori
<400> 340
   tgaagctcgc acaaagacta tgtgc 25
<210> 341
   <211> 28
   <212> DNA
   <213> Actinoplanes utahensis
<400> 341
   agtgggtaag gctcccagct agactact 28
<210> 342
   <211> 31
   <212> DNA
   <213> Bacillus halodurans
<400> 342
   aatccagtaa gaccccttag agatgatgag g 31
<210> 343
   <211> 30
   <212> DNA
   <213> Bacillus subtilis
<400> 343
   aggaagtaag atccctgaaa gatgatcagg 30
<210> 344
   <211> 32
   <212> DNA
   <213> Clostridium tyrobutyricum
<400> 344
   agctggtaag gccccttgaa gaacacaagg tg 32
<210> 345
   <211> 30
   <212> DNA
   <213> Künstliche Sequenz
<220>
   <223> Beschreibung der künstlichen Sequenz: abgeleitet
   von Arten der Gattung Frankia
<400> 345
   cctggtaagg cccccgacta gatgatcggg 30
<210> 346
   <211> 30
   <212> DNA
   <213> Microbispora bispora
<400> 346
   accgggtaag gctcccagta gatgactggg 30
<210> 347
   <211> 31
   <212> DNA
   <213> Mycobacterium leprae
<400> 347
   ggtgggataa ggccccccgc agaacacggg a 31
<210> 348
   <211> 31
   <212> DNA
   <213> Mycobacterium smegmatis
<400> 348
   ggagggataa ggccccccgc agaccacggg a 31
<210> 349
   <211> 31
   <212> DNA
   <213> Mycobacterium tuberculosis
<400> 349
   ggtgggataa ggccccccgc agaacacggg t 31
<210> 350
   <211> 30
   <212> DNA
   <213> Propionibacterium freudenreichii
<400> 350
   aatgtggtaa ggcccccggt agaccaccgg 30
<210> 351
   <211> 31
   <212> DNA
   <213> Rhodococcus erythropolis
<400> 351
   gagggggtaa ggcccccggc agaccaccgg g 31
<210> 352
   <211> 29
   <212> DNA
   <213> Staphylococcus aureus
<400> 352
   ggttataaga tccctcaaag atgatgagg 29
<210> 353
   <211> 31
   <212> DNA
   <213> Streptococcus faecalis
<400> 353
   aagaaagtaa gacccctnan agatgatcag g 31
<210> 354
   <211> 30
   <212> DNA
   <213> Streptomyces ambifaciens
<400> 354
   aggggttaag gctcccagta gacgactggg 30
<210> 355
   <211> 32
   <212> DNA
   <213> Flavobacterium resinovorum
<400> 355
   accgccttga agggtcgttc gagaccagga cg 32
<210> 356
   <211> 22
   <212> DNA
   <213> Sphingobacterium multivorans
<400> 356
   agggtcgtag aagatgacta cg 22
<210> 357
   <211> 30
   <212> DNA
   <213> Künstliche Sequenz
<220>
   <223> Beschreibung der künstlichen Sequenz: abgeleitet
   von Arten der Gattung Synechococcus
<400> 357
   aagccagtaa ggtcacgggt agaacacccg 30
<210> 358
   <211> 30
   <212> DNA
   <213> Künstliche Sequenz
<220>
   <223> Beschreibung der künstlichen Sequenz: abgeleitet
   von Arten der Gattung Synechocystis
<400> 358
   aagccagtaa ggtcacggga agactacccg 30
<210> 359
   <211> 23
   <212> DNA
   <213> Borrelia burgdorferi
<400> 359
   aagggtcctg gaagaatacc agg 23
<210> 360
   <211> 26
   <212> DNA
   <213> Chlamydia trachomatis
<400> 360
   aatgagactc catgtagact acgtgg 26
<210> 361
   <211> 40
   <212> DNA
   <213> Pseudomonas stutzeri
<400> 361
   agtaatgcat taagctaacc agtactaatt gcccgtacgg 40
<210> 362
   <211> 40
   <212> DNA
   <213> Thiobacillus ferrooxidans
<400> 362
   agcaatgcgt gcagctaagg agtactaatc ggccgtgcgg 40
<210> 363
   <211> 40
   <212> DNA
   <213> Agrobacterium vitis
<400> 363
   ggtaacctgc gaagcttacc gttactaata gctcgattgg 40
<210> 364
   <211> 40
   <212> DNA
   <213> Adalia bipunctata
<400> 364
   agtaatgcgt gtagctaacc gatactaata gctcgattga 40
<210> 365
   <211> 40
   <212> DNA
   <213> Brucella ovis
<400> 365
   ggcaacgcat gcagcttacc ggtactaata gctcgatcga 40
<210> 366
   <211> 40
   <212> DNA
   <213> Bradyrhizobium japonicum
<400> 366
   agtaatgcat gcagcttacc ggtactaatc gttcgattgg 40
<210> 367
   <211> 40
   <212> DNA
   <213> Pseudomonas paucimobilis
<400> 367
   ggcgacacat ggagctgaca gatactaatc gatcgaggac 40
<210> 368
   <211> 40
   <212> DNA
   <213> Rhodobacter sphaeroides
<400> 368
   agcaatgcgt tcagctgact ggtactaatt gcccgatagg 40
<210> 369
   <211> 40
   <212> DNA
   <213> Rickettsia prowazekii
<400> 369
   agtaatgtgt gtagctaacc gatactaata gctcgattga 40
<210> 370
   <211> 40
   <212> DNA
   <213> Sphingomonas paucimobilis
<400> 370
   agtaatgcat taagctaacc agtactaatt gcccgtncgg 40
<210> 371
   <211> 40
   <212> DNA
   <213> Zymomonas mobilis
<400> 371
   ggtaacacat gtagctaact ggtcctaatt gctctattca 40
<210> 372
   <211> 40
   <212> DNA
   <213> Künstliche Sequenz
<220>
   <223> Beschreibung der künstlichen Sequenz: abgeleitet
   von Arten der Gattung Alcaligenes
<400> 372
   agtgatatgt gaagctgacc aatactaatt gctcgtgagg 40
<210> 373
   <211> 40
   <212> DNA
   <213> Ralstonia pickettii
<400> 373
   tgtgaggcgt tgagctaacc aatactaatt gcccgtgagg 40
<210> 374
   <211> 40
   <212> DNA
   <213> Campylobacter jejuni
<400> 374
   tgaaagtcct ttagctgacc agtactaata gagcgtttgg 40
<210> 375
   <211> 40
   <212> DNA
   <213> Helicobacter pylori
<400> 375
   agtaatgcgt ttagctgact actactaata gagcgtttgg 40
<210> 376
   <211> 40
   <212> DNA
   <213> Bacillus halodurans
<400> 376
   ggcgacacgt gaagctgaca gatactaatc ggtcgaggac 40
<210> 377
   <211> 40
   <212> DNA
   <213> Bacillus subtilis
<400> 377
   ggcgacacat ggagctgaca gatactaatc gatcgaggac 40
<210> 378
   <211> 40
   <212> DNA
   <213> Clostridium tyrobutyricum
<400> 378
   ggcaacatgt tcagctgact gatactaata ggccgagggc 40
<210> 379
   <211> 41
   <212> DNA
   <213> Künstliche Sequenz
<220>
   <223> Beschreibung der künstlichen Sequenz: abgeleitet
   von Arten der Gattung Frankia
<400> 379
   cggtgacgca tggagctgac cggtactaat aggccgaggg c 41
<210> 380
   <211> 42
   <212> DNA
   <213> Microbispora bispora
<400> 380
   cggtaacgtg tggagccgac cggtactaat aagccgagag gc 42
<210> 381
   <211> 41
   <212> DNA
   <213> Mycobacterium leprae
<400> 381
   cagtaatgag tgtagggaac tggcactaac tggccgaaag c 41
<210> 382
   <211> 41
   <212> DNA
   <213> Mycobacterium smegmatis
<400> 382
   tagtaatagg tgcagggaac tggcactaac cggccgaaaa c 41
<210> 383
   <211> 41
   <212> DNA
   <213> Mycobacterium tuberculosis
<400> 383
   cagtaatggg tgtagggaac tggtgctaac cggccgaaaa c 41
<210> 384
   <211> 86
   <212> DNA
   <213> Mycobacterium gallisepticum
<400> 384
   agaatcgttg tagactacga cgttgatagg ctaaaggtgt aagtgccgcg aggtatttag 60
   ctgattagta ctaataattc gaggac 86
<210> 385
   <211> 27
   <212> DNA
   <213> Propionibacterium freudenreichii
<400> 385
   gctgaccgat actaagtggc cgagggc 27
<210> 386
   <211> 41
   <212> DNA
   <213> Rhodococcus erythropolis
<400> 386
   cagtaatgca tgcaggtgac tggtactaat aggccgagga c 41
<210> 387
   <211> 41
   <212> DNA
   <213> Rhodococcus fascians
<400> 387
   cagcaatgta tgcaggtgac tggtactaat aggccgagga c 41
<210> 388
   <211> 27
   <212> DNA
   <213> Staphylococcus aureus
<400> 388
   gctgacgaat actaatcgat cgagggc 27
<210> 389
   <211> 27
   <212> DNA
   <213> Streptococcus faecalis
<400> 389
   gcggaccaat actaatcggt cgaggac 27
<210> 390
   <211> 51
   <212> DNA
   <213> Streptomyces ambifaciens
<400> 390
   ccgcaaggtg tggaggtgac cggtactaat aggccgaggg cttgtcctca t 51
<210> 391
   <211> 51
   <212> DNA
   <213> Streptomyces galbus
<400> 391
   cggtaacgtg tggaggtgac cggtactaat aggccgaggg cttgtcctca g 51
<210> 392
   <211> 51
   <212> DNA
   <213> Streptomyces griseus
<400> 392
   cggtaacggg tggagctgac tggtactaat aggccgaggg cttgtcctca g 51
<210> 393
   <211> 51
   <212> DNA
   <213> Streptomyces lividans
<400> 393
   ccgtgaggtg tggaggtgac cggtactaat aggccgaggg cttgtcctca g 51
<210> 394
   <211> 51
   <212> DNA
   <213> Streptomyces mashuensis
<400> 394
   cggtaacggt tggagctgac tggtactaat aggccgaggg cttgtccata g 51
<210> 395
   <211> 28
   <212> DNA
   <213> Flavobacterium resinovorum
<400> 395
   gctaaccagt actaattgcc cgtaaggc 28
<210> 396
   <211> 28
   <212> DNA
   <213> Sphingobacterium multivorans
<400> 396
   gccaagtggt actaatagcc cgaagctt 28
<210> 397
   <211> 27
   <212> DNA
   <213> Künstliche Sequenz
<220>
   <223> Beschreibung der künstlichen Sequenz: abgeleitet
   von Arten der Gattung Synechococcus
<400> 397
   gctgaggcgt actaatagac cgagggc 27
<210> 398
   <211> 27
   <212> DNA
   <213> Künstliche Sequenz
<220>
   <223> Beschreibung der künstlichen Sequenz: abgeleitet
   von Arten der Gattung Synechocystis
<400> 398
   gtcgaggagt actaatagac cgagggc 27
<210> 399
   <211> 27
   <212> DNA
   <213> Borrelia burgdorferi
<400> 399
   gctgactaat actaattacc cgtatct 27
<210> 400
   <211> 28
   <212> DNA
   <213> Chlamyia trachomatis
<400> 400
   gctaaccaat actaataagt ccaaagac 28
<210> 401
   <211> 36
   <212> DNA
   <213> Salmonella typhi
<400> 401
   cttaacctta caacgccgaa gatgttttgg cggatg 36
<210> 402
   <211> 35
   <212> DNA
   <213> Buchnera aphidocola
<400> 402
   cttaacctta caacaccaga ggtgtttttt ataaa 35
<210> 403
   <211> 35
   <212> DNA
   <213> Pseudomonas stutzeri
<400> 403
   cttgaccata taacacccaa acaatttgat gtttg 35
<210> 404
   <211> 35
   <212> DNA
   <213> Thiobacillus ferrooxidans
<400> 404
   cttgaccata tatcaccaag cattaaagag cttcc 35
<210> 405
   <211> 35
   <212> DNA
   <213> Sphingomonas paucimobilis
<400> 405
   cttgtcccta taaccttggt agtccaaggt cgagt 35
<210> 406
   <211> 35
   <212> DNA
   <213> Künstliche Sequenz
<220>
   <223> Beschreibung der künstlichen Sequenz: abgeleitet
   von Arten der Gattung Alcaligenes
<400> 406
   cttgactata caacacccaa gcagttgtat ataaa 35
<210> 407
   <211> 23
   <212> DNA
   <213> Pseudomonas cepacia
<400> 407
   aggactaacg actcgtgaag ctg 23
<210> 408
   <211> 29
   <212> DNA
   <213> Ralstonia pickettii
<400> 408
   cttgaccata taacacccaa gcaatttga 29
<210> 409
   <211> 35
   <212> DNA
   <213> Campylobacter jejuni
<400> 409
   cttatcttta ataaagcatc acttccttgt taagg 35
<210> 410
   <211> 35
   <212> DNA
   <213> Helicobacter pylori
<400> 410
   cttgtttttt gctttttgat aagataacgg caata 35
<210> 411
   <211> 33
   <212> DNA
   <213> Actinoplanes utahensis
<400> 411
   cggtaacgtg ttgagttgac cggtactaat agg 33
<210> 412
   <211> 35
   <212> DNA
   <213> Bacillus halodurans
<400> 412
   ttatccaaaa acaaatcaaa agcaacgtct cgaac 35
<210> 413
   <211> 21
   <212> DNA
   <213> Bacillus subtilis
<400> 413
   ttaaccacat tttgaatgat g 21

<210> 414
   <211> 32
   <212> DNA
   <213> Clostridium tyrobutyricum
<400> 414
   ttgaccaaat ttatcttact gtgcaatttt ca 32
<210> 415
   <211> 56
   <212> DNA
   <213> Künstliche Sequenz
<220>
   <223> Beschreibung der künstlichen Sequenz: abgeleitet
   von Arten der Gattung Frankia
<400> 415
   cggtgacgca tggagctgac cggtactaat aggccgaggg cttgtcttcg aaggtg 56
<210> 416
   <211> 56
   <212> DNA
   <213> Microbispora bispora
<400> 416
   cggtaacgtg tggagccgac cggtactaat aagccgagag gcttgacttc acatgc 56
<210> 417
   <211> 56
   <212> DNA
   <213> Mycobacterium leprae
<400> 417
   cagtaatgag tgtagggaac tggcactaac tggccgaaag cttacaaaac acacac 56
<210> 418
   <211> 56
   <212> DNA
   <213> Mycobacterium smegmatis
<400> 418
   tagtaatagg tgcagggaac tggcactaac cggccgaaaa cttacaacac cccata 56
<210> 419
   <211> 56
   <212> DNA
   <213> Mycobacterium tuberculosis
<400> 419
   cagtaatggg tgtagggaac tggtgctaac cggccgaaaa cttacaacac cctccc 56
<210> 420
   <211> 39
   <212> DNA
   <213> Mycobacterium gallisepticum
<400> 420
   cgttgatagg ctaaaggtgt aagtgccgcg aggtattta 39
<210> 421
   <211> 39
   <212> DNA
   <213> Propionibacterium freudenreichii
<400> 421
   ttgtcccaca ctttaattct tgtagattgt tgtgaagag 39
<210> 422
   <211> 41
   <212> DNA
   <213> Rhodococcus erythropolis
<400> 422
   cagtaatgca tgcaggtgac tggtactaat aggccgagga c 41
<210> 423
   <211> 41
   <212> DNA
   <213> Rhodococcus fascians
<400> 423
   cagcaatgta tgcaggtgac tggtactaat aggccgagga c 41
<210> 424
   <211> 33
   <212> DNA
   <213> Staphylococcus aureus
<400> 424
   ttaaccaaaa taaatgtttt gcgaagcaaa atc 33
<210> 425
   <211> 42
   <212> DNA
   <213> Streptococcus faecalis
<400> 425
   ttaaccaaag aatggataag taaaagcaac ttggttattt tg 42
<210> 426
   <211> 56
   <212> DNA
   <213> Streptomyces lividans
<400> 426
   ccgcaaggtg tggaggtgac cggtactaat aggccgaggg cttgtcctca tttgct 56
<210> 427
   <211> 56
   <212> DNA
   <213> Streptomyces mashuensis
<400> 427
   cggtaacggt tggagctgac tggtactaat aggccgaggg cttgtccata gttgct 56
<210> 428
   <211> 43
   <212> DNA
   <213> Flavobacterium resinovorum
<400> 428
   cttgatccta taaccagtgt gttttgcctg gtgggtgatc gcg 43
<210> 429
   <211> 28
   <212> DNA
   <213> Künstliche Sequenz
<220>
   <223> Beschreibung der künstlichen Sequenz: abgeleitet
   von Arten der Gattung Synechococcus
<400> 429
   ttgacctcta acactttgat atcggcac 28
<210> 430
   <211> 28
   <212> DNA
   <213> Künstliche Sequenz
<220>
   <223> Beschreibung der künstlichen Sequenz: abgeleitet
   von Arten der Gattung Synechocystis
<400> 430
   ttgaccttta ttcttcattt ttctttct 28
<210> 431
   <211> 34
   <212> DNA
   <213> Chlamyia trachomatis
<400> 431
   cttggtcttt ttatgattgg aagagccgaa aggc 34
<210> 432
   <211> 51
   <212> DNA
   <213> Salmonella typhi
<400> 432
   cttaacctta caacaccgaa ggtgttttgg aggataaaag aaacagaatt t 51
<210> 433
   <211> 117
   <212> DNA
   <213> Buchnera aphidocola
<400> 433
   cttaacctta caacaccaga ggtgtttttt ataaaaaata aaaaatcttg ttttactgaa 60
   tttattgttg tattaatata tatatattat aatagcacta aaaaatgcct ggtaaaa    117
<210> 434
   <211> 233
   <212> DNA
   <213> Pseudomonas stutzeri
<400> 434
   cttgaccata taacacccaa acaatttgat gtttgcgtgt cagacggttg aagtcgacaa 60
   acaaaccgaa agacgcaacg ctcgcaaagc gaaagcgata ccgaagcaac catcacatac 120
   ccaattaggg aagcgactca acaccgactc cccagttgaa cttgcttgac gaccatagag 180
   cgttggaacc acctgatccc atcccgaact cagtagtgaa acgacgcatc gcc        233
<210> 435
   <211> 91
   <212> DNA
   <213> Thiobacillus ferrooxidans
<400> 435
   cttgaccata tatcaccaag cattaaagag cttcccttca gcaacacctc gagggcggca 60
   cagccgcgcc cgggaccaga ccagttttaa c                                91
<210> 436
   <211> 230
   <212> DNA
   <213> Agrobacterium vitis
<400> 436
   cttaatcgtt ctcattgacc atgctcatcg acttcgtcga tgagccatct gtttagcgct 60
   cacgcatgag cggctcgtat acgagcctat gctccgcgag ggcgccgaac gatcggcgac 120
   gcgccttgcg cttgcggact tcgtccgaaa gtgccaagca aaacgtcgcg gaatgacgtg 180
   ttcacacaat aagaaaacgg gcaatgcccg ccagcttctc atcaacattg            230
<210> 437
   <211> 162
   <212> DNA
   <213> Adalia bipunctata
<400> 437
   tttactttgc tgtgagatta cacatgcata tggtgttaat tctataaaca tgtaagtatc 60
   aactcacaaa gttatcaggt taaattagct ttatcaacca ataaagatgt tgttacatgt 120
   ctctttctat gttgttcctg tgaaagtaag aatctagaaa aa                    162
<210> 438
   <211> 120
   <212> DNA
   <213> Amycolatopsis orientalis
<400> 438
   tggtaacggg tggagttgac tggtactaat aggccgaggg cttgtcctca gttgctcgcg 60
   tccactgtgt tagttctgaa gtaacgaaca tcgccttgtc ggctggagtt caacttcata 120
<210> 439
   <211> 189
   <212> DNA
   <213> Künstliche Sequenz
<220>
   <223> Beschreibung der künstlichen Sequenz: abgeleitet
   von Arten der Gattung Brucella
<400> 439
   cttgatcact cccatttaca atatccatca agcaaaagct tgatgttgaa ggcaatatgg 60
   aagtagggca ataaggcaat atgtttgccc aaagccctca accatcgcca cgcagaaaaa 120
   caaagcacaa aggcaaagaa caggcgcagc ccaaacatac tgccctattc ccctaatgcc 180
   ttaagcccc                                                         189
<210> 440
   <211> 109
   <212> DNA
   <213> Bradyrhizobium japonicum
<400> 440
   cttgattgct ctcattttca gtgtccatag ggccgcaagg cccgcgacca gaatgaaatg 60
   agaggcgcta gtcgcccaac aaagatcgct tgcttcgtat tccttgtcc             109
<210> 441
   <211> 125
   <212> DNA
   <213> Pseudomonas paucimobilis
<400> 441
   cttaaccaat ttgaatgtat gcttactgtt atctagtttt gagagaacac tctcaatggt 60
   ttggtggcga tagcgaagag gtcacacccg ttcccatgcc gaacacggaa gttaagctct 120
   tcagc                                                             125
<210> 442
   <211> 100
   <212> DNA
   <213> Rhodobacter sphaeroides
<400> 442
   cttgatctga cccggtaaca gcaaggctca aaagccaacg ctctacccca gatcagaagc 60
   aatagacccg gaacaagcaa aagcctgatg ttgtcgtttc                       100
<210> 443
   <211> 196
   <212> DNA
   <213> Rickettsia prowazekii
<400> 443
   tttactttgc tgtgagatta tatatgcata tagtgttaat tatataagta tttaagcatc 60
   aatttgtaaa ttataatttt aatgttaaat tagctttatc aataaataaa aatgttattc 120
   tatcgtttta tgttacgatt tgatagtaaa gttttgatct ttctttaaga tattgtagac 180
   aattgtatat tatacc                                                 196
<210> 444
   <211> 249
   <212> DNA
   <213> Pseudomonas cepacia
<400> 444
   aggactaacg actcgtgaag ctgaccggta ctaataggcc gataacttac accacacacc 60
   cttttcgtga acggattcaa aagacgttca caccaggaga gggtaaaaag aaaaaacaag 120
   actgcttgcg tccactatgt ggttcccaac caacaaaccc gccacgggca cgttgcgaca 180
   ggaacacaac tgaataacaa caccacaatg ttgtaaccac aaagacttcc cacccccggc 240
   atcagaccc                                                         249
<210> 445
   <211> 209
   <212> DNA
   <213> Ralstonia pickettii
<400> 445
   cttgaccata taacacccaa gcaatttgag cgtaggcgcc aaattgtggt ggtgaagatg 60
   atacgaaccg aaagttcgca acgaaccaca acatcacata tccgaattcg ctgggctgtc 120
   catctggaca ttctggctac agaatttctt gacgaccata gagcattgga accacctgat 180
   cccatcccga actcagcagt gnaacgatg                                  209
<210> 446
   <211> 271
   <212> DNA
   <213> Campylobacter jejuni
<400> 446
   cttatcttta ataaagcatc acttccttgt taaggttttt aagaagactt tgaatataga 60
   taatatttag agtttaatag aaatctttca agtaaagttt gtattagaac ttgctcttaa 120
   cattgttttt taagtattct atataaaaac ttatcaaaga taaaagataa gaaaagaaga 180
   aagagaataa aagattaagt tttattctta aattcaattt ttcaaagaat atttaaataa 240
   caatgtccgt gattatacag atgtggaaac g                                271
<210> 447
   <211> 228
   <212> DNA
   <213> Helicobacter pylori
<400> 447
   cttgtttttt gctttttgat aagataacgg caataagcgc gaatgggtta ccactgcctt 60
   actgagtgta agagagttgg agttttatga agacttttat aagattaaac tttaatgagg 120
   aatgagatac catctcaatg gtttaaagtt aaaggctatt aacgatcttc tttgttaaaa 180
   acagctcccc tataaagaga aaggggagtt aagggtaaat gcgttttt              228
<210> 448
   <211> 155
   <212> DNA
   <213> Actinoplanes utahensis
<400> 448
   cggtaacgtg ttgagttgac cggtactaat aggccgaggg cttaaccacc ctaaattttc 60
   tgcttgcgtc cactgtgtga ttcacagcaa acgaacaacc accccggttc aagagtgccg 120
   ggttgctggt ttgttctgct gatggctgtt tcgat                            155
<210> 449
   <211> 296
   <212> DNA
   <213> Bacillus halodurans
<400> 449
   cttatccaaa aacaaatcaa aagcaacgtc tcgaactcga gaagcgtccc attatctagt 60
   tttgagagaa tcttgttctc caaagaagcg ctccgacgca gcatcgcaag atgcgaagtt 120
   gatcggaagc cgtgatcaag agattattct cttaggtcca aagaaaaggg tttcgagaaa 180
   cgagcagttt taggaatcga gcgacgacag atcggagcgt acacacggta cgtgaggatc 240
   tggaggagtg aagatgacac caaaatgcga tgttgatcgg aggccgtaac tatcta     296
<210> 450
   <211> 122
   <212> DNA
   <213> Bacillus halodurans
<400> 450
   cttaaccaca ttttgaatga tgtcacacct gttatctagt tttgagagaa cacctctcta 60
   aaggcggaag gtaaggaaac tccgctaagg gctctcacat cctgtgagaa acgcccagta 120
   cc                                                                122
<210> 451
   <211> 209
   <212> DNA
   <213> Clostridium tyrobutyricum
<400> 451
   cttgaccaaa tttatcttac tgtgcaattt tcagagaata attattctct tatctccatt 60
   agaaatataa tgtttctatt ttattataga gaataaagta agtaaattga taataaccat 120
   tagtacaagg aagatatgag cgaagagcgg aatttactta ggtaaatgag cactggagtg 180
   aataattctg acggtgtaat gagaagtta                                   209
<210> 452
   <211> 100
   <212> DNA
   <213> Künstliche Sequenz
<220>
   <223> Beschreibung der künstlichen Sequenz: abgeleitet
   von Arten der Gattung Frankia
<400> 452
   cggtgacgca tggagctgac cggtactaat aggccgaggg cttgtcttcg aaggtgctac 60
   gcgtccactg tgcggttctc gggtgtacgg ccggttcggc                       100
<210> 453
   <211> 85
   <212> DNA
   <213> Microbispora bispora
<400> 453
   cggtaacgtg tggagccgac cggtactaat aagccgagag gcttgacttc acatgcacgc 60
   acccactatg cgattctcga tcagc                                        85
<210> 454
   <211> 124
   <212> DNA
   <213> Mycobacterium leprae
<400> 454
   cagtaatgag tgtagggaac tggcactaac tggccgaaag cttacaaaac acacacatcg 60
   caaccacata attcagatcc actttgtcgt ggagcatcac accccccatc agaacaaatt 120
   ttta                                                              124
<210> 455
   <211> 146
   <212> DNA
   <213> Mycobacterium smegmatis
<400> 455
   tagtaatagg tgcagggaac tggcactaac cggccgaaaa cttacaacac cccataatcg 60
   ttgtaagaag aaaacattga cgcaccgcgc tcgcaaccac actccacgga tgatcaaacc 120
   cacaagtttg ctctccatgt gggtca                                      146
<210> 456
   <211> 135
   <212> DNA
   <213> Mycobacterium tuberculosis
<400> 456
   cagtaatggg tgtagggaac tggtgctaac cggccgaaaa cttacaacac cctccctttt 60
   ggaaaaggga ggcaaaaaca aactcgcaac cacatccgtt cacggcgcta gccgtgcgtc 120
   cacacccccc accag                                                  135
<210> 457
   <211> 169
   <212> DNA
   <213> Mycobacterium gallisepticum
<400> 457
   cgttgatagg ctaaaggtgt aagtgccgcg aggtatttag ctgattagta ctaataattc 60
   gaggacttag atttgatcaa aaacattagc tgttttttat ctaatatgat ttgttgtatt 120
   ttgtttttca aagagcaatg tgtgtgatat cgatatcgtg atggaaaca              169
<210> 458
   <211> 43
   <212> DNA
   <213> Propionibacterium freudenreichii
<400> 458
   cttgtcccac actttaattc ttgtagattg ttgtgaagag ttt 43
<210> 459
   <211> 182
   <212> DNA
   <213> Rhodococcus erythropolis
<400> 459
   cagtaatgca tgcaggtgac tggtactaat aggccgagga cttaccacaa agaagctacg 60
   cgtccactgt gcggtatctg aaacaacaca cagatactga tgagaaaccc tgttttctcc 120
   atcccccaac accagaaact ggtgttgacg tggtgaaacc aggtgatcag aagaaggtta 180
   ct 182
<210> 460
   <211> 168
   <212> DNA
   <213> Rhodococcus fascians
<400> 460
   cagcaatgta tgcaggtgac tggtactaat aggccgagga cttaccacaa agaagctacg 60
   cgtccactgt gcaatatctg aaacaacaca cgagtagttg ttcgacaaca gaaccgaata 120
   cacgaatccg ccacccacac gagtgtgggt gacaggttcg ctcgttga 168
<210> 461
   <211> 64
   <212> DNA
   <213> Staphylococcus aureus
<400> 461
   cttaaccaaa ataaatgttt tgcgaagcaa aatcactttt acttactatc tagttttgaa 60
   tgta 64
<210> 462
   <211> 87
   <212> DNA
   <213> Streptococcus faecalis
<400> 462
   cttaaccaaa gaatggataa gtaaaagcaa cttggttatt ttgattcaaa cttcaatcca 60
   gttttgagtg aatnaagatt cnctcaa 87
<210> 463
   <211> 123
   <212> DNA
   <213> Streptomyces ambifaciens
<400> 463
   ccgcaaggtg tggaggtgac cggtactaat aggccgaggg cttgtcctca tttgctcgcg 60
   tccactgtgt tggttctgaa accacgaaca accccatgtg ccacacatgg tgcggttgtc 120
   agt                                                               123
<210> 464
   <211> 134
   <212> DNA
   <213> Streptomyces galbus
<400> 464
   cggtaacgtg tggaggtgac cggtactaat aggccgaggg cttgtcctca gttgctcgcg 60
   tccactgtgt tggttctgaa accacgaaca gccccatgct ctggcatggt gcggcattgt 120
   tcgacagttt cata                                                   134
<210> 465
   <211> 143
   <212> DNA
   <213> Streptomyces griseus
<400> 465
   cggtaacggg tggagctgac tggtactaat aggccgaggg cttgtcctca gttgctcgcg 60
   tccactgtgt tgttcccggg ttgcgaacag ttatcgcacc ggttgaacag tttcactact 120
   taattgaaga gtgtgcttgt tcg                                         143
<210> 466
   <211> 137
   <212> DNA
   <213> Streptomyces lividans
<400> 466
   ccgtgaggtg tggaggtgac cggtactaat aggccgaggg cttgtcctca gttgctcgcg 60
   tccactgtgt tagttctgag gcaacgaccg ttgccggatt tgagtagaac gcacaattaa 120
   agagtgtgct tgttcgc                                                137
<210> 467
   <211> 135
   <212> DNA
   <213> Streptomyces mashuensis
<400> 467
   cggtaacggt tggagctgac tggtactaat aggccgaggg cttgtccata gttgctcgcg 60
   ttcactgtgt tggttctgaa acaacaacca agaagcatac gccgtgtgtg gttgacagtt 120
   tcatagtgtt tcggt                                                  135
<210> 468
   <211> 114
   <212> DNA
   <213> Flavobacterium resinovorum
<400> 468
   cttgatccta taaccagtgt gttttgcctg gtgggtgatc gcgactgtgc cgaaacagtt 60
   gacacgcaca accccaacta catccctatt cgcagcgttg acctcaacct cagc       114
<210> 469
   <211> 126
   <212> DNA
   <213> Sphingobacterium multivorans
<400> 469
   ctttctcaag cagataacac tgttgtcttc ctctttaatt tttagaaacg aaaagaataa 60
   caaaaaagaa acgaagctct ttcaatagat atgtcagttg gcctgacgat gatatattat 120
   cataag                                                            126
<210> 470
   <211> 63
   <212> DNA
   <213> Künstliche Sequenz
<220>
   <223> Beschreibung der künstlichen Sequenz: abgeleitet
   von Arten der Gattung Synechococcus
<400> 470
   cttgacctct aacactttga tatcggcact ctcctctatg cagccttcaa ggctctaatc 60
   tcc                                                               63
<210> 471
   <211> 67
   <212> DNA
   <213> Künstliche Sequenz
<220>
   <223> Beschreibung der künstlichen Sequenz: abgeleitet
   von Arten der Gattung Synechocystis
<400> 471
   cttgaccttt attcttcatt tttctttctc ttttcttgtg cagtcttctg ggtttcttct 60
   cagcaaa                                                           67
<210> 472
   <211> 17
   <212> DNA
   <213> Borrelia burgdorferi
<400> 472
   ctttggccat atttttg 17
<210> 473
   <211> 111
   <212> DNA
   <213> Chlamydia trachomatis
<400> 473
   cttggtcttt ttatgattgg aagagccgaa aggcaaagac aataagaaaa agagtagaga 60
   gtgcaagtac gtagaagaca agcttttaag cgtctattag tatacgtgag a          111
<210> 474
   <211> 148
   <212> DNA
   <213> Azotobacter vinelandii
<400> 474
   aaacaatctg ttgccagccc cagcggggcg gcacggagag ggcgcagccg acaggccgaa 60
   gatttggctg gaccgcacgc tgccggaaac aggctaccgc tatcacctac ccgattggct 120
   gtcgtgtcat cgacacggcg gcaaccga                                    148
<210> 475
   <211> 229
   <212> DNA
   <213> Cowduria ruminantium
<400> 475
   ggtgtgtaag tatggtaaca tatgtagcta accagtacta atagcccgat tgatttactt 60
   aatttgtaat tatatgtagt attaaaactg cagcttgtct ttttgcttat tttgttttat 120
   agtttaattg ggttggtggt aatagcagaa gtgatacacc cagctacatt tcgaacctgg 180
   aagttaagcc ttctagcgct tatggtactt tgtcttaagg cacgggaga             229
<210> 476
   <211> 110
   <212> DNA
   <213> Mycobacterium intracellulare
<400> 476
   taagcttgat tcacacactc gcaaccacag tccatttcgc gcgttctgcc gctgaagcta 60
   gaacaccgca ccccccacca aacaaattta aatagagtta cggcggccac             110
<210> 477
   <211> 107
   <212> DNA
   <213> Mycobacterium lufu
<400> 477
   aaaacttacc gaacacacaa tcgcaaccac agtccatttc acggcagcaa tgccgcgaaa 60
   cgccacaccc cccaccaaac aaatttaaat agagttacgg cggccac               107
<210> 478
   <211> 120
   <212> DNA
   <213> Mycobacterium simiae
<400> 478
   taagcttgat tcacacacat cgcaaccact atcgtcgcga cttattgtcg cgccgaatgc 60
   cacacccccc accagaacaa ctaataaaat agtgttccgt aatagagtta cggcggccac 120
<210> 479
   <211> 149
   <212> DNA
   <213> Mycobacterium smegmatis
<400> 479
   caccccataa cgttgtaaga agaaaacatt gaccaccgcg ctcgcaacca cactccacgg 60
   atgatcaaac cgatcacccc accaccaaaa caaacccaca agtttgctct ccatgtgggt 120
   caccacataa gagaatagag ttacggcgg                                   149
<210> 480
   <211> 75
   <212> DNA
   <213> Saccharomonospora azurea
<400> 480
   caaagatgct acgcacccac tctgcaactc tgaaacacca caccccggaa acatgatcct 60
   gggttgtttc acagt                                                  75
<210> 481
   <211> 73
   <212> DNA
   <213> Saccharomonospora caesia
<400> 481
   caaagatgct acgcacccac tctgcaactc tgaaacacca caccccggaa acgatcctgg 60
   gttgtttcac agt                                                    73
<210> 482
   <211> 75
   <212> DNA
   <213> Saccharomonospora cyanea
<400> 482
   caaacatgct acgcacccac tctgcaactc tgaaacacca ccccgggaac acacccggcg 60
   tgattgtttc ccaga                                                  75
<210> 483
   <211> 69
   <212> DNA
   <213> Saccharomonospora glauca
<400> 483
   caaagacgct acgcacccac tctgcgactc tgaaacacca ccctggtgtg ccagtggttg 60
   tttcacaga                                                         69
<210> 484
   <211> 74
   <212> DNA
   <213> Saccharomonospora viridis
<400> 484
   caaaggtgct acgcacccac tctgcaactc tgaaacacca cacccccaca acaccgggct 60
   ggttgtttca caga                                                   74
<210> 485
   <211> 304
   <212> DNA
   <213> Wolbachia pipientis
<400> 485
   taactggtac taatagcctg attgatttat ttgctttcta tatgtgcata tgcagtgtta 60
   aatattaagt taaaatttat taagtcagaa atttttgttg acttggtggc tatagcaaaa 120
   atgaaccacc cgatctcatt tcgaactcgg aagtgaaact ttttagcgcc gatgatactt 180
   aaaaacccaa agtaggtcgt tgccaagttt ataaaaattt cttcttattt atatcttttc 240
   agtagagcga tgaaacaagg taaacataga gtagctgtga ggtaatataa ctgatctttt 300
   agaa                                                              304
<210> 486
   <211> 34
   <212> DNA
   <213> Salmonella typhi
<400> 486
   ttcctggcgg cactagcgcg gtggtcccac ctga 34
<210> 487
   <211> 22
   <212> DNA
   <213> Buchnera aphidocola
<400> 487
   atagtgtagt ggtaccacct ga 22
<210> 488
   <211> 53
   <212> DNA
<213> Pseudomonas stutzeri
<400> 488
   catcgccgat ggtagctgtg gggtctcccc atgtgagagt aggtcatcgt caa 53
<210> 489
   <211> 35
   <212> DNA
   <213> Thiobacillus ferrooxidans
<400> 489
   cttgtctggc ggccatagcg cagtggaacc acccc 35
<210> 490
   <211> 52
   <212> DNA
   <213> Agrobacterium vitis
<400> 490
   atcaacattg cccttagctg acctggtggt catggcgggg cggccgcacc cg 52
<210> 491
   <211> 38
   <212> DNA
   <213> Adalia bipunctata
<400> 491
   gccatgcaac aatgttaaca gcagactaat acaaatct 38
<210> 492
   <211> 52
   <212> DNA
   <213> Künstliche Sequenz
<220>
   <223> Beschreibung der künstlichen Sequenz: abgeleitet
   von Arten der Gattung Brucella
<400> 492
   atgtttgtgt tcttcgccga cctggtggtt atggcggagc ggccgcaccc ga 52
<210> 493
   <211> 40
   <212> DNA
   <213> Bradyrhizobium japonicum
<400> 493
   ttcgccggcc tggtggtttt agcgaagagc ctcaacccga 40
<210> 494
   <211> 36
   <212> DNA
   <213> Pseudomonas paucimobilis
<400> 494
   tcttcagcgc cgatggtagt cggggttccc cctaat 36
<210> 495
   <211> 40
   <212> DNA
   <213> Rhodobacter sphaeroides
<400> 495
   ttctccggtc tggtggccat agcacgagca aaacacccga 40
<210> 496
   <211> 53
   <212> DNA
   <213> Rickettsia prowazekii
<400> 496
   ccttgcttaa gaataatata atagcattaa cagcatatta taatacaacc tat 53
<210> 497
   <211> 51
   <212> DNA
   <213> Rickettsia bellii
<400> 497
   aaatttcttt aagtcctgca acaacactaa cagcaaacca atacaaatct a 51
<210> 498
   <211> 53
   <212> DNA
   <213> Rickettsia rickettsii
<400> 498
   gaattttttt gagtcgtgca acaacattaa cagtagacta taatacaaat cta 53
<210> 499
   <211> 47
   <212> DNA
   <213> Sphingomonas paucimobilis
<400> 499
   gccagacaag tcaaagcctg atgaccatag caagtcggtc ccacccc 47
<210> 500
   <211> 33
   <212> DNA
   <213> Zymomonas mobilis
<400> 500
   gcttggtggc tatagcgtca gtgacccacc cga 33
<210> 501
   <211> 53
   <212> DNA
   <213> Künstliche Sequenz
<220>
   <223> Beschreibung der künstlichen Sequenz: abgeleitet
   von Arten der Gattung Alcaligenes
   <400> 501
   gcaagtatcc ataccagttg tgctggcgac catagcaaga gtgaaccacc tga 53
   <210> 502
   <211> 51
   <212> DNA
   <213> Pseudomonas cepacia
<400> 502
   cgggcggacg ggtacaaggg ttacggcggt catagcgtgg gggaaacgcc c 51
<210> 503
   <211> 48
   <212> DNA
   <213> Ralstonia pickettii
<400> 503
   catcgccgat ggtagtgtgg ggtttcccca tgcgagagta ggacatag 48
<210> 504
   <211> 51
   <212> DNA
   <213> Helicobacter pylori
<400> 504
   ttatctttag ctcccttttc cttgtgcctt tagagaagag gaactaccca g 51
<210> 505
   <211> 52
   <212> DNA
   <213> Bacillus halodurans
<400> 505
   caaagaggat caagagattt gcggaagcaa gcgagtgacg aactgagcgt at 52
<210> 506
   <211> 52
   <212> DNA
   <213> Bacillus halodurans
<400> 506
   ccttcatcct gaaggcattt gtttggtggc gatagcgaag aggtcacacc cg 52
<210> 507
   <211> 52
   <212> DNA
   <213> Clostridium tyrobutyricum
<400> 507
   ttagcagcaa tttacggttg atctggtaac aatgacgtga aggtaacact cc 52
<210> 508
   <211> 51
   <212> DNA
   <213> Künstliche Sequenz
<220>
   <223> Beschreibung der künstlichen Sequenz: abgeleitet
   von Arten der Gattung Frankia
<400> 508
   ggttgtatag ttgaatagtg tttcggtggt tttggcgaag gggaaacgcc c 51
<210> 509
   <211> 50
   <212> DNA
   <213> Microbispora bispora
<400> 509
   gtcctcacct gaaggcttgc cgctatcccg cgtcgagcag gtgaattccg 50
<210> 510
   <211> 45
   <212> DNA
   <213> Mycobacterium leprae
<400> 510
   aattttatag agttacggtg gccacagcga tagggaaacg cccgg 45
<210> 511
   <211> 52
   <212> DNA
   <213> Mycobacterium smegmatis
<400> 511
   accacataag agaatagagt tacggcggtc catagcggca gggaaacgcc cg 52
<210> 512
   <211> 49
   <212> DNA
   <213> Mycobacterium tuberculosis
<400> 512
   agaacaaatt tgcatagagt tacggcggcc acagcggcag ggaaacgcc 49
<210> 513
   <211> 51
   <212> DNA
   <213> Rhodococcus erythropolis
<400> 513
   ctgtgacagt ttcatagagt tacggcggtc atagcgaagg ggaaacgccc g 51
<210> 514
   <211> 52
   <212> DNA
   <213> Rhodococcus fascians
<400> 514
   ttgacactgt ttcgcagagt tacggcggcc atagcggagg ggaaaccgcc cg 52
<210> 515
   <211> 53
   <212> DNA
   <213> Staphylococcus aureus
<400> 515
   tgtataaatt acattcatat gtctggtgac tatagcaagg aggtcacacc tgt 53
<210> 516
   <211> 50
   <212> DNA
   <213> Streptococcus faecalis
<400> 516
   taagaaacaa cacccagtgt ggtggcgata gcgagaagga tacacctgtt 50
<210> 517
   <211> 47
   <212> DNA
   <213> Streptomyces ambifaciens
<400> 517
   tcagtttcat agtgtttcgg tggtcatagc gttagggaaa cgcccgg 47
<210> 518
   <211> 53
   <212> DNA
   <213> Künstliche Sequenz
<220>
   <223> Beschreibung der künstlichen Sequenz: abgeleitet
   von Arten der Gattung Streptomyces
<400> 518
   ttcgctagaa cccgataggg tttcggtggt cattgcgtta gggaaacgcc cgg 53
<210> 519
   <211> 47
   <212> DNA
   <213> Flavobacterium resinovorum
<400> 519
   gctgcaaccc ctcatgcctg gtgaccatag cgagctggaa ccacccc 47
<210> 520
   <211> 52
   <212> DNA
   <213> Spingobacterium multivorans
<400> 520
   taagacagac caataaagat ttttaggtgc ctatatcggc ggtgtctacc tc 52
<210> 521
   <211> 53
   <212> DNA
   <213> Künstliche Sequenz
<220>
   <223> Beschreibung der künstlichen Sequenz: abgeleitet
   von Arten der Gattung Synechococcus
<400> 521
   ccatagagtc acacccttcc tggtgtctat ggcggtatgg aaccactctg acc 53
<210> 522
   <211> 60
   <212> DNA
   <213> Künstliche Sequenz
<220>
   <223> Beschreibung der künstlichen Sequenz: abgeleitet
   von Arten der Gattung Synechocystis
<400> 522
   agcaaaaccc aaaaatcttt cttggtgtct ttagcgtcat ggaaccactc cgatcccatc 60
<210> 523
   <211> 53
   <212> DNA
   <213> Borrelia burgdorferi
<400> 523
   ttttgtcttc cttgtaaaaa ccctggtggt taaagaaaag aggaaacacc tgt 53
<210> 524
   <211> 51
   <212> DNA
   <213> Chlamydia trachomatis
<400> 524
   gagaaacgat gccaggatta gcttggtgat aatagagaga gggaaacacc t 51
<210> 525
   <211> 138
   <212> DNA
   <213> Sphingomonas paucimobilis
<400> 525
   ctataacctt ggtagtccaa ggtcgagtac aactgctcga tacaagctac aacccaacaa 60
   tacttcttcc agattcatgg ccacgctgaa caaagcgtag ggtgggcggc tgtnccgccc 120
   acgcgtaact caagcgta                                               138
<210> 526
   <211> 107
   <212> DNA
   <213> Zymomonas mobilis
<400> 526
   ttttgagaac tccactgtca atgtcagcat tgctgacctg ataatgtttt ctcttagctc 60
   ttttgaatat cttcgatttt caattaactt cacgcacagg tgtcata               107
<210> 527
   <211> 167
   <212> DNA
   <213> Künstliche Sequenz
<220>
   <223> Beschreibung der künstlichen Sequenz: abgeleitet
   von Arten der Gattung Alcaligenes
<400> 527
   atacaacacc caagcagttg tatataaagc atcaatcgat tcattaatat gcaaagcaac 60
   ttgatttagt tatacgctta gctaaaatga acaaaatata gtaagactca atcagcccat 120
   ctgtaaagat ttggaaaacg catcggcaac caataagacc aatgcaa               167
<210> 528
   <211> 225
   <212> DNA
   <213> Borrelia burgdorferi
<400> 528
   ctgcgagttc gcgggagagt aagttattgc cagggttttt tatttttttt tagtttttat 60
   gttatttaaa tggcttattc aaacaacata aaaaagaaaa tagatattga catggattaa 120
   acaaaagata tatattattc tatgttgcat aaacaaattg gcaaagtaga gatggaagat 180
   aaaaatatgg tcaaagtaat aagagtctat ggtgaatgcc tagga                 225
<210> 529
   <211> 681
   <212> DNA
   <213> Xanthomonas campestris
<400> 529
   tggagcaaga cgtcattcgt cctagtcggg cgtcctcaca aattacctgc attcagagat 60
   tcataccggc acaggtcggt atgcgaagtc ccttttgggg ccttagctca gctgggagag 120
   cacctgcttt gcaagcaggg ggtcgtcggt tcgatcccga caggctccac catattgagt 180
   gaaaagactt cgggtctgta gctcaggtgg ttagagcgca cccctgataa gggtgaggtc 240
   ggtagttcga gtctacccag acccaccact ctgaatgtag tgcacactta agaatttata 300
   tggatcagcg ttgaggctga gacatgttct tttataactt gtgacgtagc gagcgtttga 360
   gatatctatc taaacgtgtc gttgaagcta aggcggggac ttcgagtccc taaataattg 420
   agtcgtatgt tcgcgttggg tggctttgtt acccacacaa cacgtacatg ttagctccga 480
   ggcaacttgg ggttatatgg tcaagcgaat aagcgcacac ggtggatgcc taggcggtca 540
   gtggcgatgt aggacgtggt agcctgcgaa aagtgtcggg gagctggcaa caagctttga 600
   tccggcaata tccgaatggg gaaacccact gcttcggcag tatcttgcag tgaattcata 660
   gctgcttgaa gcgaaccccg t                                           681
<210> 530
   <211> 229
   <212> DNA
   <213> Cowduria ruminantium
<400> 530
   ggtgtgtaag tatggtaaca tatgtagcta accagtacta atagcccgat tgatttactt 60
   aatttgtaat tatatgtagt attaaaactg cagcttgtct ttttgcttat tttgttttat 120
   agtttaattg ggttggtggt aatagcagaa gtgatacacc cagctacatt tcgaacctgg 180
   aagttaagcc ttctagcgct tatggtactt tgtcttaagg cacgggaga              229